# EUROPÄISCHE PATENTANMELDUNG

(11) **EP 3 607 978 A1**
(43) Veröffentlichungstag der Anmeldung: **12.02.2020**
(21) Anmeldenummer: 18187611.1
(22) Anmeldetag: 06.08.2018
(51) Int. Cl.: A61M 1/36, A61M 1/34

(54) **VEREINFACHTE REGENERATION VON APHERESESÄULEN**

(71) Anmelder: Pentracor GmbH, 16761 Hennigsdorf (DE)
(72) Erfinder: SHERIFF, Ahmed, 10713 Berlin (DE)
(74) Vertreter: Arth, Hans-Lothar

(57) **Zusammenfassung**

Die vorliegende Erfindung betrifft eine Apheresevorrichtung (1) zur extrakorporalen Entfernung von CRP aus Blut eines Patienten, wobei die Apheresevorrichtung an den Blutkreislauf des Patienten angeschlossen wird. Das Blut wird über einen Teil des extrakorporalen Zirkulationssystems (2) der erfindungsgemäßen Apheresevorrichtung (1) zu einem Zellseparator (7) zur Auftrennung des Bluts in Blutplasma und zelluläre Bestandteile gepumpt. Über einen ersten Ausgang des Zellseparators (7) wird das abgetrennte Blutplasma über eine Plasmaleitung (8A) zu einer Apheresesäule (4) zur affinitätschromatographischen Entfernung von CRP aus dem Blutplasma geleitet. Nach der Entfernung des CRPs aus dem Blutplasma des Patienten wird dieses nun behandelte Blutplasma über eine Plasmaleitung (8B) mit den zellulären Bestandteilen des Bluts zusammengeführt. Des Weiteren umfasst die erfindungsgemäße Apheresevorrichtung (1) eine Bypass-Leitung (12), die von der Plasmaleitung (8A) in die Plasmaleitung (8B) führt unter Umgehung der Apheresesäule (4). Ferner umfasst die erfindungsgemäße Apheresevorrichtung (1) eine Regenerationsleitung (14), die in die Plasmaleitung (8A) oder direkt in die Apheresesäule (4) mündet.

## Beschreibung

Die vorliegende Erfindung betrifft eine Apheresevorrichtung (1) zur extrakorporalen Entfernung von CRP aus Blut eines Patienten, wobei die Apheresevorrichtung an den Blutkreislauf des Patienten angeschlossen wird. Das Blut wird über einen Teil des extrakorporalen Zirkulationssystems (2) der erfindungsgemäßen Apheresevorrichtung (1) zu einem Zellseparator (7) zur Auftrennung des Bluts in Blutplasma und zelluläre Bestandteile gepumpt. Über einen ersten Ausgang des Zellseparators (7) wird das abgetrennte Blutplasma über eine Plasmaleitung (8A) zu einer Apheresesäule (4) zur affinitätschromatographischen Entfernung von CRP aus dem Blutplasma geleitet. Nach der Entfernung des CRPs aus dem Blutplasma des Patienten wird dieses nun behandelte Blutplasma über eine Plasmaleitung (8B) mit den zellulären Bestandteilen des Bluts zusammengeführt. Des Weiteren umfasst die erfindungsgemäße Apheresevorrichtung (1) eine Bypass-Leitung (12), die von der Plasmaleitung (8A) in die Plasmaleitung (8B) führt unter Umgehung der Apheresesäule (4). Ferner umfasst die erfindungsgemäße Apheresevorrichtung (1) eine Regenerationsleitung (14), die in die Plasmaleitung (8A) oder direkt in die Apheresesäule (4) mündet.

Zusätzlich umfasst die vorliegende Erfindung ein Verfahren zur vereinfachten Regeneration einer Apheresesäule.

### Hintergrund der Erfindung

Nach Angaben der Weltgesundheitsorganisation (World Health Organization, WHO) starben im Jahr 2008 cirka 17.000.000 Menschen an kardiovaskulären Erkrankungen. Damit sind kardiovaskuläre Erkrankungen die häufigste Todesursache unter den nicht-übertragbaren Krankheiten und verantwortlich für ungefähr ein Drittel der jährlich weltweit auftretenden Todesfälle. Nach Schätzungen wird diese Zahl bis zum Jahr 2030 auf ungefähr 23.000.000 Todesfälle pro Jahr ansteigen.

Somit sind und bleiben kardiovaskuläre Erkrankungen nicht nur die Haupttodesursache weltweit, sondern verursachen auch enorme medizinische Kosten für die nationalen Gesundheitssysteme und Krankenkassen. Zwei der häufigsten und schädlichsten Erscheinungen kardiovaskulärer Erkrankungen sind das Auftreten von Arteriosklerose und Thrombose, welche wiederum unter anderem ursächlich für Herzinfarkte und Schlaganfälle sind.

In den letzten Jahren wurden große Fortschritte in der Behandlung von kardiovaskulären Erkrankungen erzielt. Dieser Fortschritt wurde nicht nur durch wachsende Erkenntnisse hinsichtlich der Krankheits-auslösenden Mechanismen ermöglicht, sondern auch durch die frühzeitige Identifizierung von Risiko-Patienten. Tatsächlich sind die Identifizierung von Erkrankungsrisiken und deren frühzeitige Behandlung wichtige Merkmale der modernen medizinischen Praxis. In den letzten 25 Jahren wurde eine Vielzahl an Faktoren und klinischen Parametern identifiziert, die entweder mit dem gegenwärtigen Zustand der Erkrankung oder mit der zukünftigen Wahrscheinlichkeit für eine kardiovaskuläre Erkrankung korrelieren. Solche Risikofaktoren können messbare biochemische oder physiologische Parameter wie z.B. Serum-Cholesterin-, HDL-, LDL-, und Fibrinogen-Spiegel sein aber auch Verhaltensmuster wie z.B. Übergewicht und Rauchen beinhalten. In Fällen, in denen ein Risikofaktor nicht lediglich auf eine Erkrankung oder deren Entstehung hinweist, sondern tatsächlich ursächlich an deren Entstehung beteiligt ist, kann eine therapeutische Beeinflussung dieses Risikofaktors den Verlauf der Erkrankung beeinflussen bzw. das Risiko ihrer Entstehung verringern.

CRP ist als Akut-Phase-Protein Teil des angeborenen Immunsystems und wird in der Leber im Zuge von Entzündungsreaktionen gebildet und ins Blut abgegeben. Die Bildung von CRP wird hierbei in erster Linie durch Cytokine induziert, die im Rahmen einer akuten oder chronischen Entzündungsreaktion exprimiert werden. Der stärkste Stimulus zur Bildung von CRP ist hierbei das Interleukin-6 (IL-6). Deshalb sind die Spiegel von CRP ebenso wie von IL-6 im Blut Indikatoren für eine lokale oder systemische Entzündungsreaktion. Chronische Entzündungen werden als eine der zugrundeliegenden und unterstützenden pathologischen Erscheinungen bei kardiovaskulären Erkrankungen vermutet. Hierbei wird zunehmend davon ausgegangen, dass CRP nicht nur prädikativ für kardiovaskuläre Erkrankungen ist, sondern auch kausal an deren Entstehung beteiligt ist bzw. deren Verlauf beeinflussen kann.

Yeh (Clin Cardiol., 2005, 28: 408-412) zeigt, dass der CRP-Spiegel verwendet werden kann, um das kardiovaskuläre Erkrankungsrisiko vorauszusagen, CRP außerdem ein Indikator für Entzündungsreaktionen ist, und dass Entzündungen alle Stadien der Atherosklerose befördern. Zoccali et al., (Semin. Nephrol., 2005, 25: 358-362) zeigen, dass der CRP-Spiegel prädiktiv für das kardiovaskuläre Mortalitätsrisiko bei Patienten mit Nierenerkrankung im Endstadium ist. Nach Nurmohamed et al., (Neth. J. Med., 2005, 63: 376-381) ist der CRP-Spiegel prädiktiv für das kardiovaskuläre Mortalitätsrisiko bei Hämodialysepatienten.

Sola et al., (J. Card. Fail., 2005, 11: 607-612) konnten zeigen, dass Statintherapien dazu genutzt werden können, die Menge an CRP zu senken und so die durch kardiovaskuläre Erkrankung hervorgerufene Mortalität und Morbidität reduziert wird. Diese Therapieform genügt aber nicht, um die hohen CRP-Mengen (bis zu 1000fach über den Normalwerten), welche nach einem Herzinfarkt entstehen, oder die hohen CRP-Mengen im Blut von Dialysepatienten signifikant zu reduzieren.

Slagman et a. 2011 (Specific Removal of C-Reactive Protein by Apheresis in a Porcine Cardiac Infarction Model, Blood Purif 2011;31:9-17), Sheriff et al. 2014 (C-Reactive Protein-Adsorber therapies: new ideas and concepts; LECTURE NOTES OF THE ICB SEMINAR: ADVANCES IN MEMBRANE AND ADSORBER TECHNOLOGY IN LIFE SCIENCES, Warsaw, April 2014), Sheriff et al. 2015 (Selective Apheresis of C-Reactive Protein: A New Therapeutic Option in Myocardial Infarction? Journal of Clinical Apheresis 30:15-21 (2015))

Folglich besteht ein zunehmendes Interesse an therapeutischen Verfahren zur Reduzierung des CRP-Spiegels im Blut von Patienten.

WO 90/12632 offenbart eine Methode und Vorrichtung zur extrakorporalen Behandlung von biologischen Flüssigkeiten mit dem Ziel der Entfernung von CRP sowie von anti-Phosphocholin-Antikörpern aus diesen biologischen Flüssigkeiten zur Behandlung von Krebs. Die hierfür verwendete Phosphocholin-haltige Matrix kann z.B. aus Kieselerde, Sepharose, Acrylbeads oder Agarose bestehen, wobei durch das enthaltene Phosphocholin sowohl CRP als auch anti-Phosphocholin-Antikörper gebunden werden.

WO 2007/076844 offenbart eine Methode durch eine extrakorporale CRP-Entfernung aus dem Blutplasma mittels Apherese das Risiko für einen Patienten verursacht durch einen erhöhten CRP-Spiegel im Blut zu verringern. Gemäß der Erfindung wird hierzu eine Säule mit enthaltener Matrix verwendet, an die Phosphocholin-Derivate gebunden sind um CRP zu binden und aus dem Plasma zu entfernen und so Autoimmunkrankheiten, kardiovaskuläre Erkrankungen, Diabetes sowie Niereninsuffizienz zu behandeln und/oder ihnen vorzubeugen.

Der Normalwert für CRP im Blut von Menschen variiert von Person zu Person, liegt aber im Median bei ungefähr 0,8 mg CRP pro Liter Blut, kann aber im Fall von akuten oder chronischen Entzündungsreaktionen (z.B. bakterielle Infektionen, Atherosklerose, nach einem Herzinfarkt) auf deutlich über 100 mg CRP pro Liter Blut steigen. Da die Halbwertszeit von CRP im Blut (ca. 19 Stunden) konstant und damit unabhängig vom gesundheitlichen Zustand des Patienten ist, ist allein die Syntheserate von CRP für die Regulation des CRP-Spiegels im Blut verantwortlich (Pepys & Hirschfield, J. Clin. Invest., 2003, 111: 1805-1812). Die stark erhöhte Synthese an CRP bei akuten pathologischen Konditionen stellt folglich besondere Anforderungen an therapeutische Ansätze zur CRP-Entfernung von Patienten (Risikopatienten oder Akut-Patienten), da eine erhebliche Menge an CRP entfernt werden muss, um den CRP-Spiegel im Blut auf Normalwerte zu senken. Es besteht also ein Bedarf an besonders effizienten Vorrichtungen zur CRP-Entfernung aus dem Blut von Patienten.

DE 102005061715 A1 offenbart eine Methode zur Behandlung des Risikos von erhöhten Mengen von C-reaktivem Protein (CRP) durch Ausführung einer extrakorporalen Perfusion von Blutplasma durch ein Gerät, wie z. B. eine Säule, welche adsorbierendes Matrixmaterial enthält, einschließend Lipide, Peptide, Polypeptide, Phosphocholin (PC) oder PC-Derivate, um C-reaktives Protein zu entfernen. Nicht offenbart wird die Möglichkeit zur Regeneration der Säule.

Aus dem Stand der Technik sind wiederverwendbare Adsorber bekannt, welche aus einem Gehäuse, gefüllt mit einer Trägersubstanz und daran gekoppeltem Bindungsfaktor bestehen. Wiederverwendbare Adsorber sind in der Regel regenerierbar, da nach Durchleitung einer von der Konzentration der zu adsorbierenden Substanz abhängigen Menge Plasma der Adsorber "gesättigt" ist und keine Bindung der Substanz mehr stattfinden kann. Für eine Behandlung werden meist zwei Adsorber eingesetzt. Während durch einen Adsorber Plasma gepumpt wird, wird der zweite Adsorber zeitgleich regeneriert. Dabei wird der Adsorber mit verschiedenen Regenerationslösungen von den gebundenen Substanzen freigespült und so wieder für eine neuerliche Plasmabeladung aufbereitet. Die zulässige Anzahl der Regenerationen wird von den Herstellern vorgegeben. Der wiederverwendbare Adsorber darf ausschließlich bei ein und demselben Patienten eingesetzt werden. Um ein Keimwachstum in den Adsorbern zu verhindern, müssen diese am Ende jeder Behandlung mit einer Konservierungsflüssigkeit befüllt werden, welche vor jeder neuen Therapie ausgespült werden muss. Jedoch könnten durch eine Wiederverwendung erhebliche Kosten gespart werden. Die Bedienung der bestehenden Vorrichtungen (Kombination aus zwei oder mehr Medizinprodukten) ist sehr komplex und hochgradig anspruchsvoll. Hinzu kommt, dass die Vorrichtungen insgesamt selten genutzt werden.

Die DE 102005019406 A1 offenbart ein Verfahren und eine Vorrichtung zur automatischen Ableitung von Spülflüssigkeit während einer Apheresebehandlung. Ebenfalls offenbart wird die Verwendung von Zusatzgeräten, wobei zwei Adsorber abwechselnd und mehrfach beladen und gespült werden. Diese Zusatzgeräte verfügen über eigene Schlauchpumpen, Schlauchklemmen und Steuerungselemente. Diese aus dem Stand der Technik bekannten Geräte und Verfahren sind zwar effizient, nachteilig daran ist jedoch die gleichzeitige Bedienung von zwei Geräten, welche wie oben erwähnt aufgrund der Komplexität nicht regelmäßig genutzt werden. Gemäß der Offenbarung der DE 102005019406 A1 bestand die Aufgabenstellung darin, Umschaltvorgänge zwischen Flüssigkeitsableitung und Flüssigkeitsrückführung während der Behandlung zu reduzieren, um einen direkten Adsorberbetrieb ohne ein zusätzliches Gerät an einem Zellseparator zu ermöglichen. Dabei lehrt die DE 102005019406 A1 eine Kombination von Auffangbehältern und Rückstromsperren um z.B. mittels eines 3-Wegehahnes im zuführenden Schlauchsystem eine Umstellung der Beladung von einem ersten Adsorber auf den zweiten Absorber zu ermöglichen. Somit lehrt die DE 102005019406 A1 eine alternative Vorrichtung mit zwei Apheresesäulen. Der Nachteil der erhöhten Kosten durch die zweite Apheresesäule, welche auch wiederum nur bei ein und demselben Patienten verwendet werden kann wird durch die Offenbarung der DE 102005019406 A1 nicht beseitigt.

Die WO 2012/143103 offenbart eine Vorrichtung zur extrakorporalen Blutbehandlung und zur Überwachung der Flüssigkeitsströmung darin, wobei die Flüssigkeitsströmung mittels einer Wechselklemme zwischen Behandlungsmodus und Füll- und Spülmodus umgeschaltet wird. Während des Füll- und Spülmodus wird die Spüllösung in einem Sammelbeutel gesammelt, wobei während dem Behandlungsmodus sichergestellt werden muss, dass das gereinigte Plasma nicht in den Sammelbeutel gelangt. Daher ist eine Überwachung der korrekten Funktion der Wechselklemme erforderlich. Die Überwachung der Flüssigkeitsströmung findet durch die Feststellung einer Veränderung des Gewichts des Sammelbeutels statt. Eine Auswerteinheit zur Überwachung der Veränderung des Gewichts des Sammelbeutels wird als zusätzliches Merkmal in der WO 2012/143103 gelehrt und trägt zum komplexen Aufbau des offenbarten Gegenstands bei. Das Fachpersonal, welches die offenbarte Vorrichtung bedient muss ein vorgegebenes Zeitintervall derart bemessen, dass sich die Schlauchleitungen des Flüssigkeitssystems vollständig mit Spülflüssigkeit füllen können. Erst mit der vollständigen Befüllung der Schlauchleitungen des Flüssigkeitssystems mit Flüssigkeit ist eine Gewichtszunahme des Sammelbeutels zu erwarten. Dabei muss das Fachpersonal auf unterschiedliche akustische und/oder optische Signale achten, den die Steuereinheit erzeugt. Die in der WO 2012/143103 gelehrte Vorrichtung stellt folglich einen sehr hohen Trainingsaufwand für das klinische Personal dar. Ferner offenbart die WO 2012/143103 zwar eine Blutbehandlungseinheit (10), welche über einen oder mehrere Filter oder Adsorber verfügt, jedoch wird weder offenbart mit welchen Flüssigkeiten die Blutbehandlungseinheit regeneriert wird, noch ob der Plasmafluss während der Regeneration unterbrochen wird. Somit wird weder die Anwesenheit einer Bypass-Leitung durch die WO 2012/143103 gelehrt noch nahegelegt.

In der DE 4338858 C1 wird ebenfalls eine Vorrichtung zur Regeneration einer Apheresesäule offenbart. Die DE 4338858 C1 lehrt die Verwendung eines Speichers worin das Plasma während der Regeneration der Apheresesäule zwischengespeichert wird. Die Regeneration der Apheresesäule findet über die aus dem Stand der Technik bekannte Kombination von Glyzin, NaCI-Lösung und PBS statt. Folglich enthält die Lehre der DE 4338858 C1 keinen Hinweis bezüglich der Verwendung der Antikoagulationslösung bzw. der Spüllösung des Plasmaseparators als Regenerationslösung für die Apheresesäule, gemäß der erfindungsgemäßen Vorrichtung. Ferner offenbart die DE 4338858 C1 auch keine Bypass-Leitung die eine Umleitung des Plasmaflusses unter Umgehung der Apheresesäule, während der Regeneration derselben ermöglicht.

**Aufgabe der vorliegenden Erfindung** ist es, eine Vorrichtung zur vereinfachten Regeneration von Apheresesäulen bereitzustellen, wobei die angeführten Nachteile der aus dem Stand der Technik bekannten Vorrichtungen minimiert werden. Anders formuliert besteht die Aufgabe der vorliegenden Erfindung darin eine Vorrichtung zur vereinfachten Regeneration von Apheresesäulen zur Verfügung zu stellen, welche mit reduziertem Trainingsaufwand, und damit verbunden mit einem reduzierten Personalaufwand und reduzierten Gesamtkosten bedient werden können.

Diese Aufgabe wird durch die Lehre der unabhängigen Ansprüche gelöst. Weitere vorteilhafte Ausgestaltungen ergeben sich aus der Beschreibung, den Beispielen und den anhängigen Patentansprüchen.

Überraschend wurde gefunden, dass durch die Bereitstellung einer Regenerationsleitung die entweder direkt in die Apheresesäule mündet oder vor der Apheresesäule aber nach der Bypass-Leitung in die Plasmaleitung mündet eine vereinfachte Regeneration der Apheresesäule ermöglicht wird, wobei die Nachteile aus dem bekannten Stand der Technik minimiert werden.

### Beschreibung der Erfindung

Die vorliegende Erfindung betrifft eine Apheresevorrichtung (1) zur extrakorporalen Entfernung von CRP aus Blut eines Patienten, wobei die Apheresevorrichtung an den Blutkreislauf des Patienten angeschlossen wird. Das Blut wird über einen Teil des extrakorporalen Zirkulationssystems (2) der erfindungsgemäßen Apheresevorrichtung (1) zu einem Zellseparator (7) zur Auftrennung des Bluts in Blutplasma und zelluläre Bestandteile gepumpt. Über einen ersten Ausgang des Zellseparators (7) wird das abgetrennte Blutplasma über eine Plasmaleitung (8A) zu einer Apheresesäule (4) zur affinitätschromatographischen Entfernung von CRP aus dem Blutplasma geleitet. Nach der Entfernung des CRPs aus dem Blutplasma des Patienten wird dieses nun behandelte Blutplasma über eine Plasmaleitung (8B) mit den zellulären Bestandteilen des Bluts zusammengeführt. Des Weiteren umfasst die erfindungsgemäße Apheresevorrichtung (1) eine Bypass-Leitung (12), die von der Plasmaleitung (8A) in die Plasmaleitung (8B) führt unter Umgehung der Apheresesäule (4). Ferner umfasst die erfindungsgemäße Apheresevorrichtung (1) eine Regenerationsleitung (14), die in Flussrichtung nach der Bypass-Leitung (12) in die Plasmaleitung (8A) oder direkt in die Apheresesäule (4) mündet.

Zusätzlich umfasst die vorliegende Erfindung ein Verfahren zu vereinfachten Regeneration einer Apheresesäule.

### Vorrichtung

Die vorliegende Erfindung betrifft eine Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7), eine Plasmaleitung (8A) von dem Zellseparator (7) zur Apheresesäule (4), eine Plasmaleitung (8B) für CRP-abgereichertes Blutplasma von der Apheresesäule (4) bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (1),
zumindest eine Anschlussleitung (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4) mündet.

Wie bereits oben erläutert, wird die erfindungsgemäße Apheresevorrichtung (1) zur extrakorporalen Entfernung von CRP aus Blut an den Blutkreislauf eines Patienten angeschlossen. Von einem Gefäßzugang am Patienten (in der Regel ein venöser Zugang) wird das Blut über einen Teil des extrakorporalen Zirkulationssystems (2) der vorliegenden Erfindung zu einem Zellseparator (7) gepumpt. Der Teil des extrakorporalen Zirkulationssystems (2), der das Blut aus dem Patienten und zu dem Zellseparator (7) leitet, leitet das Blut vom Patienten und damit auch von dessen Herzen weg und wird daher in Anlehnung an die Gefäßnomenklatur im menschlichen Körper als **"arterielle Leitung"** (5) bezeichnet.

Durch einen Eingang des Zellseparators (7) wird das Blut des Patienten in den Zellseparator (7) geleitet und dort von diesem in Blutplasma (zum Teil auch einfach als "Plasma" bezeichnet) und die zellulären Bestandteile des Bluts getrennt. Hierbei muss berücksichtigt werden, dass die Trennung in Blutplasma und zelluläre Bestandteile nicht vollständig erfolgt, sondern lediglich vorzugsweise 10 bis 90 % des gesamten Blutplasmas von den zellulären Bestandteilen getrennt wird. Über einen ersten Ausgang des Zellseparators (7) wird das abgetrennte Blutplasma über eine Plasmaleitung (8A) zu der Apheresesäule (4) zur affinitätschromatographischen Entfernung von CRP aus dem Blut (bzw. aus dem Blutplasma) geleitet. Nach der Entfernung bzw. der Reduzierung des CRPs im Blutplasma des Patienten wird dieses nun behandelte Blutplasma (auch als **"abgereichertes Blutplasma"** bezeichnet) über eine Plasmaleitung (8B) zu dem Punkt (P1) geleitet. Über einen zweiten Ausgang des Zellseparators (7) und eine sich anschließende Leitung (die so genannte Zellleitung (9)) werden die zellulären Bestandteile des Bluts an der Apheresesäule (4) vorbeigeführt und zum Punkt (P1) geleitet. Dort werden die zellulären Bestandteile mit dem abgereicherten Blutplasma zusammengeführt. Nach der Vereinigung der zellulären Bestandteile mit dem abgereicherten Blutplasma wird das nun behandelte Blut über einen weiteren Teil des extrakorporalen Zirkulationssystems (2) der vorliegenden Erfindung zurück zum Patienten geführt. Der Teil des extrakorporalen Zirkulationssystems (2), der das behandelte Blut von dem Punkt (P1) des extrakorporalen Zirkulationssystems (2) zurück zum Patienten leitet, leitet das Blut zum Patienten und damit auch zu dessen Herzen und wird daher in Anlehnung an die Gefäßnomenklatur im menschlichen Körper als **"venöse Leitung"** (6) bezeichnet.

In einer alternativen Ausführungsform der vorliegenden Erfindung ist es möglich, dass die zellulären Bestandteile auch direkt nach der Abtrennung vom Plasma dem Patienten wieder über den zweiten Ausgang am Zellseparator und eine sich anschließende Leitung zugeführt werden und lediglich das behandelte Plasma dem Patienten über die venöse Leitung zugeführt wird.

Um die Gerinnung des Bluts im extrakorporalen Zirkulationssystem verhindern zu können oder um ein Durchspülen bzw. Vorspülen des extrakorporalen Zirkulationssystems (z.B. mit einer physiologischen Salzlösung) zu ermöglichen, umfasst die erfindungsgemäße Apheresevorrichtung zumindest eine Leitung (die so genannte Anschlussleitung (11)), die den Anschluss von zumindest einem Flüssigkeitsbehälter (F) und somit die Einspeisung der in diesem zumindest einen Flüssigkeitsbehälter (F) befindlichen Flüssigkeit (z.B. ein Antikoagulanz oder eine physiologische Salzlösung) in das extrakorporale Zirkulationssystem ermöglicht. In diesem Zusammenhang spricht man auch davon, dass die Anschlussleitung (11) zum Anschluss von zumindest einem Flüssigkeitsbehälter (F) in fluidtechnischer Verbindung mit dem extrakorporalen Zirkulationssystem steht, d.h. eine Flüssigkeit aus einem Flüssigkeitsbehälter über die Anschlussleitung (11) in das extrakorporale Zirkulationssystem eingeleitet werden kann. In bevorzugten Ausführungsformen der vorliegenden Erfindung mündet die zumindest eine Anschlussleitung (11) vor dem Zellseparator (7) in das extrakorporale Zirkulationssystem (2), d.h. in die arterielle Leitung (5), oder direkt in den Zellseparator (7).

Es ist für den Fachmann offensichtlich, dass der oder die Flüssigkeitsbehälter (F) selbst nicht zu der erfindungsgemäßen Apheresevorrichtung gehören müssen, da es sich hierbei in der Regel um Einweg-Artikel, z.B. in Form von gängigen Infusionsbeuteln handelt, die von dem Bedienpersonal (z.B. dem behandelnden Arzt oder einer Krankenschwester) auf die konkrete Anwendung abgestimmt an die Anschlussleitung angeschlossen werden.

Erfindungsgemäß ist das Vorhandensein einer einzelnen Anschlussleitung (11) zum Anschluss eines Flüssigkeitsbehälters möglich. Es ist aber ebenso denkbar, dass eine einzelne Anschlussleitung (11) vorhanden ist, an die zwei oder drei oder bevorzugt mehrere Flüssigkeitsbehälter angeschlossen werden können. Ebenso sind Ausführungsformen der erfindungsgemäßen Apheresevorrichtung mit zwei, bevorzugt drei oder bevorzugt mehreren Anschlussleitungen (11', 11", 11''', etc.) zum Anschluss von jeweils zumindest einem Flüssigkeitsbehälter möglich, wobei hierbei bevorzugt ist, dass diese zwei, bevorzugt drei oder bevorzugt mehrere Anschlussleitungen unabhängig voneinander in die arterielle Leitung (5) oder direkt in den Zellseparator (7) münden können. "Unabhängig voneinander" bedeutet in diesem Zusammenhang zum Beispiel, dass bei einer Ausführungsform der erfindungsgemäßen Apheresevorrichtung mit zwei Anschlussleitungen (11', 11") die eine Anschlussleitung (11') in die arterielle Leitung (5) münden und die andere Anschlussleitung (11") direkt in den Zellseparator (7) münden kann, aber auch dass beide Anschlussleitungen (11', 11") in die arterielle Leitung (5) münden können oder dass beide Anschlussleitungen (11', 11") direkt in den Zellseparator (7) münden können.

Gemäß einer Ausführungsform der vorliegenden Erfindung ist es besonders bevorzugt, wenn die erfindungsgemäße Apheresevorrichtung (1) zwei Anschlussleitungen (11', 11") zum Anschluss von jeweils zumindest einem Flüssigkeitsbehälter aufweist, wobei die Anschlussleitungen (11', 11") unabhängig voneinander in die arterielle Leitung (5) oder direkt in den Zellseparator (7) münden. Folglich münden beide Anschlussleitungen (11', 11") in die arterielle Leitung (5) oder beide Anschlussleitungen (11', 11") münden direkt in den Zellseparator (7) oder besonders bevorzugt mündet die eine Anschlussleitung (11') in die arterielle Leitung (5) und die andere Anschlussleitung (11") direkt in den Zellseparator (7). Hierdurch ist es möglich, dass die zwei Anschlussleitungen (11', 11") an unterschiedliche Flüssigkeitsbehälter angeschlossen werden können. Besonders bevorzugt ist, wenn eine der beiden Anschlussleitungen (z.B. 11') an einen Flüssigkeitsbehälter enthaltend eine physiologische Salzlösung (z.B. NaCI-Lösung) angeschlossen wird, während die zweite der beiden Anschlussleitungen (z.B. 11") an einen Flüssigkeitsbehälter enthaltend eine Citrat-Lösung angeschlossen wird.

Somit ist besonders bevorzugt, wenn die Apheresevorrichtung (1) eine Anschlussleitung (11') für den Anschluss eines Flüssigkeitsbehälters (F1) und eine Anschlussleitung (11") für den Anschluss eines Flüssigkeitsbehälters (F2) aufweist und die Anschlussleitung (11') in die arterielle Leitung (5) oder in den Zellseparator (7) mündet und die Anschlussleitung (11") in die arterielle Leitung (5) oder in den Zellseparator (7) oder in die Anschlussleitung (11') und damit letztendlich auch in die arterielle Leitung (5) oder in den Zellseparator (7) mündet.

Die vorliegende Erfindung betrifft daher ebenfalls eine Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7) zur Auftrennung des Bluts in Blutplasma und zelluläre Bestandteile, eine Plasmaleitung (8A) von dem Zellseparator (7) zur Auftrennung des Bluts in Blutplasma und zelluläre Bestandteile zur Apheresesäule (4) zur affinitätschromatographischen Entfernung von CRP, eine Plasmaleitung (8B) für CRP abgereichertes Blutplasma von der Apheresesäule (4) bis zu einem Punkt (P1), eine Zellleitung (9) für die abgetrennten zellulären Bestandteile von dem Zellseparator (7) zur Auftrennung des Bluts in Blutplasma bis zu dem Punkt (P1) und eine venöse Leitung (6) ausgehend von dem Punkt (P1) umfasst,
eine zentrale Recheneinheit (10) zur Steuerung der Apheresevorrichtung (1),
zwei Anschlussleitungen (11', 11") zum Anschluss von jeweils zumindest einem Flüssigkeitsbehälter an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht, und
zumindest eine Regenerationsleitung (14) in Flussrichtung nach der Abzweigung der Bypass-Leitung (12) zu der Plasmaleitung (8A) oder direkt in die Apheresesäule (4) führt.

Ein wesentlicher Vorteil der erfindungsgemäßen Apheresevorrichtung besteht darin, dass die in ihrer Reinigungskapazität naturgemäß begrenzte Apheresesäule **im laufenden Betrieb regeneriert** werden kann, d.h. ohne dass die Blutabnahme und -zufuhr oder der Zellseparator gestoppt werden müssen. Hierzu existiert eine Bypass-Leitung (12, auch als "Shunt" bezeichnet), die eine Umleitung des Plasmaflusses unter Umgehung der Apheresesäule (4) ermöglicht. Diese Bypass-Leitung (12) ermöglicht eine temporäre Entkopplung der Apheresesäule (4) von dem Plasmafluss und damit die Regeneration der Apheresesäule (4) ohne dass hierbei der Blut- bzw. Blutplasmafluss in der erfindungsgemäßen Vorrichtung unterbrochen werden muss. Die Bypass-Leitung zweigt von der Plasmaleitung (8A) ab, wobei der Punkt in der Plasmaleitung (8A) von dem die Bypass-Leitung abzweigt als Punkt (P2) bezeichnet wird, und mündet vorzugsweise in die Plasmaleitung (8B), wobei der Punkt in der Plasmaleitung (8B) in den die Bypass-Leitung (12) mündet als Punkt (P6) bezeichnet wird. In einer ebenso möglichen Ausführungsform mündet die Bypass-Leitung (12) nicht in die Plasmaleitung (8B), sondern in die Zellleitung (9), wobei der Punkt in der Zellleitung (9) in den die Bypass-Leitung (12) mündet als Punkt (P3) bezeichnet wird.

Die für die Regeneration der Apheresesäule notwendige Regenerationslösung wird über die Regenerationsleitung (14) in das extrakorporale Zirkulationssystem (2) eingespeist, wobei die Regenerationsleitung (14) entweder direkt in die Apheresesäule (4) mündet oder (in Flussrichtung) vor der Apheresesäule (4) aber (in Flussrichtung) nach der Abzweigung der Bypass-Leitung, also nach dem Punkt (P2) in die Plasmaleitung (8A) mündet.

Damit die Regenerationslösung nach dem Durchfließen der Apheresesäule (4) aus dem System entfernt werden kann (und nicht dem Patienten zugeführt wird), existiert die Abfallleitung (13), die von der Plasmaleitung (8B) abzweigt, wobei der Punkt in der Plasmaleitung (8B) von der die Abfallleitung (13) abzweigt als Punkt (P4) bezeichnet wird. In Ausführungsformen, in denen die Bypass-Leitung (12) in die Zellleitung (9) mündet, liegt der Punkt (P4) vorzugweise in einem Bereich von der Apheresesäule (4) bis zu dem Punkt (P1). In Ausführungsformen, in denen die Bypass-Leitung (12) in die Plasmaleitung (8B) mündet, liegt der Punkt (P4) vorzugweise in einem Bereich von der Apheresesäule (4) bis zu dem Punkt (P6). An besagte Abfallleitung (13) kann natürlich z.B. ein Sammelbehälter angeschlossen werden. Als Regenerationslösung kann beispielsweise eine physiologische Natriumchlorid-Lösung, TRIS-Glycin-Lösung, oder eine Citrat-Lösung verwendet werden.

Zusätzlich zur Regenerationslösung kann auch noch eine Spüllösung eingesetzt werden. Die Spüllösung kann, muss aber nicht zur Regeneration der Apheresesäule (4) dienen, sondern hat vorrangig die Aufgabe, das Blutplasma aus der Plasmaleitung (8A) in dem Bereich von Punkt P2 bis zur Apheresesäule (4), aus der Apheresesäule (4) sowie aus der Plasmaleitung (8B) von der Apheresesäule (4) bis zum Punkt P4 zu verdrängen, bevor die Regenerationslösung eingesetzt wird, welche nach Durchfluss durch die Apheresesäule (4) dann über die Abfallleitung (13) verworfen wird. Die Spüllösung kann hingegen zumindest teilweise als auch vollständig dem Patienten zugeführt werden und muss nicht verworfen werden, zumindest so lange nicht, wie in der Spüllösung noch keine Regenerationslösung enthalten ist. Als Spüllösung wird vorzugsweise eine physiologische NaCI-Lösung verwendet. Noch bevorzugter ist, als Spüllösung eine physiologische NaCI-Lösung zu verwenden, wenn als Regenerationslösung eine Citrat-Lösung eingesetzt wird.

Die vorliegende Erfindung betrifft daher ebenso eine Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7) zur Auftrennung des Bluts in Blutplasma und zelluläre Bestandteile, eine Plasmaleitung (8A) von dem Zellseparator (7) zur Auftrennung des Bluts in Blutplasma und zelluläre Bestandteile zur Apheresesäule (4) zur affinitätschromatographischen Entfernung von CRP, eine Plasmaleitung (8B) für CRP-abgereichertes Blutplasma von der Apheresesäule (4) bis zu einem Punkt (P1), eine Zellleitung (9) für die abgetrennten zellulären Bestandteile von dem Zellseparator (7) zur Auftrennung des Bluts in Blutplasma bis zu dem Punkt (P1) und eine venöse Leitung (6) ausgehend von dem Punkt (P1) umfasst,
eine zentrale Recheneinheit (10) zur Steuerung der Apheresevorrichtung (1),
zumindest eine Anschlussleitung (11) zum Anschluss von zumindest einem Flüssigkeitsbehälter an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht, und
zumindest eine Regenerationsleitung (14) in einem Bereich von der Abzweigung der Bypass-Leitung (12) an der Plasmaleitung (8A) bis zur Apheresesäule (4) in das extrakorporale Zirkulationssystem (2) mündet.

Ebenso ist die vorliegende Erfindung gerichtet auf eine Apheresevorrichtung (1) zur extrakorporalen Entfernung von CRP aus Blut oder Blutplasma umfassend:
ein extrakorporales Zirkulationssystem (2) für Blut oder Blutplasma anschließbar an den Blutkreislauf eines Patienten,
Mittel (3) zur Generierung und Regulation eines Flusses von Blut oder Blutplasma in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut oder Blutplasma,
wobei das extrakorporale Zirkulationssystem (2) eine arterielle Leitung (5) vom Patienten zu dem Zellseparator (7),
eine von dem Zellseparator (7) ausgehende Plasmaleitung (8A) für das abgetrennte Blutplasma in fluidtechnischer Verbindung mit der Apheresesäule (4) zur affinitätschromatographischen Entfernung von CRP,
eine von der Apheresesäule (4) ausgehende Plasmaleitung (8B) für das CRP-abgereicherte Blutplasma,
eine von dem Zellseparator (7) ausgehende Zellleitung (9) für die abgetrennten zellulären Bestandteile, die an einem Punkt (P1) in die Plasmaleitung (8B) mündet, und
eine venöse Leitung (6) von dem Punkt (P1) zum Patienten umfasst;
eine zentrale Recheneinheit (10) zur Steuerung der Apheresevorrichtung (1),
zumindest eine Anschlussleitung (11) zum Anschluss von zumindest einem Flüssigkeitsbehälter in fluidtechnischer Verbindung mit dem extrakorporalen Zirkulationssystem (2),
dadurch gekennzeichnet, dass die Apheresevorrichtung (1) des Weiteren umfasst:
   eine Bypass-Leitung (12), die von einem Punkt (P2) in der Plasmaleitung (8A) zu einem Punkt (P3) in der Zellleitung (9) oder zu einem Punkt (P6) in der Plasmaleitung (8B) führt, eine Abfallleitung (13), die von einem Punkt (P4) in der Plasmaleitung (8B) abzweigt,
   zumindest eine Regenerationsleitung (14), die in einem Bereich von Punkt (P2) bis zur Apheresesäule (4) in das extrakorporale Zirkulationssystem (2) mündet,
   und der Punkt (P4) vor den Punkten (P1) und (P6) angeordnet ist oder Punkt (P4) mit Punkt (P6) zusammenfällt.

Ebenso ist die vorliegende Erfindung gerichtet auf eine Apheresevorrichtung (1) zur extrakorporalen Entfernung von CRP aus Blut oder Blutplasma umfassend:
ein extrakorporales Zirkulationssystem (2) für Blut oder Blutplasma anschließbar an den Blutkreislauf eines Patienten,
Mittel (3) zur Generierung und Regulation eines Flusses von Blut oder Blutplasma in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut oder Blutplasma,
wobei das extrakorporale Zirkulationssystem (2) eine arterielle Leitung (5) vom Patienten zu dem Zellseparator (7),
eine von dem Zellseparator (7) ausgehende Plasmaleitung (8A) für das abgetrennte Blutplasma in fluidtechnischer Verbindung mit der Apheresesäule (4) zur affinitätschromatographischen Entfernung von CRP,
eine von der Apheresesäule (4) ausgehende Plasmaleitung (8B) für das CRP-abgereicherte Blutplasma,
eine von dem Zellseparator (7) ausgehende Zellleitung (9) für die abgetrennten zellulären Bestandteile, die an einem Punkt (P1) in die Plasmaleitung (8B) mündet, und
eine venöse Leitung (6) von dem Punkt (P1) zum Patienten umfasst;
eine zentrale Recheneinheit (10) zur Steuerung der Apheresevorrichtung (1),
zwei Anschlussleitungen (11', 11") zum Anschluss von jeweils zumindest einem Flüssigkeitsbehälter in fluidtechnischer Verbindung mit dem extrakorporalen Zirkulationssystem (2),
dadurch gekennzeichnet, dass die Apheresevorrichtung (1) des Weiteren umfasst:
   eine Bypass-Leitung (12), die von einem Punkt (P2) in der Plasmaleitung (8A) zu einem Punkt (P3) in der Zellleitung (9) oder zu einem Punkt (P6) in der Plasmaleitung (8B) führt, eine Abfallleitung (13), die von einem Punkt (P4) in der Plasmaleitung (8B) abzweigt,
   zumindest eine Regenerationsleitung (14), die in einem Bereich von Punkt (P2) bis zur Apheresesäule (4) in das extrakorporale Zirkulationssystem (2) mündet,
   und der Punkt (P4) vor den Punkten (P1) und (P6) angeordnet ist oder Punkt (P4) mit Punkt (P6) zusammenfällt.

Gemäß einer Ausführungsform der vorliegenden Erfindung ist es daher bevorzugt, wenn die erfindungsgemäße Apheresevorrichtung (1) zumindest zwei Anschlussleitungen (11) zum Anschluss von jeweils zumindest einem Flüssigkeitsbehälter (F) an die arterielle Leitung (5) oder den Zellseparator (7) aufweist.

Ferner sind Ausführungsformen der Apheresevorrichtung (1) bevorzugt, bei denen die Apheresevorrichtung (1) zumindest zwei Anschlussleitungen (11) zum Anschluss von jeweils zumindest einem Flüssigkeitsbehälter (F) an die arterielle Leitung (5) oder den Zellseparator (7) aufweist, und wobei je Flüssigkeitsbehälter (F) eine Regenerationsleitung (14) existiert, die von dem jeweiligen Flüssigkeitsbehälter (F) oder dessen Anschlussleitung (11) abgehen und die jeweils in die Plasmaleitung (8A) oder direkt in die Apheresesäule (4) führen.
Möglich ist zudem, dass sich die zumindest zwei Anschlussleitungen (11) vor ihrer Einmündung vereinen, d.h. zu einer Leitung zusammenlaufen. Möglich ist auch, dass sich die Regenerationsleitungen (14) vor ihrer Einmündung vereinen, d.h. zu einer Leitung zusammenlaufen.

Wird in der vorliegenden Anmeldung beschrieben, dass ein Vorrichtungsmerkmal in einem Bereich von einer ersten Position in der Vorrichtung bis zu einer zweiten Position in der Vorrichtung liegt oder in diesen Bereich mündet oder von diesem Bereich abzweigt, so ist dies derart zu verstehen, dass sowohl die erste Position als auch die zweite Position und der dazwischen liegende Anschnitt von diesem Bereich eingeschlossen wird. Dies soll an dem folgenden Beispiel verdeutlicht werden: Die Aussage, dass die "Regenerationsleitung (14) in einem Bereich von Punkt (P2) bis zur Apheresesäule (4) in das extrakorporale Zirkulationssystem (2) mündet" bedeutet, dass die Regenerationsleitung (14) in einen Bereich des extrakorporalen Zirkulationssystems (2) mündet, der nicht nur den Abschnitt zwischen Punkt (P2) und der Apheresesäule (4) umfasst, sondern auch den Punkt (P2) selbst auch als die Apheresesäule (4) einschließt. D.h. die Regenerationsleitung (14) kann in Punkt (P2) münden oder in die Apheresesäule (4) oder auch in den Abschnitt der Plasmaleitung (8A), der zwischen dem Punkt (P2) und der Apheresesäule (4) liegt.

Der Punkt (**P1**) ist der Knotenpunkt im extrakorporalen Zirkulationssystem (2), an dem die Plasmaleitung (8B) in die venöse Leitung (6) übergeht. Der Punkt (**P2**) ist der Knotenpunkt im extrakorporalen Zirkulationssystem (2), an dem die Bypass-Leitung (12) von der Plasmaleitung (8A) abzweigt. Der Punkt (**P3**) ist der Knotenpunkt im extrakorporalen Zirkulationssystem (2), an dem die Bypass-Leitung (12) in die Zellleitung (9) mündet. Der Punkt (**P4**) ist der Knotenpunkt im extrakorporalen Zirkulationssystem (2), an dem die Abfallleitung (13) von der Plasmaleitung (8B) abzweigt. Der Punkt (**P5**) ist der Knotenpunkt im extrakorporalen Zirkulationssystem (2), an dem die Regenerationsleitung (15) in die Anschlussleitung (11) mündet. Der Punkt (**P6**) ist der Knotenpunkt im extrakorporalen Zirkulationssystem (2), an dem die Bypass-Leitung (12) in die Plasmaleitung (8B) mündet.

Ebenso ist die vorliegende Erfindung gerichtet auf eine Apheresevorrichtung (1) zur extrakorporalen Entfernung von CRP aus Blut oder Blutplasma umfassend:
ein extrakorporales Zirkulationssystem (2) für Blut oder Blutplasma anschließbar an den Blutkreislauf eines Patienten,
Mittel (3) zur Generierung und Regulation eines Flusses von Blut oder Blutplasma in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut oder Blutplasma,
wobei das extrakorporale Zirkulationssystem (2) eine arterielle Leitung (5) vom Patienten zu dem Zellseparator (7),
eine von dem Zellseparator (7) ausgehende Plasmaleitung (8A) für das abgetrennte Blutplasma in fluidtechnischer Verbindung mit der Apheresesäule (4) zur affinitätschromatographischen Entfernung von CRP,
eine von der Apheresesäule (4) ausgehende Plasmaleitung (8B) für das CRP-abgereicherte Blutplasma,
eine von dem Zellseparator (7) ausgehende Zellleitung (9) für die abgetrennten zellulären Bestandteile, die an einem Punkt (P1) in die Plasmaleitung (8B) mündet, und
eine venöse Leitung (6) von dem Punkt (P1) zum Patienten umfasst;
eine zentrale Recheneinheit (10) zur Steuerung der Apheresevorrichtung (1),
zumindest eine Anschlussleitung (11) zum Anschluss von zumindest einem Flüssigkeitsbehälter an die arterielle Leitung (5) oder den Zellseparator (7),
dadurch gekennzeichnet, dass die Apheresevorrichtung (1) des Weiteren umfasst:
   eine Bypass-Leitung (12), die von einem Punkt (P2) in der Plasmaleitung (8A) zu einem Punkt (P3) in der Zellleitung (9) oder zu einem Punkt (P6) in der Plasmaleitung (8B) führt, eine Abfallleitung (13), die von einem Punkt (P4) in der Plasmaleitung (8B) abzweigt,
   zumindest eine Regenerationsleitung (14), die nach dem Punkt (P2) in die Plasmaleitung (8A) oder direkt in Apheresesäule (4) mündet,
   und der Punkt (P4) vor den Punkten (P1) und (P6) angeordnet ist oder Punkt (P4) mit Punkt (P6) zusammenfällt.

Gemäß einer bevorzugten Ausführungsform der vorliegenden Erfindung mündet die Anschlussleitung (11) in die arterielle Leitung (5). Gemäß einer weiteren bevorzugten Ausführungsform der vorliegenden Erfindung mündet die Anschlussleitung (11) direkt in den Zellseparator (7).

Wie bereits ausgeführt, umfasst die erfindungsgemäße Apheresevorrichtung zumindest eine Leitung (die so genannte Regenerationsleitung (14)), die die Einspeisung einer Regenerationslösung (z.B. eine Citrat-Lösung, eine TRIS-Glycin-Lösung oder eine NaCl-Lösung) in das extrakorporale Zirkulationssystem bevorzugt kurz vor der Apheresesäule (4) oder direkt in die Apheresesäule (4) ermöglicht. In diesem Zusammenhang spricht man auch davon, dass die Regenerationsleitung (14) zum Anschluss von zumindest einem Flüssigkeitsbehälter (F) in fluidtechnischer Verbindung mit dem extrakorporalen Zirkulationssystem steht, d.h. eine Flüssigkeit aus einem Flüssigkeitsbehälter über die Regenerationsleitung in das extrakorporale Zirkulationssystem eingeleitet werden kann.

Gemäß einer bevorzugten Ausführungsform der vorliegenden Erfindung mündet die Regenerationsleitung (14) nach Punkt (P2) in die Plasmaleitung (8A), d.h. zwischen Punkt (P2) und der Apheresesäule (4). Gemäß einer weiteren bevorzugten Ausführungsform der vorliegenden Erfindung mündet die Regenerationsleitung (14) an Punkt (P2) in die Plasmaleitung (8A). Gemäß einer weiteren bevorzugten Ausführungsform der vorliegenden Erfindung mündet die Regenerationsleitung (14) direkt in die Apheresesäule (4).

Es ist für den Fachmann offensichtlich, dass ein Flüssigkeitsbehälter (F) zum Anschluss an die Regenerationsleitung selbst nicht zu der erfindungsgemäßen Apheresevorrichtung gehören muss, da es sich hierbei in der Regel um Einweg-Artikel, z.B. in Form von gängigen Infusionsbeuteln, handelt, die von dem Bedienpersonal (z.B. dem behandelnden Arzt oder einer Krankenschwester) auf die konkrete Anwendung abgestimmt an die Anschlussleitung angeschlossen werden.

Erfindungsgemäß ist das Vorhandensein einer einzelnen Regenerationsleitung (14) zum Anschluss eines Flüssigkeitsbehälters (F) möglich. Hierbei ist zum Beispiel denkbar, dass an die Regenerationsleitung (14) ein separater Flüssigkeitsbehälter, z.B. ein Infusionsbeutel mit NaCI-Lösung angeschlossen werden kann. Es ist aber ebenso denkbar, dass das Ende der Regenerationsleitung (14), das den Anschluss eines Flüssigkeitsbehälters ermöglicht, in räumlicher Nähe zu dem Ende einer Anschlussleitung (11), das den Anschluss eines Flüssigkeitsbehälters ermöglicht, liegt, so dass ein Flüssigkeitsbehälter (mit mindestens zwei Anschlussmöglichkeiten bzw. einem entsprechender Adapter) sowohl an die Anschlussleitung (11) als auch an die Regenerationsleitung (14) angeschlossen werden kann.

Erfindungsgemäß ist das Vorhandensein einer einzelnen Regenerationsleitung (14) möglich und besonders bevorzugt sind 1 oder 2 Regenerationsleitungen. Ebenso sind Ausführungsformen der erfindungsgemäßen Apheresevorrichtung mit zwei, drei oder mehreren Regenerationsleitungen (14', 14", 14''', etc.) möglich, wobei hierbei diese zwei, drei oder mehrere Regenerationsleitungen unabhängig voneinander in einem Bereich von der Abzweigung der Bypass-Leitung (12) an der Plasmaleitung (8A) (d.h. vom Punkt P2) bis zur Apheresesäule (4) in das extrakorporale Zirkulationssystem (2) münden können. "Unabhängig voneinander" bedeutet in diesem Zusammenhang zum Beispiel, dass bei einer Ausführungsform der erfindungsgemäßen Apheresevorrichtung mit zwei Regenerationsleitungen (14', 14") die eine Regenerationsleitung (14') in die Plasmaleitung (8A) zwischen Punkt (P2) und der Apheresesäule (4) mündet und die andere Regenerationsleitung (14") direkt in die Apheresesäule (4) mündet, aber auch dass beide Regenerationsleitungen (14', 14") in die Plasmaleitung (8A) zwischen Punkt (P2) und der Apheresesäule (4) münden können. Auch möglich ist, dass eine Regenerationsleitung (14') in die andere Regenerationsleitung (14") mündet. Bei Vorhandensein von zwei oder mehr Regenerationsleitungen (14', 14", 14''', etc.) ist es jedoch besonders bevorzugt, wenn alle Regenerationsleitungen (14', 14", 14''', etc.) an demselben Punkt im Bereich von Punkt (P2) bis zur Apheresesäule (4) in das extrakorporale Zirkulationssystem (2) münden, noch bevorzugter wenn alle Regenerationsleitungen (14', 14", 14''', etc.) an Punkt (P2) in das extrakorporale Zirkulationssystem (2) münden.

Erfindungsgemäß ist es besonders vorteilhaft, wenn eine Anschlussleitung (11) und eine Regenerationsleitung (14) dieselbe Flüssigkeitsquelle nutzen, da so nicht nur Platz gespart wird, sondern auch der Aufwand für Bedienung und Wartung der erfindungsgemäßen Apheresevorrichtung minimiert wird. Auf diese Weise können auch bestehende Apherese-Systeme modifiziert bzw. ergänzt werden, ohne dass ein separates zusätzliches Großgerät angeschlossen werden muss. In bevorzugten Ausführungsformen der vorliegenden Erfindung zweigt daher die Regenerationsleitung (14) von der Anschlussleitung (11) ab, wobei der Punkt in der Anschlussleitung (11), von dem die Regenerationsleitung (14) abzweigt als Punkt (**P5**) bezeichnet wird.

Es ist daher gemäß einigen Ausführungsformen der vorliegenden Erfindung bevorzugt, dass die zumindest eine Regenerationsleitung (14), die in die Plasmaleitung (8A) oder direkt in die Apheresesäule (4) führt, von einem Punkt (P5) in der zumindest einen Anschlussleitung (11) ausgeht.

In Ausführungsformen in denen mehr als eine Anschlussleitung (11', 11", 11''' etc.) vorhanden ist und eine Regenerationsleitung (14) mit mehreren Anschlussleitungen (11', 11", 11''' etc.) verbunden ist, so richtet dich die Nomenklatur der Verzweigungspunkte (P5', P5", P5''' etc.) nach der Nomenklatur der Anschlussleitung (11', 11", 11''' etc.). D.h. beispielhaft, dass bei einer Regenerationsleitung (14), die in zwei vorhandene Anschlussleitungen (11', 11") mündet bzw. eine Verbindung zu diesen herstellt, der Punkt, an dem die Regenerationsleitung (14) in die Anschlussleitung (11') mündet, als Punkt (P5') bezeichnet wird und der Punkt, an dem die Regenerationsleitung (14) in die Anschlussleitung (11") mündet, als Punkt (P5") bezeichnet wird.

Bevorzugt ist eine Apheresevorrichtung (1), wobei die Apheresevorrichtung (1) zwei Anschlussleitungen (11', 11") zum Anschluss von jeweils einem Flüssigkeitsbehälter (F1, F2) an die arterielle Leitung (5) oder den Zellseparator (7) aufweist, und wobei zwei Regenerationsleitungen (14', 14") von den zwei Flüssigkeitsbehältern (F1, F2) oder den zwei Anschlussleitungen (11', 11") abgehen und in die Plasmaleitung (8A) oder direkt in die Apheresesäule (4) führen.

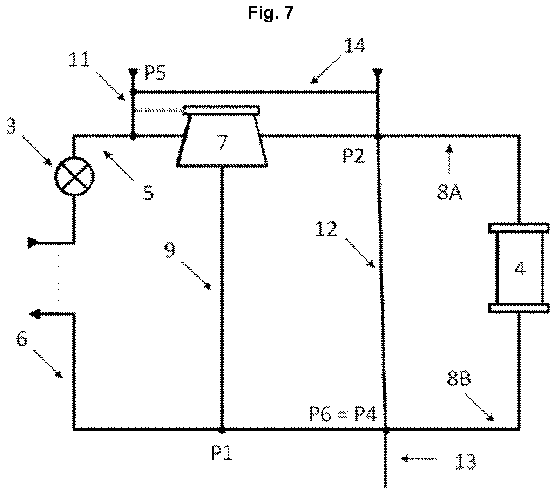

Ebenso sind Ausführungsformen denkbar, in denen eine Regenerationsleitung (14), die in die Plasmaleitung (8A) oder direkt in die Apheresesäule (4) führt und die von einem Punkt (P5) in der zumindest einen Anschlussleitung (11) ausgeht, zumindest einen zusätzlichen Anschluss für einen Flüssigkeitsbehälter aufweist (siehe Fig. 7).

Bei Ausführungsformen mit mehr Anschlussleitungen als Regenerationsleitungen, wobei jede Regenerationsleitung eine Verbindung zu mindestens einer Anschlussleitung herstellt, ist es möglich dass jede Regenerationsleitung mit jeweils einer Anschlussleitung verbunden ist und die überzählige/n Anschlussleitung/en lediglich mit der arteriellen Leitung oder dem Zellseparator verbunden sind, oder dass die zahlreicheren Anschlussleitungen auf die Regenerationsleitungen konvergieren, d.h. mehrere Anschlussleitungen sind mit einer Regenerationsleitung verbunden. Ebenso sind Mischformen möglich.

Es existieren mehrere Möglichkeiten, um die Flussraten in dem Teil der Anschlussleitung (11) nach dem Punkt (P5) und der Regenerationsleitung (14) zu regulieren. Dies könnte z.B. durch separat ansteuerbare Pumpen in dem Teil der Anschlussleitung (11) nach dem Punkt (P5) und in der Regenerationsleitung (14) geschehen. Eine andere Möglichkeit wäre eine Pumpe, die sich in der Anschlussleitung (11) vor dem Punkt (P5) befindet, wobei die Aufteilung der Flussraten nach dem Punkt (P5) entweder durch die Durchmesser von Regenerationsleitung (14) und Anschlussleitung (11) fest determiniert ist oder durch entsprechende Mittel (Klemmen, Ventile) reguliert werden kann (z.B. durch Variation des jeweiligen Leitungsdurchmessers). Die Regulation der Flussraten ist natürlich dann besonders wichtig, wenn eine Lösung (z.B. eine Citrat-Lösung) über die Anschlussleitung (11) in das System eingespeist werden muss (z.B. zur Antikoagulation des Blutes) und gleichzeitig über die Regenerationsleitung (14) in die Apheresesäule gelangen muss (zur Regeneration). Durch derartige Mechanismen kann z.B. die Einspeisung der Lösung über die Anschlussleitung (11) konstant gehalten werden (z.B. zur gleichbleibenden Antikoagulation), auch wenn phasenweise Lösung zur Regeneration der Apheresesäule über die Regenerationsleitung (14) abgezweigt wird.
Im Vergleich zu anderen Systemen kommt die Apheresevorrichtung (1) mit einer Maximalzahl von 8, vorzugsweise 7, weiter bevorzugt 6 und am meisten bevorzugt 5 Pumpen aus.

Daher ist die vorliegende Erfindung auch gerichtet auf eine Apheresevorrichtung (1) zur extrakorporalen Entfernung von CRP aus Blut oder Blutplasma umfassend:
ein extrakorporales Zirkulationssystem (2) für Blut oder Blutplasma anschließbar an den Blutkreislauf eines Patienten,
Mittel (3) zur Generierung und Regulation eines Flusses von Blut oder Blutplasma in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut oder Blutplasma,
wobei das extrakorporale Zirkulationssystem (2) eine arterielle Leitung (5) vom Patienten zu dem Zellseparator (7),
eine von dem Zellseparator (7) ausgehende Plasmaleitung (8A) für das abgetrennte Blutplasma in fluidtechnischer Verbindung mit der Apheresesäule (4) zur affinitätschromatographischen Entfernung von CRP,
eine von der Apheresesäule (4) ausgehende Plasmaleitung (8B) für das CRP-abgereicherte Blutplasma,
eine von dem Zellseparator (7) ausgehende Zellleitung (9) für die abgetrennten zellulären Bestandteile, die an einem Punkt (P1) in die Plasmaleitung (8B) mündet, und
eine venöse Leitung (6) von dem Punkt (P1) zum Patienten umfasst;
eine zentrale Recheneinheit (10) zur Steuerung der Apheresevorrichtung (1),
zumindest eine Anschlussleitung (11) zum Anschluss von zumindest einem Flüssigkeitsbehälter in fluidtechnischer Verbindung mit dem extrakorporalen Zirkulationssystem (2),
dadurch gekennzeichnet, dass die Apheresevorrichtung (1) des Weiteren umfasst:
   eine Bypass-Leitung (12), die von einem Punkt (P2) in der Plasmaleitung (8A) zu einem Punkt (P3) in der Zellleitung (9) oder zu einem Punkt (P6) in der Plasmaleitung (8B) führt, eine Abfallleitung (13), die von einem Punkt (P4) in der Plasmaleitung (8B) abzweigt,
   zumindest eine Regenerationsleitung (14), die von einem Punkt (P5) in der mindestens einen Anschlussleitung (11) ausgehend in einem Bereich von Punkt (P2) bis zur Apheresesäule (4) in das extrakorporale Zirkulationssystem (2) mündet,
   und der Punkt (P4) vor den Punkten (P1) und (P6) angeordnet ist oder im Falle, dass die Bypass-Leitung (12) in Punkt (P3) mündet, der Punkt (P1) mit dem Punkt (P4) zusammenfällt oder im Falle, dass die Bypass-Leitung (12) in Punkt (P6) mündet, der Punkt (P4) mit dem Punkt (P6) zusammenfällt.

Ebenso ist die vorliegende Erfindung gerichtet auf eine Apheresevorrichtung (1) zur extrakorporalen Entfernung von CRP aus Blut oder Blutplasma umfassend:
ein extrakorporales Zirkulationssystem (2) für Blut oder Blutplasma anschließbar an den Blutkreislauf eines Patienten,
Mittel (3) zur Generierung und Regulation eines Flusses von Blut oder Blutplasma in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut oder Blutplasma,
wobei das extrakorporale Zirkulationssystem (2) eine arterielle Leitung (5) vom Patienten zu dem Zellseparator (7),
eine von dem Zellseparator (7) ausgehende Plasmaleitung (8A) für das abgetrennte Blutplasma in fluidtechnischer Verbindung mit der Apheresesäule (4) zur affinitätschromatographischen Entfernung von CRP,
eine von der Apheresesäule (4) ausgehende Plasmaleitung (8B) für das CRP-abgereicherte Blutplasma,
eine von dem Zellseparator (7) ausgehende Zellleitung (9) für die abgetrennten zellulären Bestandteile, die an einem Punkt (P1) in die Plasmaleitung (8B) mündet, und
eine venöse Leitung (6) von dem Punkt (P1) zum Patienten umfasst;
eine zentrale Recheneinheit (10) zur Steuerung der Apheresevorrichtung (1),
zumindest eine Anschlussleitung (11) zum Anschluss von zumindest einem Flüssigkeitsbehälter an die arterielle Leitung (5) oder den Zellseparator (7),
dadurch gekennzeichnet, dass die Apheresevorrichtung (1) des Weiteren umfasst:
   eine Bypass-Leitung (12), die von einem Punkt (P2) in der Plasmaleitung (8A) zu einem Punkt (P3) in der Zellleitung (9) oder zu einem Punkt (P6) in der Plasmaleitung (8B) führt,
   eine Abfallleitung (13), die von einem Punkt (P4) in der Plasmaleitung (8B) abzweigt,
   zumindest eine Regenerationsleitung (14), die von einem Punkt (P5) in der mindestens einen Anschlussleitung (11) ausgehend nach dem Punkt (P2) in die Plasmaleitung (8A) oder direkt in Apheresesäule (4) mündet,
   und der Punkt (P4) vor den Punkten (P1) und (P6) angeordnet ist oder im Falle, dass die Bypass-Leitung (12) in Punkt (P3) mündet, der Punkt (P1) mit dem Punkt (P4) zusammenfällt oder im Falle, dass die Bypass-Leitung (12) in Punkt (P6) mündet, der Punkt (P4) mit dem Punkt (P6) zusammenfällt.

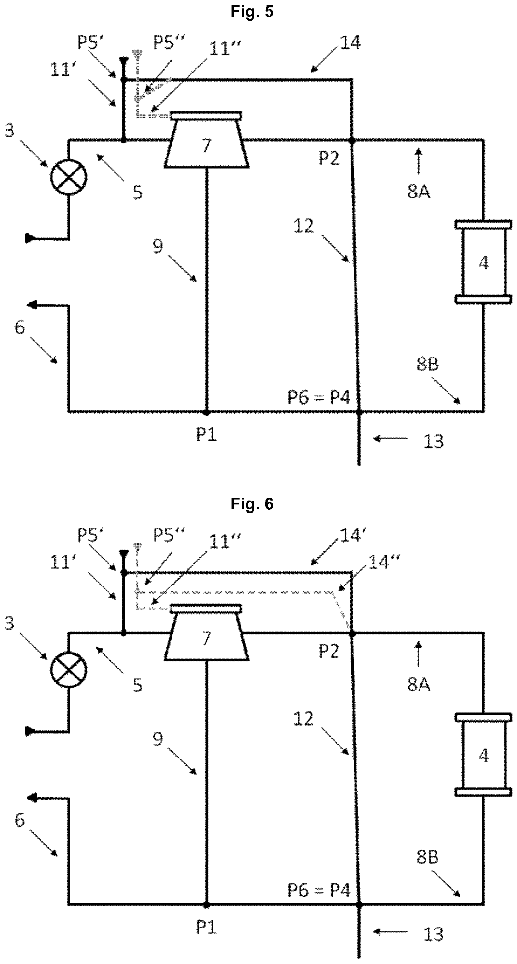

In Ausführungsformen der vorliegenden Erfindung mit mehreren Anschlussleitungen (11', 11", 11''', etc.) und mehreren Regenerationsleitungen (14', 14", 14''', etc.) ist es möglich, dass jeweils eine Anschlussleitung mit jeweils einer Regenerationsleitung in Verbindung steht, welche wiederum nach dem Punkt (P2) in die Plasmaleitung (8A) oder direkt in Apheresesäule (4) mündet. Hierbei kann jede Regenerationsleitung unabhängig von anderen Regenerationsleitungen an einer Stelle nach dem Punkt (P2) in die Plasmaleitung (8A) oder direkt in Apheresesäule (4) münden. Bevorzugt ist jedoch, wenn alle Regenerationsleitungen an derselben Stelle nach dem Punkt (P2) in die Plasmaleitung (8A) oder direkt in Apheresesäule (4) münden, noch bevorzugter direkt in die Apheresesäule (4) und am meisten bevorzugt an Punkt (P2). Eine derartige beispielhafte Ausführungsform sei anhand von Fig. 6 erläutert: Hierin besitzt die Apheresevorrichtung (1) eine erste Anschlussleitung (11'), die erstens in die arterielle Leitung (5) führt und von der zweitens am Punkt (P5') eine erste Regenerationsleitung (14') abzweigt. Die Apheresevorrichtung (1) besitzt zudem eine zweite Anschlussleitung (11"), die erstens direkt in den Zellseparator (7) führt und von der zweitens am Punkt (P5") eine zweite Regenerationsleitung (14") abzweigt. Beide Regenerationsleitungen (14', 14") münden in dieser Ausführungsform am Punkt (P2) in das extrakorporale Zirkulationssystem (2).

Bevorzugt ist demnach eine Apheresevorrichtung (1), wobei die Apheresevorrichtung (1) zwei Anschlussleitungen (11', 11") zum Anschluss von jeweils zumindest einem Flüssigkeitsbehälter (F) an die arterielle Leitung (5) oder den Zellseparator (7) aufweist, und wobei die zumindest eine Regenerationsleitung (14), die in die Plasmaleitung (8A) oder direkt in die Apheresesäule (4) führt, sich an einem Punkt (P5') mit der Anschlussleitung (11') und an einem Punkt (P5") mit der Anschlussleitung (11") verbindet.

Somit sind insbesondere Ausführungsformen der Apheresevorrichtung (1) bevorzugt, wobei die Apheresevorrichtung (1) zwei Anschlussleitungen (11', 11") zum Anschluss von jeweils zumindest einem Flüssigkeitsbehälter (F1, F2) an die arterielle Leitung (5) oder den Zellseparator (7) aufweist, und wobei die zumindest eine Regenerationsleitung (14), die in die Plasmaleitung (8A) oder direkt in die Apheresesäule (4) führt, sich an einem Punkt (P5') mit der Anschlussleitung (11') und an einem Punkt (P5") mit der Anschlussleitung (11") verbindet und wobei eine Regenerationsleitung (14') von dem Flüssigkeitsbehälter (F1) oder von der vom Flüssigkeitsbehälter (F1) abgehenden Anschlussleitung (11') zur Apheresesäule (4) oder zur Plasmaleitung (8A) führt und eine Regenerationsleitung (14") von dem Flüssigkeitsbehälter (F2) oder von der vom Flüssigkeitsbehälter (F2) abgehenden Anschlussleitung (11") zur Apheresesäule (4) oder zur Plasmaleitung (8A) oder in die Regenerationsleitung (14') führt.
Vorzugsweise enthält der Flüssigkeitsbehälter (F1) eine physiologische NaCI-Lösung und der Flüssigkeitsbehälter (F2) eine Citrat-Lösung.

Somit ist besonders bevorzugt, wenn die Apheresevorrichtung (1) eine Anschlussleitung (11') für den Anschluss eines Flüssigkeitsbehälters (F1) und eine Anschlussleitung (11") für den Anschluss eines Flüssigkeitsbehälters (F2) aufweist und die Anschlussleitung (11') in die arterielle Leitung (5) oder in den Zellseparator (7) mündet und die Anschlussleitung (11") in die arterielle Leitung (5) oder in den Zellseparator (7) oder in die Anschlussleitung (11') und damit letztendlich auch in die arterielle Leitung (5) oder in den Zellseparator (7) mündet und eine Regenerationsleitung (14') von dem Flüssigkeitsbehälter (F1) oder von der Anschlussleitung (11') zur Apheresesäule (4) oder zur Plasmaleitung (8A) führt und eine Regenerationsleitung (14") von dem Flüssigkeitsbehälter (F2) oder von der Anschlussleitung (11") zur Apheresesäule (4) oder zur Plasmaleitung (8A) oder in die Regenerationsleitung (14') führt.
Vorzugsweise enthält der Flüssigkeitsbehälter (F1) eine physiologische NaCI-Lösung und der Flüssigkeitsbehälter (F2) eine Citrat-Lösung.

Ausführungsformen der Apheresevorrichtung (1) sind daher insbesondere bevorzugt, bei denen die Apheresevorrichtung (1) eine Anschlussleitung (11') zum Anschluss von einem Flüssigkeitsbehälter (F1) an die arterielle Leitung (5) oder den Zellseparator (7) und eine Anschlussleitung (11") zum Anschluss von einem Flüssigkeitsbehälter (F2) an die arterielle Leitung (5) oder den Zellseparator (7) aufweist, und wobei eine Regenerationsleitung (14') von dem Flüssigkeitsbehälter (F1) oder der Anschlussleitung (11') abgeht und in Flussrichtung nach der Abzweigung der Bypass-Leitung (12) in die Plasmaleitung (8A) oder direkt in die Apheresesäule (4) führt und eine Regenerationsleitung (14") von dem Flüssigkeitsbehälter (F2) oder der Anschlussleitung (11") abgeht und in Flussrichtung nach der Abzweigung der Bypass-Leitung (12) in die Plasmaleitung (8A) oder in die Regenerationsleitung (14') oder direkt in die Apheresesäule (4) führt.

Besonders bevorzugt ist daher eine Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7), eine Plasmaleitung (8A) von dem Zellseparator (7) zur Apheresesäule (4), eine Plasmaleitung (8B) für CRP-abgereichertes Blutplasma von der Apheresesäule (4) bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (1),
eine Anschlussleitung (11') zum Anschluss von einem Flüssigkeitsbehälter (F1) an die arterielle Leitung (5) oder den Zellseparator (7) und eine Anschlussleitung (11") zum Anschluss von einem Flüssigkeitsbehälter (F2) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht, und eine Regenerationsleitung (14') von dem Flüssigkeitsbehälter (F1) oder der Anschlussleitung (11') abgeht und in Flussrichtung nach der Abzweigung der Bypass-Leitung (12) in die Plasmaleitung (8A) oder direkt in die Apheresesäule (4) führt und eine Regenerationsleitung (14") von dem Flüssigkeitsbehälter (F2) oder der Anschlussleitung (11") abgeht und in Flussrichtung nach der Abzweigung der Bypass-Leitung (12) in die Plasmaleitung (8A) oder in die Apheresesäule (4) führt oder sich mit der Regenerationsleitung (14') vereinigt.
Vorzugsweise handelt es sich bei dem Flüssigkeitsbehälter (F1) um einen Behälter für eine physiologische Natriumchlorid-Lösung und bei dem Flüssigkeitsbehälter (F2) um einen Behälter für eine Citrat-Lösung.

Anders ausgedrückt ist daher gemäß einer Ausführungsform eine Apheresevorrichtung (1) bevorzugt, wobei die Apheresevorrichtung (1) zwei Anschlussleitungen (11', 11") zum Anschluss von jeweils zumindest einem Flüssigkeitsbehälter an die arterielle Leitung (5) oder den Zellseparator (7) aufweist,
und wobei die zumindest eine Regenerationsleitung (14), die in die Plasmaleitung (8A) oder direkt in die Apheresesäule (4) führt, eine Verbindung an Punkt (P5') zu der Anschlussleitung (11') und an Punkt (P5") zu der Anschlussleitung (11") herstellt. Dies ist derart zu verstehen, dass die eine Regenerationsleitung (14) das Verbindungselement zwischen den Anschlussleitungen (11', 11") auf der einen Seite und der Plasmaleitung (8A) bzw. der Apheresesäule (4) auf der anderen Seite darstellt. Eine Flüssigkeit aus einem der an einer der beiden Anschlussleitungen (11', 11") angeschlossenen Flüssigkeitsbehälter (F) könnte also über die Regenerationsleitung (14) nach Punkt (P2) in die Plasmaleitung (8A) oder direkt in die Apheresesäule (4) fließen.

Daher ist die vorliegende Erfindung auch gerichtet auf eine Apheresevorrichtung (1) zur extrakorporalen Entfernung von CRP aus Blut oder Blutplasma umfassend:
ein extrakorporales Zirkulationssystem (2) für Blut oder Blutplasma anschließbar an den Blutkreislauf eines Patienten,
Mittel (3) zur Generierung und Regulation eines Flusses von Blut oder Blutplasma in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut oder Blutplasma,
wobei das extrakorporale Zirkulationssystem (2) eine arterielle Leitung (5) vom Patienten zu dem Zellseparator (7),
eine von dem Zellseparator (7) ausgehende Plasmaleitung (8A) für das abgetrennte Blutplasma in fluidtechnischer Verbindung mit der Apheresesäule (4) zur affinitätschromatographischen Entfernung von CRP,
eine von der Apheresesäule (4) ausgehende Plasmaleitung (8B) für das CRP-abgereicherte Blutplasma,
eine von dem Zellseparator (7) ausgehende Zellleitung (9) für die abgetrennten zellulären Bestandteile, die an einem Punkt (P1) in die Plasmaleitung (8B) mündet, und
eine venöse Leitung (6) von dem Punkt (P1) zum Patienten umfasst;
eine zentrale Recheneinheit (10) zur Steuerung der Apheresevorrichtung (1),
zwei Anschlussleitungen (11', 11") zum Anschluss von jeweils zumindest einem Flüssigkeitsbehälter in fluidtechnischer Verbindung mit dem extrakorporalen Zirkulationssystem (2),
dadurch gekennzeichnet, dass die Apheresevorrichtung (1) des Weiteren umfasst:
   eine Bypass-Leitung (12), die von einem Punkt (P2) in der Plasmaleitung (8A) zu einem Punkt (P3) in der Zellleitung (9) oder zu einem Punkt (P6) in der Plasmaleitung (8B) führt,
   eine Abfallleitung (13), die von einem Punkt (P4) in der Plasmaleitung (8B) abzweigt,
   zumindest eine Regenerationsleitung (14), die in einem Bereich von Punkt (P2) bis zur Apheresesäule (4) in das extrakorporale Zirkulationssystem (2) mündet und die eine Verbindung an Punkt (P5') zu der Anschlussleitung (11') und an Punkt (P5") zu der Anschlussleitung (11") herstellt,
   und der Punkt (P4) vor den Punkten (P1) und (P6) angeordnet ist oder im Falle, dass die Bypass-Leitung (12) in Punkt (P3) mündet, der Punkt (P1) mit dem Punkt (P4) zusammenfällt oder im Falle, dass die Bypass-Leitung (12) in Punkt (P6) mündet, der Punkt (P4) mit dem Punkt (P6) zusammenfällt.

Ebenso ist die vorliegende Erfindung gerichtet auf eine Apheresevorrichtung (1) zur extrakorporalen Entfernung von CRP aus Blut oder Blutplasma umfassend:
ein extrakorporales Zirkulationssystem (2) für Blut oder Blutplasma anschließbar an den Blutkreislauf eines Patienten,
Mittel (3) zur Generierung und Regulation eines Flusses von Blut oder Blutplasma in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut oder Blutplasma,
wobei das extrakorporale Zirkulationssystem (2) eine arterielle Leitung (5) vom Patienten zu dem Zellseparator (7),
eine von dem Zellseparator (7) ausgehende Plasmaleitung (8A) für das abgetrennte Blutplasma in fluidtechnischer Verbindung mit der Apheresesäule (4) zur affinitätschromatographischen Entfernung von CRP,
eine von der Apheresesäule (4) ausgehende Plasmaleitung (8B) für das CRP-abgereicherte Blutplasma,
eine von dem Zellseparator (7) ausgehende Zellleitung (9) für die abgetrennten zellulären Bestandteile, die an einem Punkt (P1) in die Plasmaleitung (8B) mündet, und
eine venöse Leitung (6) von dem Punkt (P1) zum Patienten umfasst;
eine zentrale Recheneinheit (10) zur Steuerung der Apheresevorrichtung (1),
zwei Anschlussleitungen (11', 11") zum Anschluss von zumindest einem Flüssigkeitsbehälter an die arterielle Leitung (5) oder den Zellseparator (7),
dadurch gekennzeichnet, dass die Apheresevorrichtung (1) des Weiteren umfasst:
   eine Bypass-Leitung (12), die von einem Punkt (P2) in der Plasmaleitung (8A) zu einem Punkt (P3) in der Zellleitung (9) oder zu einem Punkt (P6) in der Plasmaleitung (8B) führt, eine Abfallleitung (13), die von einem Punkt (P4) in der Plasmaleitung (8B) abzweigt,
   zumindest eine Regenerationsleitung (14), die nach dem Punkt (P2) in die Plasmaleitung (8A) oder direkt in Apheresesäule (4) mündet und die eine Verbindung an Punkt (P5') zu der Anschlussleitung (11') und an Punkt (P5") zu der Anschlussleitung (11") herstellt,
   und der Punkt (P4) vor den Punkten (P1) und (P6) angeordnet ist oder im Falle, dass die Bypass-Leitung (12) in Punkt (P3) mündet, der Punkt (P1) mit dem Punkt (P4) zusammenfällt oder im Falle, dass die Bypass-Leitung (12) in Punkt (P6) mündet, der Punkt (P4) mit dem Punkt (P6) zusammenfällt.

Gemäß einer Ausführungsform der vorliegenden Erfindung ist es bevorzugt, wenn die erfindungsgemäße Apheresevorrichtung (1) zwei Anschlussleitungen (11', 11") aufweist, wobei die erste Anschlussleitung (11') zum Anschluss von zumindest einem Flüssigkeitsbehälter (bevorzugt eine Flüssigkeitsbehälter für oder enthaltend eine NaCl-Lösung) mit der arteriellen Leitung (5) verbunden ist (d.h. fluidtechnisch verbunden) und eine zweite Anschlussleitung (11") zum Anschluss von zumindest einem Flüssigkeitsbehälter (bevorzugt eine Flüssigkeitsbehälter für oder enthaltend eine Citrat-Lösung) direkt mit dem Zellseparator (7) verbunden ist (d.h. fluidtechnisch verbunden). Zusätzlich ist hierin bevorzugt, wenn die erfindungsgemäße Apheresevorrichtung (1) eine einzelne Regenerationsleitung (14) aufweist, die nach dem Punkt (P2) in die Plasmaleitung (8A) oder direkt in Apheresesäule (4) mündet, und die jedoch sowohl von der ersten Anschlussleitung (11') als auch von der zweiten Anschlussleitung (11") ausgeht bzw. mit diesen verbunden ist (siehe Fig. 5). D.h. die Regenerationsleitung (14) ist am Punkt (P5') mit der ersten Anschlussleitung (11') und am Punkt (P5") mit der zweiten Anschlussleitung (11") verbunden und führt dann nach dem Punkt (P2) in die Plasmaleitung (8A) oder direkt in die Apheresesäule (4). Durch entsprechende Ventile oder Schlauchklemmen kann so nach Bedarf über die Regenerationsleitung (14) Flüssigkeit aus dem an die erste Anschlussleitung (11') angeschlossenen Flüssigkeitsbehälter in die Apheresesäule (4) oder nach Punkt (P2) in die Plasmaleitung (8A) geleitet werden, oder aber auch Flüssigkeit aus dem an die zweite Anschlussleitung (11") angeschlossenen Flüssigkeitsbehälter.

Derartige Ausführungsformen mit zwei (oder sogar mehr) Anschlussleitungen sind ideal dafür geeignet verschiedene Regenerationslösungen zur Regeneration der Apheresesäule (4) zu verwenden und sukzessive in die Apheresesäule (4) einzuleiten. Eine derartige Vorrichtung ist zum Beispiel ideal dafür geeignet, zunächst eine NaCI-Lösung zur Verdrängung des in der Apheresesäule enthaltenen Plasmas einzuleiten, gefolgt von einer Citrat-Lösung zur effizienten und raschen Regeneration des Adsorbers und abschließend erneut von einer NaCI-Lösung zur Verdrängung der in der Apheresesäule enthaltenen Citrat-Lösung, bevor dann wieder Plasma in die Apheresesäule eingeleitet wird.

Daher betrifft ist eine besonders bevorzugte Ausführungsform der vorliegenden Erfindung eine Apheresevorrichtung (1) zur extrakorporalen Entfernung von CRP aus Blut oder Blutplasma umfassend:
ein extrakorporales Zirkulationssystem (2) für Blut oder Blutplasma anschließbar an den Blutkreislauf eines Patienten,
Mittel (3) zur Generierung und Regulation eines Flusses von Blut oder Blutplasma in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut oder Blutplasma,
wobei das extrakorporale Zirkulationssystem (2) eine arterielle Leitung (5) vom Patienten zu dem Zellseparator (7),
eine von dem Zellseparator (7) ausgehende Plasmaleitung (8A) für das abgetrennte Blutplasma in fluidtechnischer Verbindung mit der Apheresesäule (4) zur affinitätschromatographischen Entfernung von CRP,
eine von der Apheresesäule (4) ausgehende Plasmaleitung (8B) für das CRP-abgereicherte Blutplasma,
eine von dem Zellseparator (7) ausgehende Zellleitung (9) für die abgetrennten zellulären Bestandteile, die an einem Punkt (P1) in die Plasmaleitung (8B) mündet, und
eine venöse Leitung (6) von dem Punkt (P1) zum Patienten umfasst;
eine zentrale Recheneinheit (10) zur Steuerung der Apheresevorrichtung (1),
eine erste Anschlussleitung (11') zum Anschluss von zumindest einem Flüssigkeitsbehälter an die arterielle Leitung (5) und eine zweite Anschlussleitung (11") zum Anschluss von zumindest einem Flüssigkeitsbehälter direkt an den Zellseparator (7), dadurch gekennzeichnet, dass die Apheresevorrichtung (1) des Weiteren umfasst:
   eine Bypass-Leitung (12), die von einem Punkt (P2) in der Plasmaleitung (8A) zu einem Punkt (P3) in der Zellleitung (9) oder zu einem Punkt (P6) in der Plasmaleitung (8B) führt,
   eine Abfallleitung (13), die von einem Punkt (P4) in der Plasmaleitung (8B) abzweigt,
   zumindest eine Regenerationsleitung (14), die nach dem Punkt (P2) in die Plasmaleitung (8A) oder direkt in Apheresesäule (4) mündet und die eine Verbindung an Punkt (P5') zu der Anschlussleitung (11') und an Punkt (P5") zu der Anschlussleitung (11") herstellt,
   und der Punkt (P4) vor den Punkten (P1) und (P6) angeordnet ist.

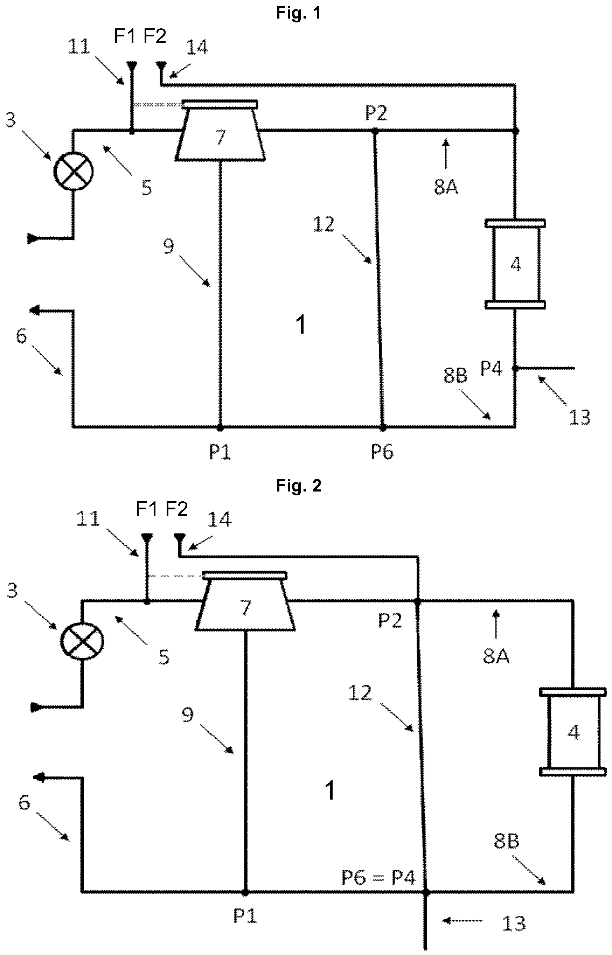

In Ausführungsformen der erfindungsgemäßen Apheresevorrichtung, in denen die Bypass-Leitung (12) zu dem Punkt (P6) in der Plasmaleitung (8B) führt, ist es bevorzugt, wenn der Punkt (P6) (in Flussrichtung) vor dem Punkt (P1) angeordnet ist (siehe **Fig. 1 - 3**).

Gemäß einer bevorzugten Ausführungsform der vorliegenden Erfindung mündet die Anschlussleitung in die arterielle Leitung. Gemäß einer weiteren bevorzugten Ausführungsform der vorliegenden Erfindung mündet die Anschlussleitung direkt in den Zellseparator.

Gemäß einer bevorzugten Ausführungsform der vorliegenden Erfindung mündet die Regenerationsleitung (14) nach Punkt (P2) in die Plasmaleitung (8A), d.h. zwischen Punkt (P2) und der Apheresesäule (4). Gemäß einer weiteren bevorzugten Ausführungsform der vorliegenden Erfindung mündet die Regenerationsleitung (14) direkt in die Apheresesäule (4).

Um das Totvolumen des Systems möglichst gering zu halten, ist es erfindungsgemäß besonders bevorzugt, wenn bei der erfindungsgemäßen Apheresevorrichtung (1) die zumindest eine Regenerationsleitung (14) am Punkt (P2) in das extrakorporale Zirkulationssystem (2) mündet. In Ausführungsformen, in denen mehr als eine Regenerationsleitung (14', 14", 14''', etc.) vorhanden ist, ist es besonders bevorzugt, wenn alle vorhandenen Regenerationsleitungen (14', 14", 14''', etc.) am Punkt (P2) in das extrakorporale Zirkulationssystem (2) münden, bzw. am Punkt (P2) in die Plasmaleitung (8A) münden.

Die vorliegende Erfindung ist daher ebenso gerichtet auf eine erfindungsgemäße Apheresevorrichtung (1), wobei die Bypass-Leitung (12) von einem Punkt (P2) in der Plasmaleitung (8A) zu einem Punkt (P6) in der Plasmaleitung (8B) führt, und die Abfallleitung (13) von einem Punkt (P4) von der Plasmaleitung (8B) abgeht, und die zumindest eine Regenerationsleitung (14) am Punkt (P2) in die Plasmaleitung (8A) mündet.

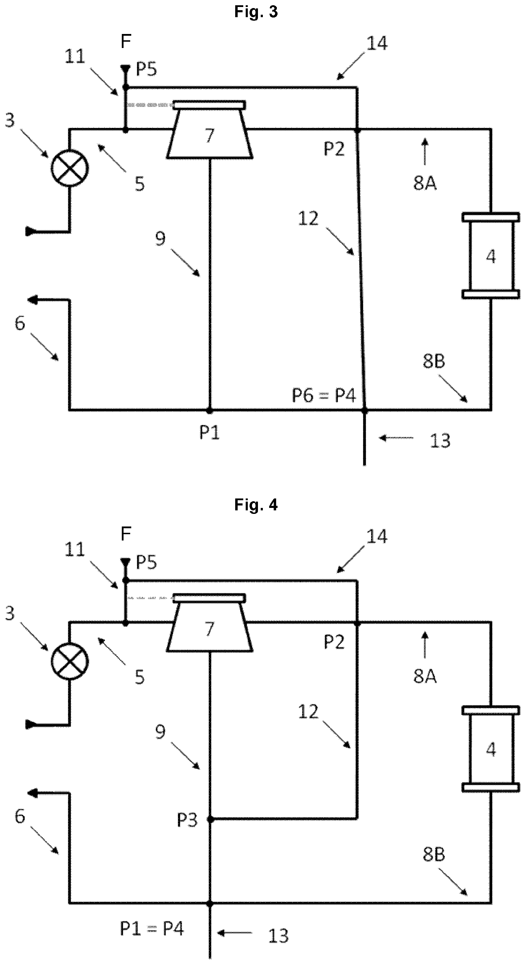

Um das Totvolumen des Systems noch weiter zu verringern, ist es noch weiter bevorzugt, wenn nicht nur die Regenerationsleitung (14) an dem Punkt (P2) in die Plasmaleitung (8A) mündet, an dem auch die Bypass-Leitung (12) von der Plasmaleitung (8A) abzweigt, sondern wenn auch die Abfallleitung (13) von demselben Punkt in der Plasmaleitung (8B) abzweigt in den auch die Bypass-Leitung (12) mündet. Anders ausgedrückt ist bevorzugt, wenn der Punkt (P6), an dem die Bypass-Leitung (12) in die Plasmaleitung (8B) mündet, und der Punkt (P4), an dem die Abfallleitung (13) von der Plasmaleitung (8B) abzweigt, übereinstimmen, d.h. wenn P4 = P6 ist (siehe hierzu auch **Fig. 2** und **Fig. 3**)

Die vorliegende Erfindung ist daher ebenso gerichtet auf eine erfindungsgemäße Apheresevorrichtung (1), wobei die Bypass-Leitung (12) von einem Punkt (P2) in der Plasmaleitung (8A) zu einem Punkt (P6) in der Plasmaleitung (8B) führt, und die Abfallleitung (13) von einem Punkt (P4) von der Plasmaleitung (8B) abgeht, und die zumindest eine Regenerationsleitung (14) am Punkt (P2) in die Plasmaleitung (8A) mündet und wobei der Punkt (P6) und der Punkt (P4) identisch sind.

In der erfindungsgemäßen Vorrichtung ist ein Zellseparator eingebaut, der das ihm (über die arterielle Leitung) zugeleitete Blut des Patienten in das Blutplasma und die zellulären Bestandteile aufteilt, und diese Fraktionen über die entsprechenden Leitungen, also die Plasmaleitung bzw. die Zellleitung fortleitet. Hierbei muss, wie bereits erwähnt, berücksichtigt werden, dass die Trennung in Blutplasma und zelluläre Bestandteile durch die verwendeten Zellseparatoren nicht vollständig erfolgt, sondern lediglich vorzugsweise 10 bis 90 % des gesamten Blutplasmas von den zellulären Bestandteilen getrennt wird. Bei der Verwendung von zentrifugalen Zellseparatoren wird vorzugsweise 70% bis 90%, weiter bevorzugt 80% bis 87% des gesamten Blutplasmas von den zellulären Bestandteilen getrennt. Bei der Verwendung von Membran-Zellseparatoren wird vorzugsweise 10% bis 30%, weiter bevorzugt 13% bis 25%, noch weiter bevorzugt 15% bis 20% des gesamten Blutplasmas von den zellulären Bestandteilen getrennt.

Mögliche Typen von Zellseparatoren, die im Zusammenhang mit der vorliegenden Erfindung verwendet werden können, umfassen zentrifugale Zellseparatoren, Membran-Zellseparatoren wie z.B. Membran-Zellseparatoren mit semipermeablen Membranen sowie Membran-Zellseparatoren mit rotierenden Membranen.

Die vorliegende Erfindung ist daher ebenso gerichtet auf eine Apheresevorrichtung zur extrakorporalen Entfernung von CRP aus Blut, wobei der Zellseparator (7) entweder ein zentrifugaler Zellseparator oder ein Membran-Zellseparator ist.

Wird in der vorliegenden Anmeldung die Position einer oder mehrerer Komponenten der erfindungsgemäßen Apheresevorrichtung in Relation zu einer anderen Komponente der erfindungsgemäßen Apheresevorrichtung durch die Begriffe "**vor**" oder "**nach**" (bzw. "in Flussrichtung vor" und "in Flussrichtung nach") beschrieben, so nimmt dies Bezug zur generellen Flussrichtung des Bluts bzw. des Blutplasmas in der erfindungsgemäßen Apheresevorrichtung. "Vor" in Bezug zu einer Komponente der erfindungsgemäßen Vorrichtung bedeutet folglich entgegen der allgemeinen Flussrichtung des Bluts bzw. des Blutplasmas, und "nach" in Bezug zu einer Komponente der erfindungsgemäßen Vorrichtung bedeutet folglich mit der allgemeinen Flussrichtung des Bluts bzw. des Blutplasmas. Es ist bevorzugt, dass die Flussrichtung in der Apheresevorrichtung sich nicht umkehrt oder durch das Mittel zur Generierung und Regulation eines Flusses nicht umgekehrt wird.

Gemäß der vorliegenden Erfindung umfasst die erfindungsgemäße Apheresevorrichtung zur extrakorporalen Entfernung von CRP aus Blut eine Apheresesäule (4) zur affinitätschromatographischen Entfernung von CRP aus dem Blut oder Blutplasma, dessen Funktion in der Bindung von CRP besteht, welches sich im Blut bzw. im Blutplasma eines Patienten befindet und das durch die Apheresesäule (4) hindurchgeleitet wird.

Der Begriff **"affinitätschromatographisch"** in Bezug zur Entfernung von CRP, wie in der vorliegenden Anmeldung verwendet, bedeutet, dass die Entfernung von CRP durch eine spezifische Bindung zwischen CRP und Bestandteilen der Apheresesäule (4) zur Entfernung von CRP geschieht. Man kann in diesem Zusammenhang auch von einer "selektiven Entfernung von CRP" oder von einer "selektiven CRP-Apherese" sprechen. Eine derartige spezifische Bindung zwischen CRP und Bestandteilen der Apheresesäule (4) beruhen auf den strukturellen Eigenschaften des CRP-Proteins und umfassen beispielsweise die charakteristische Bindung von CRP an Phosphocholin sowie dessen Derivate oder die Bindung von CRP an Antikörper, die gegen ein Epitop des CRP gerichtet sind. Die selektive oder molekülspezifische Entfernung von CRP beinhaltet, dass CRP mit höherer Affinität an die Matrix in der Apheresesäule (4) bindet als an andere Strukturen/Moleküle. Auch bindet CRP mit höherer Affinität an die Matrix in der Apheresesäule (4) als andere im Blut befindliche Substanzen.

Wie eine derartige Apheresesäule (bzw. Kartusche oder Patrone) prinzipiell gestaltet bzw. aufgebaut sein kann, gehört zum Stand der Technik und geht u.a. aus EP 0237659 B1 hervor. Die genauen Dimensionen der erfindungsgemäß verwendeten Kartusche, Säule oder Patrone (als Vorrichtung zur selektiven Entfernung von CRP) hängt hierbei maßgeblich von dem angestrebten Verwendungszweck der erfindungsgemäßen Vorrichtung ab. Apheresesäule (4) zur affinitätschromatographischen Entfernung von CRP umfasst hierbei in der Regel ein Gehäuse, z.B. in Form einer Kartusche oder einer Patrone, das über mindestens einen Zugang und mindestens einen Ausgang in fluidtechnischer Verbindung zum extrakorporalen Zirkulationssystem steht und welches eine Matrix zur affinitätschromatographischen bzw. zur adsorptiven Entfernung von CRP enthält.

Die Matrix zur affinitätschromatographischen (oder adsorptiven) Entfernung von CRP umfasst ein Matrixsubstratmaterial, an welches wiederum Verbindungen gebunden sind, die die Eigenschaft haben, CRP spezifisch zu binden. Die Matrix ist gemäß einer bevorzugten Ausführungsform der vorliegenden Erfindung derart in die Apheresesäule (4) zur affinitätschromatographischen Entfernung von CRP integriert bzw. in dieser immobilisiert, dass sie nicht mit dem Blutplasmafluss aus der Säule herausgespült werden kann. Dies kann je nach Ausführungsform z.B. in Form von Filtern am Eingang und Ausgang der Vorrichtung realisiert werden.

Grundsätzlich sind für die Herstellung der Matrix sämtliche inerten Chromatographie- oder Säulenmaterialien als Matrixsubstratmaterialien geeignet, welche insbesondere nicht mit Blut oder Blutplasma reagieren oder Blut bzw. Blutplasma derart verändern oder kontaminieren, dass das Blut bzw. Blutplasma nach Kontakt mit der Matrix einem Patienten nicht mehr zurückgeführt werden kann. Die erfindungsgemäß geeigneten Matrixsubstratmaterialien umfassen daher, ohne jedoch hierauf eingeschränkt zu sein, Eupergit, Polyvinylpyrollidon, Methacrylat, Methacrylat-Harze, Agarose, Sepharose, AcrylBeads, Zellulosematrizen, Keramikmatrizen, Glas-Beads und/oder Festphasen-Kieselerde oder Mischungen und/oder Derivate dieser Stoffe. Die Festphasen-Kieselerde-Matrix kann nahezu jede Form von partikulärer Kieselerde umfassen, einschließlich amorpher Kieselerden, wie kolloidale Kieselerde, Kieselgele, präzipitierte Kieselerden, und geräucherte oder pyrogene Kieselerden; mikrokristalline Kieselerden wie Kieselgur; und kristalline Kieselerden wie Quartz. Erfindungsgemäß werden die an die Matrixsubstratmaterialien gebundenen Verbindungen, die die Eigenschaft haben CRP spezifisch zu binden, ausgewählt aus der Gruppe umfassend oder bestehend aus Lipide, Lysophospholipide, Lysophosphatidylcholin, Peptide, Peptide mit geladenen Aminosäuren, Peptide enthaltend die Sequenz ArgProArg, Polypeptide, Antikörper, monoklonale Antikörper, Antikörperfragmente, manipulierte Antikörper, Phosphocholin, Derivate von Phosphocholin, DNA, DNA-Derivate, RNA, RNA-Derivate, L-Ribonukleinsäureaptamere, wie Spiegelmere® (ein RNA-ähnliches Molekül, welches aus L-Ribose Einheiten besteht) und Aptamere.

### Pumpen

Gemäß der vorliegenden Erfindung sind in der erfindungsgemäßen Apheresevorrichtung zur extrakorporalen Entfernung von CRP aus Blut Mittel zur Generierung und Regulation eines Flusses des Blutes (oder Blutplasmas) in dem extrakorporalen Zirkulationssystem vorgesehen. Hierzu werden in der Regel eine oder mehrere Pumpen bzw. Pumpsysteme verwendet, die einen regelbaren Fluss des Bluts (bzw. Blutplasmas oder auch der Regenerationslösung oder Antikoagulationslösung) durch das extrakorporale Zirkulationssystem und die fluidtechnisch mit diesem verbundenen Komponenten der erfindungsgemäßen Vorrichtung ermöglichen.

Die erfindungsgemäß bevorzugte Flussrichtung innerhalb des extrakorporalen Zirkulationssystems und der fluidtechnisch mit diesem verbundenen Komponenten der erfindungsgemäßen Vorrichtung verläuft von dem Zugang am Patienten, durch den das Blut in die erfindungsgemäße Vorrichtung gelangt, über die arterielle Leitung des extrakorporalen Zirkulationssystems zu der venösen Leitung des extrakorporalen Zirkulationssystems und zu dem Zugang am Patienten, an dem das behandelte Blut wieder in der Patienten zurückgeführt wird.

Bei den erfindungsgemäß verwendeten Mitteln zur Generierung und Regulation eines Flusses in dem extrakorporalen Zirkulationssystem handelt es sich vorzugsweise um Pumpen in Gestalt von Peristaltikpumpen (auch als Schlauchpumpen bezeichnet), Kolbenpumpen, pneumatischen Pumpen, Hydraulikpumpen oder andere dem Fachmann bekannte Pumpentypen. Folglich kann der Begriff "Mittel zur Generierung und Regulation eines Flusses" und der Begriff "Pumpe" hierin gleichbedeutend verwendet werden.

Es ist erfindungsgemäß bevorzugt, wenn die verwendeten Mittel zur Generierung und Regulation eines Flusses von Blut (bzw. Blutplasma oder auch der Regenerationslösung oder Antikoagulationslösung) in dem extrakorporalen Zirkulationssystem keinen direkten physischen Kontakt mit dem Blut (bzw. Blutplasma oder auch der Regenerationslösung oder Antikoagulationslösung) in der erfindungsgemäßen Vorrichtung haben. Dies ist besonders aus hygienischen Gründen vorteilhaft und verhindert die Kontaminierung von komplexen mechanischen Komponenten wie z.B. einer Pumpe durch Blut als natürlich auch des Blutes durch die verwendeten Mittel zur Flussgenerierung.

In einer besonders bevorzugten Ausführungsform der vorliegenden Erfindung handelt es sich bei den Mitteln zur Generierung und Regulation eines Flusses in dem extrakorporalen Zirkulationssystem daher um eine oder mehrere Peristaltikpumpe(n).

Die exakte Position der Mittel zur Generierung und Regulation eines Flusses in dem extrakorporalen Zirkulationssystem, d.h. der einen oder mehreren Pumpe(n), ist nicht wesentlich für die vorliegende Erfindung. Es sind Ausführungsformen der vorliegenden Erfindung unter Verwendung nur einer Pumpe möglich, bei denen sich die Pumpe im Bereich der arteriellen Leitung der erfindungsgemäßen Apheresevorrichtung zur extrakorporalen Entfernung von CRP aus Blut, d.h. vor dem Zellseparator befindet. Sind erfindungsgemäß mehrere Mittel zur Generierung und Regulation eines Flusses in dem extrakorporalen Zirkulationssystem vorgesehen, d.h. mehrere Pumpen, so ist bevorzugt, wenn diese unabhängig voneinander angesteuert und reguliert werden können (z.B. durch die CPU). Je nach der konkreten Anwendung können unterschiedliche Flussraten innerhalb des extrakorporalen Zirkulationssystems gewünscht bzw. erforderlich sein. Es ist auch denkbar, dass während einer konkreten Anwendung unterschiedliche Flussraten in verschiedenen Komponenten der erfindungsgemäßen Vorrichtung erwünscht sind.

Erfindungsgemäß können auch mehrere Mittel zur Generierung und Regulation eines Flusses (d.h. Pumpen) in der erfindungsgemäßen Apheresevorrichtung integriert sein. So ist es möglich, dass sich Mittel zur Generierung und Regulation eines Flusses in der arteriellen Leitung (5) und/oder in der Plasmaleitung (8A) und/oder in der Plasmaleitung (8B) und /oder in der venösen Leitung (6) und/oder in der Bypass-Leitung (12) und/oder in der Zellleitung (9) und/oder in der Anschlussleitung (11) bzw. den Anschlussleitungen (11', 11", 11''', etc.) und/oder in der Regenerationsleitung (14) bzw. den Regenerationsleitungen (14', 14", 14''', etc.) befinden. Wie bereits oben angedeutet, ist es gemäß einer Ausführungsform der vorliegenden Erfindung, in der die Regenerationsleitung (14) am Punkt (P5) von der Anschlussleitung (11) abzweigt, bevorzugt, dass ein Mittel zur Generierung und Regulation eines Flusses (von anorganischen Salzlösungen) in der Anschlussleitung (11) vor dem Punkt (P5) angebracht ist.

Gemäß einer weiteren Ausführungsform der vorliegenden Erfindung, in der die Regenerationsleitung (14) am Punkt (P5) von der Anschlussleitung (11) abzweigt, ist es bevorzugt, dass ein Mittel zur Generierung und Regulation eines Flusses in der Anschlussleitung (11) nach dem Punkt (P5) und ein Mittel zur Generierung und Regulation eines Flusses in der Regenerationsleitung (14) angebracht ist.

Des Weiteren weist die Apheresevorrichtung (1) vorzugsweise zumindest einen Partikelfilter auf, der in Flussrichtung hinter der Apheresesäule (4) in der Plasmaleitung (8B) oder der venösen Leitung (6) vorgesehen ist.
Des weiteren weist die Apheresevorrichtung (1) vorzugsweise zumindest einen Blasenfänger (Bubblecatcher) auf, der in Flussrichtung hinter der Apheresesäule (4) in der Plasmaleitung (8B) oder der venösen Leitung (6) vorgesehen ist.
Im Falle einer Zentrifuge als Zellseparator (7) weist die Apheresevorrichtung (1) vorzugsweise zumindest ein Plasmareservoir auf, das in Flussrichtung nach der Zentrifuge (7) und vor der Apheresesäule (4) in der Plasmaleitung (8A) vorgesehen ist.

In weiteren Ausführungsformen kann die erfindungsgemäße Apheresevorrichtung zur extrakorporalen Entfernung von CRP aus Blut oder Blutplasma eine oder mehrere Drucksensoren umfassen, die dazu dienen, den Druck in einem bestimmten Abschnitt der erfindungsgemäßen Vorrichtung zu messen bzw. zu überwachen. Dies dient nicht nur zur Überwachung und Einstellung der Betriebsparameter der erfindungsgemäßen Apheresevorrichtung, sondern ist auch dahingehend von Vorteil, dass im Falle einer Fehlfunktion (z.B. einer Verstopfung eines Schlauches oder Filters der Vorrichtung) der Betrieb gestoppt werden kann, um für den Patienten schädliche Folgen zu vermeiden. Die genaue Funktionsweise und Einbauposition in der erfindungsgemäßen Vorrichtung gehört zum Stand der Technik und ist dem Fachmann bekannt. In einer bevorzugten Ausführungsform der vorliegenden Erfindung ist sowohl mindestens ein Drucksensor in der arteriellen Leitung der erfindungsgemäßen Apheresevorrichtung als auch mindestens ein Drucksensor in der venösen Leitung der erfindungsgemäßen Apheresevorrichtung, angeordnet. In einer weiteren bevorzugten Ausführungsform der vorliegenden Erfindung sind derartige Drucksensoren in den verwendeten Mitteln zur Generierung und Regulation eines Flusses in dem extrakorporalen Zirkulationssystem der erfindungsgemäßen Apheresevorrichtung integriert.

Um an den Knotenpunkten des extrakorporalen Zirkulationssystems, also an den Punkten an denen mehrere Leitungen zusammenführen bzw. voneinander abgehen, die Flussrichtung im System kontrollieren zu können sind vorzugsweise Mittel vorgesehen, die den Fluss der Lösung (also z.B. des Bluts, des Plasmas oder der Regenerationslösung) bestimmen. Hierbei kann es sich um Ventile, Mehrwegeventile, Klemmen, oder Ventile in Gestalt von Sperrventilen, Rückschlagventilen, Druckventilen, Wegeventilen oder andere dem Fachmann bekannte Ventiltypen handeln, die den Fluss in einer bestimmten Richtung freigeben und in einer anderen Richtung blockieren. Bevorzugt befinden sich derartige Mittel zur Flussregulation (z.B. Ventile) am Punkt (P1) und/oder am Punkt (P2) und/oder am Punkt (P3) und/oder am Punkt (P4) und/oder am Punkt (P5) und/oder am Punkt (P6). Zudem ist es möglich, das z.B. an einem Punkt zwei oder mehr Ventile in Reihe geschaltet sind, um eine komplexere Flussregulation zu ermöglichen.

Es ist zudem besonders bevorzugt wenn die Mittel zur Flussregulation (z.B. Ventile) elektronisch steuerbar sind, d.h. deren Stellung durch die zentrale Recheneinheit (10) erfolgen kann.

Die vorliegende Erfindung ist daher ebenso gerichtet auf eine Apheresevorrichtung zur extrakorporalen Entfernung von CRP aus Blut, wobei elektronisch gesteuerte Ventile an den Punkten (P1), (P2), (P4), (P5), (P6), (P7) und (P8) angebracht sind.

Ebenso ist es denkbar und erfindungsgemäß, wenn sich Ventile nicht direkt an den Verzweigungspunkten (P1, P2, P4, P5, P6, P7 und P8) befinden, sondern in den vorgeschalteten und/oder den nachgeschalteten Leitungen befinden, und so den Fluss der Lösungen in dem extrakorporalen Zirkulationsystem steuern. Zu diesem Zweck können auch Schlauchklemmen verwendet werden. Besonders bevorzugt ist, wenn diese Ventile doer Schlauchklemmen elektronisch gesteuert sind.

Ein weiterer Vorteil der vorliegenden Erfindung, der damit zusammenhängt, dass die Apherese und die Regeneration der Apherese-Säule in einer einzelnen Vorrichtung realisiert ist, besteht darin, dass die gesamte Vorrichtung über nur eine einzelne zentrale Recheneinheit (CPU) kontrolliert werden kann. So können die unterschiedlichen Programme während einer Apherese-Sitzung, also zum Beispiel der Normalbetrieb, in dem das Blutplasma durch die Apheresesäule geleitet wird, und der Regenerationsbetrieb, in dem das Blutplasma durch die Bypass-Leitung an der Apheresesäule vorbeigeleitet wird und die Apheresesäule mit Regenerationslösung gespült wird, durch eine einzelne Recheneinheit bzw. eine auf ihr befindliche Software gesteuert werden. Dies erleichtert die Automatisierung vieler Prozesse und verringert somit den Spielraum für Bedienungsfehler seitens des Personals. Bei Geräten aus dem Stand der Technik müssen hingegen unterschiedliche komplexe Systeme (Primärsystem zur Bluttrennung in Plasma und zelluläre Bestandteile; und Sekundärsystem zur Apherese und Regeneration) kombiniert werden, wobei jedes System separat gesteuert werden muss.

Die vorliegende Erfindung ist daher ebenso gerichtet auf eine Apheresevorrichtung zur extrakorporalen Entfernung von CRP aus Blut, wobei die gesamte Vorrichtung nur mittes der einen zentralen Recheneinheit (10) kontrolliert wird.

Ein weiterer Aspekt der vorliegenden Erfindung betrifft eine Apheresevorrichtung, wobei eine zweite Apheresesäule (4") mit der Bypass-Leitung verbunden ist oder die Bypassleitung eine zweite Apheresesäule umfasst. Bevorzugt ist die zweite Apheresesäule (4") in der Bypass-Leitung enthalten. Daher können die hierin beschriebenen erfindungsgemäßen Apheresevorrichtungen zur extrakorporalen Entfernung von CRP aus Blut eine zweite Apheresesäule (4") enthalten, wobei die zweite Aphersesäule (4") in der Bypassleitung enthalten ist. Eine Apheresesäule (4") ist in der Bypass-Leitung enthalten, wenn ein Abschnitt der Bypass-Leitung (12') der Bypass-Leitung (12) in die zweite Apheresesäule (4") mündet und ein weiterer Abschnitt der Bypass-Leitung (12") der Bypass-Leitung (12) am Ausgang der Apheresesäule (4") von dieser wegführt.

Daher ist ein weiterer Aspekt der der vorliegenden Erfindung eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (1), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") nur abwechselnd betreibbar sind, d.h. nicht zeitgleich zur CRP-Entfernung nutzbar sind.

Die oben genannten Ausgestaltungen der erfindungsgemäßen Apheresevorrichtung (1) sind auf die erfindungsgemäße Apheresevorrichtung (II) zu übertragen.

Mithilfe dieser erfindungsgemäßen Apheresevorrichtung (II) ist es möglich CRP bei gleicher Behandlungsdauer effizienter aus Blut zu entfernen als mit Vorrichtungen aus dem Stand der Technik. Durch die Verwendung von zwei parallel geschalteten Apheresesäulen, die nur abwechselnd zur CRP-Entfernung einsetzbar sind, kann mittels der erfindungsgemäßen Apheresevorrichtung eine Apheresesäule für die Entfernung von CRP aus dem Blut genutzt werden, während die zweite Apheresesäule entweder durch eine andere Apherese ausgetauscht oder die zweite Apheresesäule während der laufenden Apheresesitzung regeneriert werden kann. Dadurch kann auch ein hoher Klinikdurchsatz mittels einer Apheresevorrichtung erzielt werden. Weiterhin ist die Verwendung der erfindungsgemäßen Apheresevorrichtung nicht durch das Totvolumen limitiert. Üblicherweise werden überdimensionierte Apheresesäulen aber auch in Reihe geschaltete Apheresesäulen durch ihr großes Totvolumen in ihrer Anwendung für die Apherese stark beschränkt. Zudem wird das Volumen einer Apheresevorrichtung und damit das Volumen oder die Anzahl an in Reihe geschalteten Apheresesäulen durch die Blutflussrate des Menschen vorgegeben. Auch Apheresevorrichtungen mit parallel geschalteten Apheresesäulen, die zeitgleich verwendet werden, können aufgrund des großen Totvolumens nicht effizient und ohne Risiko für den Patienten für die Entfernung von CRP aus Blut genutzt werden. Demnach kann die Bypass-Leitung (12) als Plasmaleitung verwendet werden.

Eine hierin beschriebene erfindungsgemäße Apheresevorrichtung (II) zeichnet sich dadurch aus, dass eine zweite Apheresesäule zu einer ersten Apheresesäule (4') parallel geschaltet ist. "Parallel" bedeutet in diesem Zusammenhang, dass mehrere Kreisläufe innerhalb des extrakorporalen Zirkulationssystems (2) nebeneinander vorhanden sind, d.h., dass z.B. eine erste Apheresesäule (4') mit der Plasmaleitung (8A) für das abgetrennte Plasma und mit der Plasmaleitung (8B) für das CRP-abgereicherte Plasma ein erstes Kreislaufsystem des extrakorporalen Zirkulationssystems (2) darstellt und eine zweite Apheresesäule (4") mit dem Bypass-Leitungsabschnitt (12') der Bypass-Leitung (12) und dem Bypass-Leitungsabschnitt (12") der Bypass-Leitung (12) ein zweites Kreislaufsystem des extrakorporalen Zirkulationssystems (2) darstellt. "Parallel" bedeutet auch, dass die zwei Apheresesäulen nicht in Reihe geschaltet sind, d.h. nicht hintereinander, dass der Ausfluss der ersten Apheresesäule in die zweite Apheresesäule eingeleitet wird. Durch die parallele Anordnung der Apheresesäulen addieren sich deren Kapazitäten auch nicht.
Hiervon zu unterscheiden ist die serielle Schaltung der Apheresesäulen, die nicht erfindungsgemäß ist. "Seriell" bedeutet, dass mehrere Apheresesäulen sich nur in einem Kreislauf des extrakorporalen Zirkulationssystems (2) befinden, d.h., dass z.B. die erste Apheresesäule (4') und die zweite Apheresesäule (4") zusammen mit der Plasmaleitung (8A) und der Plasmaleitung (8B) nur einen Kreislauf des extrakorporalen Zirkulationssystems (2) bilden, d.h. in Reihe geschaltet oder angeordnet wären.

Erfindungsgemäß können die beiden zueinander parallel geschalteten oder parallel angeordneten Apheresesäulen (4', 4") nur abwechselnd betrieben werden. "Abwechselnd" bedeutet, dass das abgetrennte Blutplasma entweder durch die Apheresesäule (4') oder durch die Apheresesäule (4") aber nicht zeitgleich durch beide Apheresesäulen (4', 4") geleitet wird. "Abwechselnd" betrieben meint dabei die therapeutische CRP-Entfernung. Beide Apheresesäulen (4' und 4") sind nicht zeitgleich zur CRP-Entfernung nutzbar. Natürlich kann eine der beiden Apheresesäulen regeneriert werden, während die andere zeitgleich zur CRP-Entfernung eingesetzt wird. Ausgeschlossen ist nur der zeitgleiche therapeutische Betrieb zur CRP-Entfernung beider Apheresesäulen.

Folgende Zustände sind daher möglich. Durch eine Apheresesäule wird das Blutplasma geleitet, um das CRP zu entfernen. Zeitgleich ist die zweite Apheresesäule gebrauchsfertig und es kann der Plasmafluss auf diese zweite Apheresesäule umgeleitet werden sobald die Kapazität der ersten Apheresesäule erschöpft ist oder andere Probleme mit der ersten Apheresesäule auftreten sollten oder die zweite Apheresesäule wurde bereits zur CRP-Entfernung genutzt und muss ausgetauscht oder regeneriert werden oder die zweite Apheresesäule wird regeneriert während die erste CRP entfernt.

In Ausführungsformen der vorliegenden Erfindung ist die Apheresevorrichtung (II) mit zwei Apheresesäulen daher derart ausgestaltet, dass die Apheresesäulen nur abwechselnd betreibbar sind.

Somit ist gemäß einer Ausführungsform der erfindungsgemäßen Apheresevorrichtung (II) das Blutplasma zeitgleich entweder nur durch die erste Apheresesäule (4') oder nur durch die zweite Apheresesäule (4") leitbar. In weiteren Ausführungsformen der erfindungsgemäßen Vorrichtung ist somit die Apheresevorrichtung derart ausgestaltet, dass das Blutplasma zeitgleich entweder nur durch die erste Apheresesäule (4') oder nur durch die zweite Apheresesäule (4") leitbar ist.

Eine Ausführungsform der vorliegenden Erfindung betrifft daher eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (1), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") nur abwechselnd betreibbar sind, und wobei das Blutplasma zeitgleich entweder nur durch die erste Apheresesäule (4') oder nur durch die zweite Apheresesäule (4") leitbar ist.

Während des abwechselnden Betriebs der beiden Apheresesäulen (4', 4") wird entweder durch die Apheresesäule (4') oder durch die Apheresesäule (4") kein Blutplasma geleitet. Dadurch ergibt sich die Möglichkeit, eine der beiden Apheresesäule aus der Apheresevorrichtung während des Betriebs der Apheresevorrichtung auszutauschen. "Austauschen" bedeutet hierbei, dass eine der beiden Apheresesäulen durch eine neue Apheresesäule ersetzt oder eine der beiden Apheresesäulen regeneriert wird. Die Regeneration einer der beiden Apheresesäulen kann z.B. durch Spülen mit einer Citrat-Lösung erfolgen. Die Verwendung einer Citrat-Lösung ist für die Regeneration der Apheresesäulen bevorzugt. "Während des Betriebs" bedeutet in diesem Zusammenhang, dass die Entfernung des CRP aus dem Blut weiterhin abläuft.

Eine Ausführungsform der hierin beschriebenen erfindungsgemäßen Apheresevorrichtungen (II) betrifft daher eine Apheresevorrichtung, in dem eine erste Apheresesäule (4') während des Betriebs einer zweiten Apheresesäule (4") austauschbar ist und die zweite Apheresesäule (4") während des Betriebs der ersten Apheresesäule (4') austauschbar ist.

Ebenso sind Ausführungsformen denkbar, wobei eine erste Apheresesäule (4') während des Betriebs einer zweiten Apheresesäule (4") regenerierbar ist und die zweite Apheresesäule (4") während des Betriebs der ersten Apheresesäule (4') regenerierbar ist.

Somit ist in einer Ausführungsform der vorliegenden Erfindung die Apheresevorrichtung (II) derart ausgestaltet, dass eine erste Apheresesäule (4') während des Betriebs einer zweiten Apheresesäule (4") austauschbar oder regenerierbar ist und die zweite Apheresesäule (4") während des Betriebs der ersten Apheresesäule (4') austauschbar oder regenerierbar ist.

Eine Ausführungsform der vorliegenden Erfindung betrifft ferner eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (1), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") zeitgleich nur abwechselnd betreibbar sind, und wobei die erste Apheresesäule (4') während des Betriebs der zweiten Apheresesäule (4") austauschbar oder regenerierbar ist und die zweite Apheresesäule (4") während des Betriebs der ersten Apheresesäule (4') austauschbar oder regenerierbar ist.

Die vorliegende Erfindung betrifft zudem eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (1), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind, und wobei eine der Apheresesäulen (4', 4") zeitgleich zur CRP-Entfernung durch die andere Apheresesäule regenerierbar ist.

Daher ist gemäß einer Ausführungsform der vorliegenden Erfindung die Apheresevorrichtung (II) derart ausgestaltet, dass die erste Apheresesäule (4') während des Betriebs der zweien Apheresesäule (4") austauschbar und die Apheresesäule derart ausgestaltet ist, dass sie regenerierbar ist und die zweite Apheresesäule (4") während des Betriebs der ersten Apheresesäule (4') austauschbar ist und die Apheresesäule (4") derart ausgestaltet ist, dass die Apheresesäule (4") regenerierbar ist.

Die zur ersten Apheresesäule (4') parallel geschaltete zweite Apheresesäule (4") kann in die Bypass-Leitung eingebunden sein, d.h. die Bypass-Leitung (12) setzt sich zusammen aus einen Bypass-Leitungs-Abschnitt (12') und einen Bypass-Leitungs-Abschnitt (12"), wobei zwischen den besagten Bypass-Leitungsabschnitten die zweite Apheresesäule (4") befindet.

Die vorliegende Erfindung betrifft daher auch eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind.

Eine Ausführungsform der vorliegenden Erfindung betrifft daher eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest zwei Anschlussleitungen (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest zwei Anschlussleitungen (11) abgeht und in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind (d.h. nur abwechselnd betreibbar sind).

Gemäß einer Ausführungsform der vorliegenden Erfindung ist es besonders bevorzugt, wenn die erfindungsgemäße Apheresevorrichtung (II) zwei Anschlussleitungen (11',11") zum Anschluss von jeweils zumindest einen Flüssigkeitsbehälter (F) aufweist, wobei die Anschlussleitungen (11', 11") unabhängig voneinander in die arterielle Leitung (5) oder direkt in den Zellseparator (7) münden. Folglich münden beide Anschlussleitungen (11', 11") in die arterielle Leitung (5) oder beide Anschlussleitungen (11', 11") münden direkt in den Zellseparator (7) oder besonders bevorzugt mündet eine Anschlussleitung (11') in die arterielle Leitung (5) und die andere Anschlussleitung (11") direkt in den Zellseparator (7). Hierdurch wird ermöglich, dass die zwei Anschlussleitungen (11', 11") an unterschiedliche Flüssigkeitsbehälter angeschlossen werden können. Besonders bevorzugt ist, wenn eine der beiden Anschlussleitungen (z.B. 11') an einen Flüssigkeitsbehälter enthaltend eine physiologische Salzlösung (z.B. NaCI-Lösung) angeschlossen wird, während die zweite der beiden Anschlussleitungen (z.B. 11") an einen Flüssigkeitsbehälter enthaltend eine Citrat-Lösung angeschlossen wird.

Eine weitere Ausführungsform der vorliegenden Erfindung ist eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zwei Anschlussleitung (11',11") zum Anschluss von jeweils zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4') mündet, und
wobei die zweite Apheresesäule (4") parallel zur ersten Apheresesäule (4') geschaltet ist und beide Apheresesäulen (4', 4") nicht zeitgleich zur CRP-Entfernung nutzbar sind, d.h. nur abwechselnd betreibbar sind.

Gemäß einer weiteren Ausführungsform der vorliegenden Erfindung enthält die Apheresevorrichtung (II) eine Abfallleitung (13'), die direkt von Apheresesäule (4') abgeht oder von der Plasmaleitung (8B) in Flussrichtung vor der Einmündung des Bypass-Leitungsabschnitts (12") der Bypass-Leitung in die Plasmaleitung (8B) abgeht und eine Abfallleitung (13"), die direkt von der Apheresesäule (4") abgeht oder von dem Bypass-Leitungsabschnitt (12") in Flussrichtung vor der Einmündung in die Plasmaleitung (8B).

Die vorliegende Erfindung betrifft somit auch eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13') direkt von der Apheresesäule (4') abgeht oder von der Plasmaleitung (8B) in Flussrichtung vor der Einmündung des Bypass-Leitungsabschnitts (12") der Bypass-Leitung (12) abgeht, eine Abfallleitung (13"), die direkt von Apheresesäule (4") abgeht oder von der Plasmaleitung (8B) in Flussrichtung vor der Einmündung des Bypass-Leitungsabschnitts (12") der Bypass-Leitung in die Plasmaleitung (8B) abgeht und eine Abfallleitung (13"), die direkt von der Apheresesäule (4") abgeht oder von dem Bypass-Leitungsabschnitt (12") in Flussrichtung vor der Einmündung in die Plasmaleitung (8B),
   und
zumindest eine Regenerationsleitung (14), die von dem zumindest einen Flüssigkeitsbehälter (F) oder der zumindest einen Anschlussleitung (11) abgeht und in Flussrichtung nach der Abzweigung des Bypass-Leitungsabschnitts (12') der Bypass-Leitung (12) zu der Plasmaleitung (8A) oder zu dem Bypass-Leitungsabschnitt (12') führt oder direkt in die Apheresesäule (4') oder Apheresesäule (4") mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind, d.h. nur abwechselnd betreibbar sind.

Gemäß einer weiteren Ausführungsform der vorliegenden Erfindung enthält die Apheresevorrichtung (II) weiterhin zumindest eine Regenerationsleitung (14), die von dem zumindest einen Flüssigkeitsbehälter (F) oder der zumindest einen Anschlussleitung (11) abgeht und in die Plasmaleitung (8A) oder in den Bypass-Leitungsabschnitt (12' der Bypass-Leitung (12) führt oder direkt in die Apheresesäule (4') oder direkt in die Apheresesäule (4") mündet. Gemäß einer weiteren Ausführungsform der vorliegenden Erfindung enthält die Apheresevorrichtung (II) weiterhin zumindest eine Regenerationsleitung (14), die in einem Bereich von dem Punkt (P2) und der Apheresesäule (4') in den Bypass-Leitungsabschnitt (12') oder in einem Bereich von dem Punkt (P2) und der Apheresesäule (4") in die Plasmaleitung (8A) mündet oder direkt in die Apheresesäule (4') oder direkt in die Apheresesäule (4") mündet.

In einer besonders bevorzugten Ausführungsform der vorliegenden Erfindung umfasst die Apheresevorrichtung (II) eine Abfallleitung (13'), die direkt von der Apheresesäule (4') abgeht oder von der Plasmaleitung (8B) in Flussrichtung vor der Einmündung des Bypass-Leitungsabschnitts (12") der Bypass-Leitung (12) abgeht und zumindest eine Regenerationsleitung (14), die von dem zumindest einen Flüssigkeitsbehälter (F) oder der zumindest einen Anschlussleitung (11) abgeht und in den Bypass-Leitungsabschnitt (12') oder in die Plasmaleitung (8A) führt oder direkt in die Apheresesäule (4') oder direkt in die Apheresesäule (4") mündet.

Besonders bevorzugt ist eine Apheresevorrichtung (II) zur extrakorporalen Entfernung von CRP aus Blut oder Blutplasma umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1), eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung des Bypass-Leitungsabschnitts (12') der Bypass-Leitung (12) zu der Plasmaleitung (8A) oder in den Bypass-Leitungsabschnitt (12') der Bypass-Leitung (12) führt oder direkt in die Apheresesäule (4') oder direkt in die Apheresesäule (4") mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind.

In einer insbesondere bevorzugte Ausführungsform der vorliegenden Erfindung umfasst die Apheresevorrichtung (II) eine Abfallleitung (12'), die direkt von der Apheresesäule (4') abgeht oder von der Plasmaleitung (8B) in Flussrichtung vor der Einmündung des Bypass-Leitungsabschnitts (12") der Bypass-Leitung abgeht, eine Abfallleitung (13"), die direkt von der Apheresesäule (4") abgeht oder von dem Bypass-Leitungsabschnitt (12") der Bypass-Leitung (12) in Flussrichtung vor der Einmündung des Bypass-Leitungsabschnitts (12') der Bypass-Leitung abgeht und zumindest eine Regenerationsleitung (14), die von dem zumindest einen Flüssigkeitsbehälter (F) oder der zumindest einen Anschlussleitung (11) abgeht und in die Plasmaleitung (8A) oder in den Bypass-Leitungsabschnitt (12') der Bypass-Leitung (12) führt oder direkt in die Apheresesäule (4') oder direkt in die Apheresesäule (4") mündet.

Eine besonders bevorzugte Ausführungsform der zugrundeliegenden Erfindung betrifft eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des Bypass-Leitungsabschnitt (12') der Bypass-Leitung (12) abgeht, eine Abfallleitung (13"), die direkt von der Apheresesäule (14") abgeht oder von dem Bypass-Leitungsabschnitt (12") der Bypass-Leitung (12) in Flussrichtung vor der Einmündung der Bypass-Leitungsabschnitt (12') der Bypass-Leitung (12),
   und
zumindest eine Regenerationsleitung (14), die von dem zumindest einen Flüssigkeitsbehälter (F) oder der zumindest einen Anschlussleitung (11) abgeht und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet,
   und
wobei eine zweite Apheresesäule (4") parallel zur ersten Apheresesäule (4') geschaltet ist und beide Apheresesäulen (4', 4") nicht zeitgleich zur CRP-Entfernung nutzbar sind.

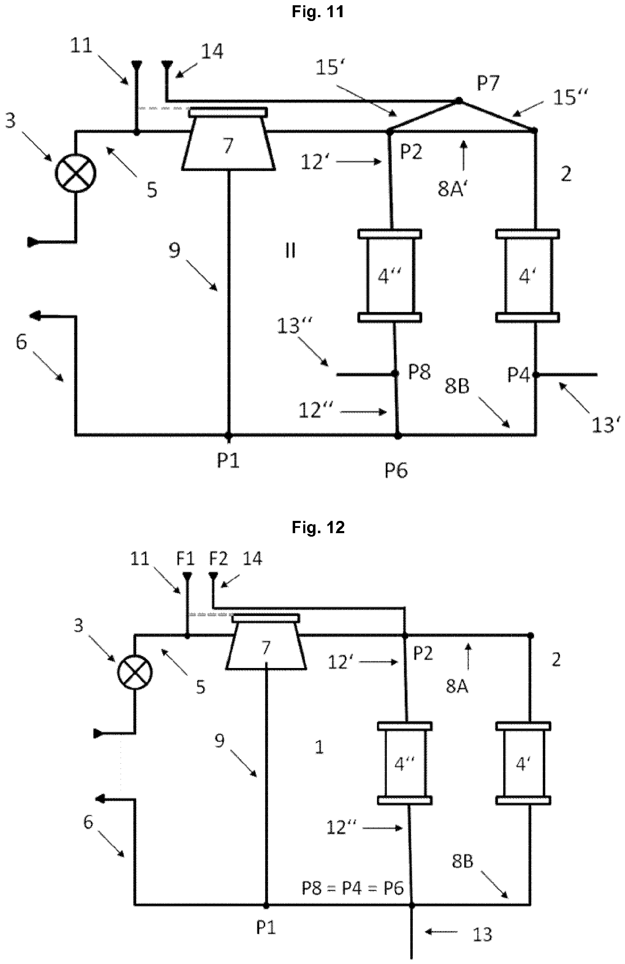

Weiterhin sind Ausführungsformen der erfindungsgemäßen Apheresevorrichtung (II) möglich, wobei die zumindest eine Regenerationsleitung (13) zu einem Punkt (P7) läuft und von dem Punkt (P7) eine Leitung (14') bis zum Punkt (P2) führt oder in die Plasmaleitung (8A) mündet und von dem Punkt (P7) eine Leitung (14") in Plasmaleitung (8A) (siehe Fig. 11).

Für den Fall, dass die zumindest eine Regenerationsleitung (14) für die Spüllösung in die Plasmaleitung (8A) zwischen den Punkt (P2) und der Apheresesäule (4') mündet oder für den Fall, dass die zumindest eine Regenerationsleitung (14) in den Bypass-Leitungsabschnitt (12') der Bypass-Leitung (12) zwischen den Punkt (P2) und der Apheresesäule (4") mündet, kann die Spüllösung entweder nur für die Apheresesäule (4') oder für die Apheresesäule (4") verwendet werden. Die Regenerationsleitung (14) ist somit entweder selektiv zur Apheresesäule (4') oder selektiv zur Apheresesäule (4").

Ebenso sind Ausführungsformen der erfindungsgemäßen Apheresevorrichtung (II) mit zwei, drei oder mehrere Regenerationsleitungen (14'. 14", 14''', etc.) möglich, wobei hierbei diese zwei, drei oder mehrere Regenerationsleitungen unabhängig voneinander in die Plasmaleitung (8A) [d.h. vom Punkt (P2) bis zur Apheresesäule (4')] oder in den Bypass-Leitungsabschnitt (12') [d.h. vom Punkt (P2) bis zur Apheresesäule (4")] oder in die Apheresesäule (4') oder in die Apheresesäule (4") münden können. "Unabhängig voneinander" bedeutet in diesem Zusammenhang zum, dass bei einer Ausführungsform der erfindungsgemäßen Apheresevorrichtungen mit zwei Regenerationsleitungen (14', 14") die eine Regenerationsleitung (14') in die Plasmaleitung (8A) zwischen den Punkt (P2) und der Apheresesäule (4') mündet und die andere Regenerationsleitung (14") direkt in die Apheresesäule (4") mündet, aber auch dass beide Regenerationsleitungen (14', 14") in die Plasmaleitung (8A) zwischen den Punkt (P2) und der Apheresesäule (4') münden können. Ein weitere Möglichkeit ist, dass eine Regenerationsleitung (14') an dem Punkt (P2) in das extrakorporale Zirkulationssystem (2) mündet und die andere Regenerationsleitung (14") in den Bypass-Leitungsabschnitt (12') der Bypass-Leitung (12) zwischen den Punkt (P2) und der Apheresesäule (4") mündet. Auch denkbar ist, dass eine Regenerationsleitung (14') an dem Punkt (P2) in das extrakorporale Zirkulationssystem mündet und die andere Regenerationsleitung (14") in die Apheresesäule (4") mündet. Auch möglich ist, dass eine Regenerationsleitung (14') in die andere Regenerationsleitung (14") mündet. Bei Vorhandensein von zwei oder mehr Regenerationsleitungen (14', 14", 14''', etc.) ist es jedoch, dass bevorzugt alle Regenerationsleitungen (14',14", 14''', etc.) am Punkt (P2) in das extrakorporale Zirkulationssystem (2) münden.

Weiterhin bevorzugt ist, wenn eine Regenerationsleitung (14') in den Bypass-Leitungsabschnitt (12') der Bypass-Leitung (12) zwischen den Punkt (P2) und der Apheresesäule (4') mündet und die andere Regenerationsleitung (14") in den Bypass-Leitungsabschnitt (12') der Bypass-Leitung (12) zwischen dem Punkt P2 und der Apheresesäule (4") mündet. Weiterhin bevorzugt ist, wenn die Regenerationsleitung (14') in die Apheresesäule (4') und die andere Regenerationsleitung (14") in die Apheresesäule (4") mündet. Hierbei ist die Regenerationsleitung (14') selektiv zur ersten Apheresesäule (4') und die Regenerationsleitung selektiv zur zweiten Apheresesäule (4").

Gemäß einer besonders bevorzugten Ausführungsform der vorliegenden Erfindung umfasst die Apheresevorrichtung (II) daher des Weiteren eine Regenerationsleitung (14') für eine Spüllösung selektiv zur ersten Apheresesäule (4') und/oder des Weiteren eine Regenerationsleitung (14") für eine Spüllösung selektiv zur zweiten Apheresesäule (4").

Daher betrifft eine Ausführungsform der vorliegenden Erfindung eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
eine Regenerationsleitung (14') für eine Spüllösung selektiv zur ersten Apheresesäule (4') und/oder eine Regenerationsleitung (14") für eine Spüllösung selektiv zur zweiten Apheresesäule (4")
   und
wobei eine zweite Apheresesäule (4") parallel zur ersten Apheresesäule (4') geschaltet ist und beide Apheresesäulen (4', 4") nicht zeitgleich zur CRP-Entfernung nutzbar sind, d.h. nur abwechselnd betreibbar sind.

Wie bereits oben erwähnt, können die für die Regeneration der Apheresesäulen notwendige Regenerationslösung über die Regenerationsleitung (14) in das extrakorporale Zirkulationssystem (2) eingespeist werden, und somit zusätzlich zur Spüllösung auch noch eine Regenerationslösung (z.B. eine Citrat-Lösung, eine TRIS-Glycin-Lösung, oder eine Natriumchlorid-Lösung) eingesetzt werden. Die Spüllösung kann, muss aber nicht, zur Regeneration der ersten Apheresesäule (4') und/oder Apheresesäule (4") dienen, sondern hat neben der oben genannten Funktion die Aufgabe, das Blutplasma aus der Plasmaleitung (8A) in dem Bereich von Punkt (P2) bis zu der Apheresesäule (4') sowie aus der Plasmaleitung (8B) von der Apheresesäule (4') bis zu dem Punkt (P8) oder das Blutplasma aus dem Bypass-Leitungsabschnitt (12') der Bypass-Leitung (12) in dem Bereich von Punkt (P2) bis zu der Apheresesäule (4") sowie aus dem Bypass-Leitungsabschnitt (12") der Bypass-Leitung (12) von der Apheresesäule (4") bis zu dem Punkt (P8) zu verdrängen und damit zurück in den Blutkreislauf des Patienten geführt, bevor die Regenerationslösung eingesetzt wird, welche nach Durchfluss durch eine der beiden Apheresesäulen (4', 4") dann über die Ablaufleitung (13', 13") verworfen wird.

Somit ist es denkbar, dass die parallel geschaltet Apheresesäulen (4', 4") nicht nur abwechselnd betrieben werden können, sondern auch abwechselnd regeneriert werden.

In den hierin beschriebenen erfindungsgemäßen Apheresevorrichtungen (II) kann die erste Apheresesäule (4') während des Betriebs der zweiten Apheresesäule (4") austauschbar oder regenerierbar ist und die zweite Apheresesäule (4") während des Betriebs der ersten Apheresesäule (4') austauschbar oder regenerierbar ist.

Eine Ausführungsform der vorliegenden Erfindung ist daher gerichtet auf eine Apheresevorrichtung (II) zur extrakorporalen Entfernung von CRP aus Blut oder Blutplasma umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des Bypass-Leitungsabschnitt (12') der Bypass-Leitung (12) abgeht, eine Abfallleitung (13"), die direkt von der Apheresesäule (14") abgeht oder von dem Bypass-Leitungsabschnitt (12") der Bypass-Leitung (12) in Flussrichtung vor der Einmündung der Bypass-Leitungsabschnitt (12') der Bypass-Leitung (12),
   und
zumindest eine Regenerationsleitung (14), die von dem zumindest einen Flüssigkeitsbehälter (F) oder der zumindest einen Anschlussleitung (11) abgeht und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet, und
wobei eine zweite Apheresesäule (4") parallel zur ersten Apheresesäule (4') geschaltet ist und beide Apheresesäulen (4', 4") nur abwechselnd betreibbar sind, und wobei die erste Apheresesäule (4') während des Betriebs der zweiten Apheresesäule (4") austauschbar oder regenerierbar ist und die zweite Apheresesäule (4") während des Betriebs der ersten Apheresesäule (4') austauschbar oder regenerierbar ist.

Weiterhin sind Ausführungsformen der vorliegenden Erfindung denkbar, in dem die Apheresevorrichtung je Flüssigkeitsbehälter (F) eine Regenerationsleitung (14) aufweist, die von dem jeweiligen Flüssigkeitsbehälter (F) oder dessen Anschlussleitung (11) abgehen und die jeweils in die Plasmaleitung (8A) oder in die Bypass-Leitungsabschnitt (12') der Bypass-Leitung (12) oder direkt in die Apheresesäule (4') oder direkt in die Apheresesäule (4") führt.

Eine besonders bevorzugte Ausführungsform der zugrundeliegenden Erfindung betrifft eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des Bypass-Leitungsabschnitt (12') der Bypass-Leitung (12) abgeht, eine Abfallleitung (13"), die direkt von der Apheresesäule (14") abgeht oder von dem Bypass-Leitungsabschnitt (12") der Bypass-Leitung (12) in Flussrichtung vor der Einmündung der Bypass-Leitungsabschnitt (12') der Bypass-Leitung (12),
und je Flüssigkeitsbehälter (F) eine Regenerationsleitung (14) enthalten ist, die von dem jeweiligen Flüssigkeitsbehälter (F) oder dessen Anschlussleitung (11) abgehen und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind.

Ferner sind Ausführungsformen der Apheresevorrichtung (II) bevorzugt, bei denen die Apheresevorrichtung (II) zumindest zwei Anschlussleitungen (11) zum Anschluss von jeweils zumindest einem Flüssigkeitsbehälter (F) an die arterielle Leitung (5) oder den Zellseparator (7) aufweist, und wobei je Flüssigkeitsbehälter (F) eine Regenerationsleitung (13) existiert, die von dem jeweiligen Flüssigkeitsbehälter (F) oder dessen Anschlussleitung (11) abgehen und die jeweils in die Plasmaleitung (8A) oder in den Bypass-Leitungsabschnitt (12') der Bypass-Leitung (12) oder direkt in die Apheresesäule (4') oder direkt in die Apheresesäule (4") führen.

Gemäß einigen Ausführungsformen der vorliegenden Erfindung bevorzugt, dass die zumindest eine Regenerationsleitung (14), die in die Plasmaleitung (8A) oder in den Bypass-Leitungsabschnitt (12') der Bypass-Leitung (12) führt oder direkt in die Apheresesäule (4') oder direkt in die Apheresesäule (4") mündet, von einem Punkt (P5) in der zumindest einen Anschlussleitung (11) ausgeht.

Die vorliegende Erfindung betrifft daher auch eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von einem Punkt (P5) in der mindestens einen Anschlussleitung (11) zum Anschluss von zumindest einem Flüssigkeitsbehälter (F) an die arterielle Leitung (5) oder den Zellseparator (7) abgeht und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind.

Bevorzugt ist eine Apheresevorrichtung (II), wobei die Apheresevorrichtung (II) zwei Anschlussleitungen (11', 11') zum Anschluss von jeweils einem Flüssigkeitsbehälter (F1, F2) an die arterielle Leitung (5) oder den Zellseparator (7) aufweist, und zwei Regenerationsleitungen (14', 14") von zwei Flüssigkeitsbehältern (F1, F2) oder den zwei Anschlussleitungen (11', 11") abgehen und in die Plasmaleitung (8A) oder in den Bypass-Leitungsabschnitt (12') der Bypass-Leitung (12) oder direkt in Apheresesäule (4') oder direkt die Apheresesäule (4") führen.

Eine besonders bevorzugte Ausführungsform der zugrundeliegenden Erfindung betrifft eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zwei Anschlussleitungen (11', 11") zum Anschluss von zwei Flüssigkeitsbehältern (F1, F2) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des Bypass-Leitungsabschnitt (12') der Bypass-Leitung (12) abgeht, eine Abfallleitung (13"), die direkt von der Apheresesäule (14") abgeht oder von dem Bypass-Leitungsabschnitt (12") der Bypass-Leitung (12) in Flussrichtung vor der Einmündung der Bypass-Leitungsabschnitt (12') der Bypass-Leitung (12),
   und
zwei Regenerationsleitungen (14', 14"), die von den zwei Flüssigkeitsbehältern (F1, F2) oder den zwei Anschlussleitungen (11', 11") abgehen und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet,
   und
wobei die zweite Apheresesäule (4") parallel zur ersten Apheresesäule (4') geschaltet ist und beide Apheresesäulen (4', 4") nicht zeitgleich zur CRP-Entfernung nutzbar sind, d.h. nur abwechselnd betreibbar sind.

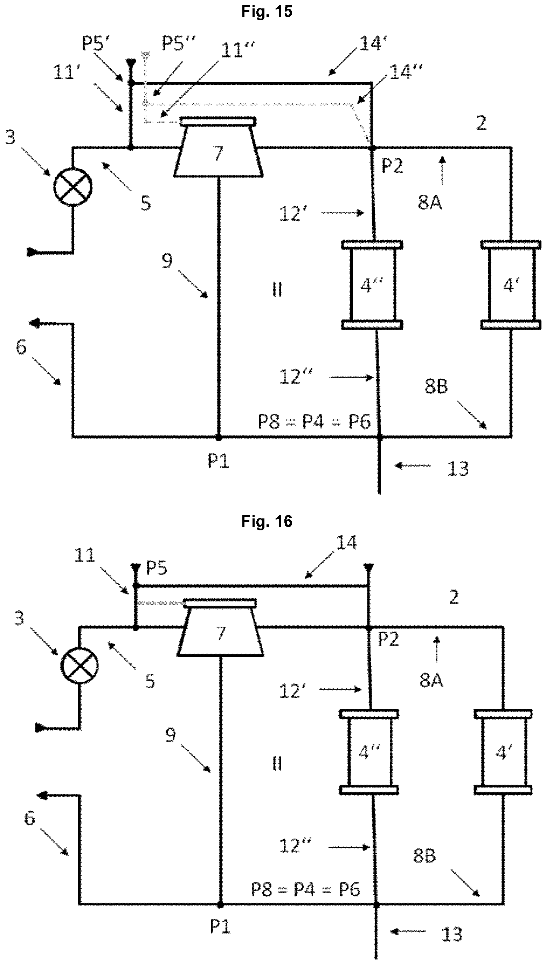

Ebenso sind Ausführungsformen denkbar, in denen eine Regenerationsleitung (14), die in die Plasmaleitung (8A) oder in den Bypass-Leitungsabschnitt (12') der Bypass-Leitung (12) oder direkt in die Apheresesäule (4') oder direkt in die Apheresesäule (4") führt und die von einem Punkt (P5) in der zumindest einen Anschlussleitung (11) ausgeht, zumindest einen zusätzlichen Anschluss für einen Flüssigkeitsbehälter aufweist (Fig. 16).

In Ausführungsformen der vorliegenden Erfindung mit mehreren Anschlussleitungen (11', 11", 11'", etc.) und mehreren Regenerationsleitungen (14', 14", 14'", etc.) ist es möglich, dass jeweils eine Anschlussleitung mit jeweils einer Regenerationsleitung in Verbindung steht, welche wiederum nach dem Punkt (P2) in die Plasmaleitung (8A) oder in den Bypass-Leitungsabschnitt (12') der Bypass-Leitung (12) oder direkt in Apheresesäule (4') oder direkt in die Apheresesäule (4") mündet. Hierbei kann jede Regenerationsleitung unabhängig von den anderen Regenerationsleitungen in die Plasmaleitung (8A) oder in den Bypass-Leitungsabschnitt (12') der Bypass-Leitung (12) oder direkt in Apheresesäule (4') oder direkt in die Apheresesäule (4") münden. Bevorzugt ist jedoch, wenn alle Regenerationsleitungen direkt in die Apheresesäulen (4'; 4"), bevorzugt am Punkt (P2) in das extrakorporale Zirkulationssystem (2) münden. Eine derartige beispielhafte Ausführungsform sei anhand von Fig. 7 erläutert. Hierin besitzt die Apheresevorrichtung (II) eine erste Anschlussleitung (11'), die erstens in die arterielle Leitung (5) führt und von der zweitens am Punkt (P5') eine erste Regenerationsleitung (14') abzweigt. Die Apheresevorrichtung (II) besitzt zudem eine zweite Anschlussleitung (11"), die erstens direkt in den Zellseparator (7) führt und von der zweitens am Punkt (P5") eine zweite Regenerationsleitung (14") abzweigt. Beide Regenerationsleitungen münden in dieser Ausführungsform am Punkt (P2) in das extrakorporale Zirkulationssystem (2).

Bevorzugt ist demnach eine Apheresevorrichtung (II), wobei die Apheresevorrichtung (II) zwei Anschlussleitungen (11', 11") zum Anschluss von jeweils zumindest einem Flüssigkeitsbehälter (F) an die arterielle Leitung (5) oder den Zellseparator (7) aufweist, und wobei die zumindest eine Regenerationsleitung (14), die in die Plasmaleitung (8A) oder in den Bypass-Leitungsabschnitt (12') der Bypass-Leitung (12) oder direkt in die Apheresesäule (4') oder direkt in die Apheresesäule (4") führt, sich an einem Punkt (P5') mit der Anschlussleitung (11') und an einem Punkt (P5") mit der Anschlussleitung (11") verbindet.

Eine bevorzugte Ausführungsform der zugrundeliegenden Erfindung betrifft eine Apheresevorrichtung (II) zur extrakorporalen Entfernung von CRP aus Blut umfassend: ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zwei Anschlussleitungen (11', 11") zum zum Anschluss von jeweils zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des Bypass-Leitungsabschnitt (12') der Bypass-Leitung (12) abgeht, eine Abfallleitung (13"), die direkt von der Apheresesäule (14") abgeht oder von dem Bypass-Leitungsabschnitt (12") der Bypass-Leitung (12) in Flussrichtung vor der Einmündung der Bypass-Leitungsabschnitt (12') der Bypass-Leitung (12),
und
zumindest eine Regenerationsleitung (14),
die in die Plasmaleitung (8A) oder in den Bypass-Leitungsabschnitt (12') der Bypass-Leitung (12) oder direkt in die Apheresesäule (4') oder direkt in die Apheresesäule (4") führt, sich an einem Punkt (P5') mit der Anschlussleitung (11') und an einem Punkt (P5") mit der Anschlussleitung (11") verbindet,
die von dem zumindest einen Flüssigkeitsbehälter (F) oder der zumindest einen Anschlussleitung (11) abgeht und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet,
und
wobei eine zweite Apheresesäule (4") parallel zur ersten Apheresesäule (4') geschaltet ist und beide Apheresesäulen (4', 4") nicht zeitgleich zur CRP-Entfernung nutzbar sind.

Somit sind insbesondere Ausführungsformen der Apheresevorrichtung bevorzugt, wobei die Apheresevorrichtung (II) zwei Anschlussleitungen (11', 11") zum Anschluss von jeweils einem Flüssigkeitsbehälter (F1, F2) an die arterielle Leitung (5) oder den Zellseparator (7) aufweist, und wobei die zumindest eine Regenerationsleitung (14), die in die Plasmaleitung (8A) oder in den Bypass-Leitungsabschnitt (12') der Bypass-Leitung (12) oder direkt die Apheresesäule (4') oder direkt in die Apheresesäule (4") führt, sich an einem Punkt (P5') mit der Anschlussleitung (11') und an einem Punkt (P5") mit der Anschlussleitung (11") verbindet und wobei eine Regenerationsleitung (14') von dem Flüssigkeitsbehälter (F1) oder von der vom Flüssigkeitsbehälter (F1) abgehenden Anschlussleitung (11') zur Apheresesäule (4') oder zur Apheresesäule (4") oder zur Plasmaleitung (8A') oder zur Plasmaleitung (8A") und eine Regenerationsleitung (14") von dem Flüssigkeitsbehälter (F2) oder der vom Flüssigkeitsbehälter (F2) abgehenden Anschlussleitung (11") zur Apheresesäule (4') oder zur Apheresesäule (4") oder zur Plasmaleitung (8A) oder zur Bypass-Leitungsabschnitt (12') der Bypass-Leitung (12) oder in die Regenerationsleitung (14') führt.

Vorzugsweise enthalten der Flüssigkeitsbehälter (F1) eine physiologische Lösung und der Flüssigkeitsbehälter (F2) eine Citrat-Lösung.

Somit ist besonders bevorzugt, wenn die Apheresevorrichtung (II) eine Anschlussleitung (11') für den Anschluss eines Flüssigkeitsbehälters (F1) und eine Anschlussleitung (11') für den Anschluss eines Flüssigkeitsbehälters (F2) aufweist und die Anschlussleitung (11') in die arterielle Leitung (5) oder in den Zellseparator (7) mündet und die Anschlussleitung (11") in die arterielle Leitung (5) oder in den Zellseparator (7) oder in Anschlussleitung (11') und damit letztendlich auch in die arterielle Leitung (5) oder in den Zellseparator (7) mündet und eine Regenerationsleitung (14') von dem Flüssigkeitsbehälter (F1) oder von der Anschlussleitung (11') zur Apheresesäule (4') oder zur Apheresesäule (4") oder zur Plasmaleitung (8A) oder zur Plasmaleitung (8A") führt und eine Regenerationsleitung (14") von dem Flüssigkeitsbehälter (F2) oder von der Anschlussleitung (11") zur Apheresesäule (4') oder zur Apheresesäule (4") oder zur Plasmaleitung (8A') oder zum Bypass-Leitungsabschnitt (12') der Bypass-Leitung (12) oder in die Regenerationsleitung (14') führt.

Ausführungsformen der Apheresevorrichtung (II) sind daher insbesondere bevorzugt, bei denen die Apheresevorrichtungen (II) eine Anschlussleitung (11') zum Anschluss von einem Flüssigkeitsbehälter (F1) an die arterielle Leitung (5) oder den Zellseparator (7) und eine Anschlussleitung (11") zum Anschluss von einem Flüssigkeitsbehälter (F2) an die arterielle Leitung (5) oder den Zellseparator (7) aufweist, und wobei eine Regenerationsleitung (14') von dem Flüssigkeitsbehälter (F1) oder der Anschlussleitung (11') abgeht und in die Plasmaleitung (8A) oder in den Bypass-Leitungsabschnitt (12') der Bypass-Leitung (12) oder direkt in die Apheresesäule (4') oder direkt in die Apheresesäule (4") führt und eine Regenerationsleitung (14") von einem Flüssigkeitsbehälter (F2) oder der Anschlussleitung (11") abgeht und in die Plasmaleitung (8A) oder in den Bypass-Leitungsabschnitt (12') der Bypass-Leitung (12) oder in die Regenerationsleitung (14') oder direkt in die Apheresesäule (4') oder direkt in die Apheresesäule (4") führt.

Eine bevorzugte Ausführungsform der zugrundeliegenden Erfindung betrifft eine Apheresevorrichtung (II) zur extrakorporalen Entfernung von CRP aus Blut umfassend: ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11') zum Anschluss von einem Flüssigkeitsbehälter (F1) an die arterielle Leitung (5) oder den Zellseparator (7) und eine Anschlussleitung (11") zum Anschluss von einem Flüssigkeitsbehälter (F2) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des Bypass-Leitungsabschnitt (12') der Bypass-Leitung (12) abgeht, eine Abfallleitung (13"), die direkt von der Apheresesäule (14") abgeht oder von dem Bypass-Leitungsabschnitt (12") der Bypass-Leitung (12) in Flussrichtung vor der Einmündung der Bypass-Leitungsabschnitt (12') der Bypass-Leitung (12),
und
eine Regenerationsleitung (14') von dem Flüssigkeitsbehälter (F1) oder der Anschlussleitung (11') abgeht und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet, und eine Regenerationsleitung (14") von einem Flüssigkeitsbehälter (F2) oder der Anschlussleitung (11") abgeht und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet
wobei eine zweite Apheresesäule (4") parallel zur ersten Apheresesäule (4') geschaltet ist und beide Apheresesäulen (4', 4") nicht zeitgleich zur CRP-Entfernung nutzbar sind. Vorzugsweise handelt es sich bei dem Flüssigkeitsbehälter (F1) um einen Behälter für eine physiologische Natriumchlorid-Lösung und bei dem Flüssigkeitsbehälter (F2) um einen Behälter für eine Citrat-Lösung.

Die vorliegende Erfindung ist daher ebenso gerichtet auf eine erfindungsgemäße Apheresevorrichtung (II), wobei die Plasmaleitung (8A) und der Bypass-Leitungsabschnitt (12') der Bypass-Leitung (12) von einem Punkt (P2) auseinanderlaufen, und die Plasmaleitung (8B) und der Bypass-Leitungsabschnitt (12") der Bypass-Leitung (12) Plasmaleitung (8B) am Punkt (P6) zusammenlaufen, und die Abfallleitung (13') von einem Punkt (P4) von der Plasmaleitung (8B) abgeht und die Abfallleitung (13") von einem Punkt (P8) von dem Bypass-Leitungsabschnitt (12") der Bypass-Leitung (12) abgeht, und die zumindest eine Regenerationsleitung (14) am Punkt (P2) in das extrakorporale Zirkulationssystem (2) mündet.

Eine bevorzugte Ausführungsform der zugrundeliegenden Erfindung betrifft eine Apheresevorrichtung (II) zur extrakorporalen Entfernung von CRP aus Blut umfassend: ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (II), zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein **Bypass-Leitungsabschnitt (12')** der Bypass-Leitung (12) von der Plasmaleitung (8A) abzweigt und in die zweite Apheresesäule (4') mündet und der **Bypass-Leitungsabschnitt (12")** der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des **Bypass-Leitungsabschnitt (12')** der Bypass-Leitung (12) abgeht, eine Abfallleitung (13"), die direkt von der Apheresesäule (14") abgeht oder von dem Bypass-Leitungsabschnitt (12") der Bypass-Leitung (12) in Flussrichtung vor der Einmündung der **Bypass-Leitungsabschnitt (12') der** Bypass-Leitung (12), wobei die Plasmaleitung (8A) und der Bypass-Leitungsabschnitt (12') der Bypass-Leitung (12) von einem Punkt (P2) auseinanderlaufen, und die Plasmaleitung (8B) und der Bypass-Leitungsabschnitt (12") der Bypass-Leitung (12) an einem Punkt (P6) zusammenlaufen, und
zumindest eine Regenerationsleitung (14), die von dem zumindest einen Flüssigkeitsbehälter (F) oder der zumindest einen Anschlussleitung (11) abgeht und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet, und
die Abfallleitung (13') von einem Punkt (P4) von der Plasmaleitung (8B) abgeht und die Abfallleitung (13") von einem Punkt (P8) von dem Bypass-Leitungsabschnitt (12") der Bypass-Leitung (12) abgeht, und
die zumindest eine Regenerationsleitung (14) am Punkt (P2) in das extrakorporale Zirkulationssystem (2) mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind, d.h. nur abwechselnd nutzbar sind.

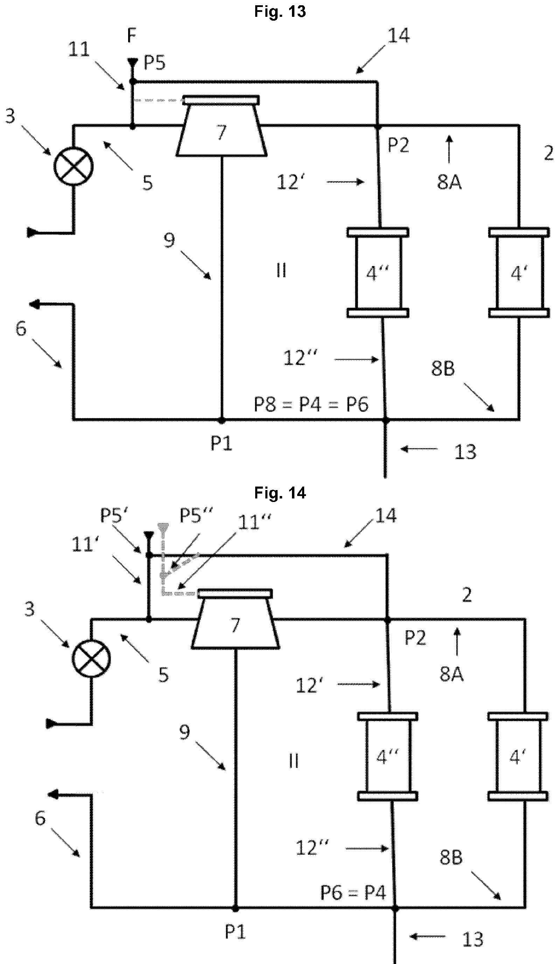

Um das Totvolumen des Systems noch weiter zu verringern, ist es noch weiter bevorzugt, wenn nicht nur die Regenerationsleitung (14) an dem Punkt (P2), an dem die Plasmaleitung (8A) und der Bypass-Leitungsabschnitt (12') der Bypass-Leitung (12) auseinanderlaufen, in das extrakorporale Zirkulationssystem mündet, sondern wenn auch die Ablaufleitungen (13', 13") von demselben Punkt (P6), an dem die Plasmaleitung (8B) und der Bypass-Leitungsabschnitt (12") der Bypass-Leitung (12) zusammenlaufen, abzweigen. Anders ausgedrückt ist bevorzugt, wenn der Punkt (P6), an dem die Plasmaleitungen (8B) und der Bypass-Leitungsabschnitt (12") der Bypass-Leitung (12) zusammenlaufen, der Punkt (P8), an dem die Abfallleitung (13") abzweigt und der Punkt (P4), an dem Abfallleitung (13') abzweigt übereinstimmen, d.h. wenn P8 = P4 = P6 ist (siehe hierzu Fig. 12 und Fig. 13).

Die vorliegende Erfindung ist daher ebenso gerichtet auf eine erfindungsgemäße Apheresevorrichtung (II), wobei die Plasmaleitung (8B) und der Bypass-Leitungsabschnitt (12") der Bypass-Leitung (12) an einem Punkt (P6) zusammenlaufen, und die Abfallleitung (13") von einem Punkt (P8) von dem Bypass-Leitungsabschnitt (12") der Bypass-Leitung (12) abgeht und die Abfallleitung (13') von einem Punkt (P4) von der Plasmaleitung (8B) abgeht, und die zumindest eine Regenerationsleitung (14) am Punkt (P2) in das extrakorporale Zirkulationssystem (2) mündet und wobei der Punkt (P6), der Punkt (P4) und der Punkt (P8) identisch sind.

Gemäß der vorliegenden Erfindung umfasst eine erfindungsgemäße Ausführungsform der Apheresevorrichtung (II) zur extrakorporalen Entfernung von CRP aus Blut zwei Apheresesäulen (4', 4") zur affinitätschromatographischen Entfernung von CRP aus dem Blut oder Blutplasma, dessen Funktion in der Bindung von CRP besteht, welches sich im Blut bzw. im Blutplasma eines Patienten befindet und das durch die Apheresesäule (4') oder (4") hindurch geleitet wird.

### Verfahren

Die vorliegende Erfindung betrifft auch ein Verfahren zur Regeneration einer Apheresesäule (4) zur affinitätschromatographischen Entfernung von CRP in einer Apheresevorrichtung (1), wobei das Verfahren die Regeneration im laufenden Betrieb ermöglicht und durch die folgende Schritte gekennzeichnet ist:
(A) Beginn der Umleitung des abgetrennten Plasmas aus der Plasmaleitung (8A) in die Bypass-Leitung (12) und damit Stoppen der Einleitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung und Stoppen der Umleitung des abgetrennten Plasmas aus der Plasmaleitung (8A) in die Bypass-Leitung (12) und damit Einleitung des abgetrennten Plasmas aus der Plasmaleitung (8A) in die Apheresesäule (4),
(E) Schließung der Abfallleitung (13) und Weiterleiten des aus der Apheresesäule (4) austretenden Flüssigkeitsstromes in die venöse Leitung (6).

Unter dem Begriff "Stoppen der Einleitung des abgetrennten Plasmas" gemäß Schritt (A) kann je nach Ausführungsform der vorliegenden Erfindung die Verwendung von Schlauchklemmen, Steuerungselementen, Ventilen und/oder Schlauchpumpen zum Verhindern des weiteren Flusses von Blutplasma in die Plasmaleitung (8A) oder in den Bypass-Leitungsabschnitt (12') der Bypass-Leitung (12) oder in die in die Apheresesäule (4') oder (4") verstanden werden.

Unter dem Begriff "Stoppen der Einleitung von Regenerationslösung" gemäß Schritt (D) kann je nach Ausführungsform der vorliegenden Erfindung die Verwendung von Schlauchklemmen, Steuerungselementen, Ventilen und/oder Schlauchpumpen zum Verhindern des weiteren Flusses von Regenerationslösung in die Plasmaleitung (8A) bzw. in die Apheresesäule (4) verstanden werden. Hierbei soll verstanden werden, dass in Ausführungsformen, bei denen nur eine Regenerationslösung verwendet wird, die Einleitung ebendieser gestoppt wird. In Ausführungsformen, in denen mehrere Regenerationslösungen sukzessive eingeleitet werden, bedeutet dies, dass die Einleitung der zuletzt verwendeten Regenerationslösung gestoppt wird und damit auch die Einleitung jeglicher Regenerationslösung beendet wird.

Unter dem Begriff "Schließen der Abfallleitung (13)" gemäß Schritt (E) kann je nach Ausführungsform der vorliegenden Erfindung die Verwendung von Schlauchklemmen, Steuerungselementen, Ventilen und/oder Schlauchpumpen zum Verhindern des weiteren Flusses des aus der Apheresesäule (4) austretenden Flüssigkeitsstromes verstanden werden. Hierbei soll verstanden werden, dass in Ausführungsformen, bei denen nur eine Regenerationslösung verwendet wird, die Einleitung eben dieser gestoppt wird. In Ausführungsformen, in denen mehrere Regenerationslösungen sukzessive eingeleitet werden, bedeutet dies, dass die Einleitung der zuletzt verwendeten Regenerationslösung gestoppt wird und damit auch die Einleitung jeglicher Regenerationslösung beendet wird.

Durch "Weiterleiten des aus der Apheresesäule (4) austretenden Flüssigkeitsstromes" gemäß Schritt (E) fließt das abgetrennte Plasma fortan nach Durchlaufen der Apheresesäule (4) wieder in die Plasmaleitung (8B) und von dort weiter durch die venöse Leitung (6) zurück zum Patienten. Je nach Ausführungsform der vorliegenden Erfindung können zur Änderung der Flussrichtung des aus der Apheresesäule (4) austretenden Flüssigkeitsstromes Schlauchklemmen, Steuerungselemente, Ventile und/oder Schlauchpumpen verwendet werden.

Ferner betrifft die vorliegende Erfindung ein Verfahren zur Regeneration einer Apheresesäule (4) zur affinitätschromatographischen Entfernung von CRP in einer Apheresevorrichtung (1) wie hierin beschrieben, wobei das Verfahren die Regeneration im laufenden Betrieb durch Umschalten von einem Apherese-Modus in einen Regenerations-Modus ermöglicht, wobei im Apherese-Modus Plasma, das mittels des Zellseparators (7) aus Blut abgetrennt wird, über die Plasmaleitung (8A) in die Apheresesäule (4) geleitet wird und der aus der Apheresesäule (4) austretende Flüssigkeitsstrom über die Plasmaleitung (8B) in die venöse Leitung (6) geleitet wird;
und wobei der Regenerations-Modus durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4),
(E) Schließung der Abfallleitung (13) und Weiterleiten des aus der Apheresesäule (4) austretenden Flüssigkeitsstromes in die venöse Leitung (6) und damit wieder ein Umschalten in den Apherese-Modus.

Hinsichtlich der beiden vorgenannten Verfahren handelt es sich bei der Regenerationslösung vorzugsweise um eine Kochsalzlösung bzw. physiologische NaCl-Lösung.

Ferner sind Verfahren bevorzugt, bei denen Schritt (C) initiiert wird, nachdem ein Gesamtvolumen X an Regenerationslösung/-en in die Plasmaleitung (8A) bzw. direkt in die Apheresesäule (4) eingeleitet wurde, wobei X mindestens 75 % des Volumens der Vorrichtung zwischen dem Punkt, an dem die Regenerationsleitung (14) in Flussrichtung nach der Abzweigung der Bypass-Leitung (12) in das extrakorporale Zirkulationssystem (2) mündet, und dem Punkt, an dem die Abfallleitung (13) dem extrakorporalen Zirkulationssystem (2) entspringt, entspricht. Hier handelt es sich bei der Regenerationslösung beispielsweise um eine Kochsalzlösung bzw. eine physiologische Kochsalzlösung.

Zudem sind Verfahren bevorzugt, bei denen Schritt (E) initiiert wird, nachdem ein Volumen Y an Plasma in die Plasmaleitung (8A) oder direkt in die Apheresesäule (4) eingeleitet wurde, wobei Y mindestens 90 % des Volumens der Vorrichtung zwischen dem Punkt, an dem die Regenerationsleitung (14) in Flussrichtung nach der Abzweigung der Bypass-Leitung (12) in das extrakorporale Zirkulationssystem (2) mündet, und dem Punkt, an dem die Abfallleitung (13) dem extrakorporalen Zirkulationssystem (2) entspringt, entspricht.

**"Im laufenden Betrieb",** wie hierin verwendet, bedeutet, dass zur Durchführung des erfindungsgemäßen Verfahrens zur Regeneration einer Apheresesäule (4) die Blutabnahme und -zufuhr sowie der Betrieb des Zellseparators nicht gestoppt werden müssen. Anders ausgedrückt wird während des erfindungsgemäßen Verfahrens zur Regeneration einer Apheresesäule (4) das kontinuierlich gewonnene Plasma über die Bypass-Leitung (12) unter Umgehung der Apheresesäule (4) mit den Zellbestandteilen zusammengeführt und dem Patienten zugeführt. Während der Zeit, in der die Umleitung des Plasmas über die Bypass-Leitung (12) stattfindet, wird die in der Regel in ihrer Kapazität geminderte Apheresesäule (4) regeneriert. Somit wird der Kreislauf des Patienten nicht belastet, weil das kontinuierlich entnommene Blut ohne Verzögerung dem Patienten wieder zurückgeführt wird.

"Im laufenden Betrieb" wie hierin verwendet bedeutet demnach nicht, dass zur Durchführung des erfindungsgemäßen Verfahrens zur Regeneration einer Apheresesäule (4) die kontinuierliche Plasmagewinnung unterbrochen werden muss. Ferner bedeutet es auch nicht, dass während der Regeneration der Apheresesäule die CRP-Abreicherung stattfindet.

Somit ist bei den beiden vorgenannten und den allgemein hierin offenbarten Verfahren bevorzugt, dass die Einleitung von Regenerationslösung die Einleitung einer einzelnen Regenerationslösung oder die sukzessive Einleitung mehrerer Regenerationslösungen umfasst.

Für den Fachmann ist völlig klar, dass ein initialer Spülvorgang des Adsorbers bzw. des gesamten Systems vor der Ausführung des erfindungsgemäßen Verfahrens stattgefunden haben muss. Damit verbunden ist ein Vorfüllen des gesamten Schlauchsystems. Hierzu können unter Umständen weitere Anschlüsse an dem System vorhanden sein, die ein Durchspülen des gesamten Systems ermöglichen. Nachdem der Patient vom Schlauchsystem getrennt wurde besteht die Möglichkeit der Konservierung des Adsorbers, sodass dieser für eine weitere Behandlung an demselben Patienten wieder verwendet werden kann.

Anders ausgedrückt betrifft die vorliegende Erfindung auch ein erfindungsgemäßes Verfahren zur Regeneration einer Apheresesäule (4) zur affinitätschromatographischen Entfernung von CRP in der Apheresevorrichtung (1), wobei das Verfahren die Regeneration im laufenden Betrieb ermöglicht und durch die folgenden Schritte gekennzeichnet ist:
(A) Umleitung des abgetrennten Plasmas aus der Plasmaleitung (8A) in die Bypass-Leitung (12),
(B) Einleitung zumindest einer Regenerationslösung aus einem Flüssigkeitsbehälter an der Anschlussleitung (11) über die Regenerationsleitung (14) in die Plasmaleitung (8A) oder direkt in die Apheresesäule (4),
(C) Umleitung des aus der Apheresesäule (4) austretenden Flüssigkeitsstromes von der Plasmaleitung (8B) in die Abfallleitung (13),
(D) Umleitung des abgetrennten Plasmas aus der Plasmaleitung (8A) in die Apheresesäule (4) und Stoppen der Einleitung von Regenerationslösung,
(E) Schließung der Abfallleitung (13) und Weiterleiten des aus der Apheresesäule (4) austretenden Flüssigkeitsstromes in die venöse Leitung (6).

Der Begriff "Umleitung" wie hierin verwendet betrifft eine Änderung der Fließrichtung der jeweiligen Flüssigkeit. Während des Behandlungsmodus strömt das abgetrennte Plasma durch die Plasmaleitung (8A) in die Apheresesäule (4). Nach Verlassen der Apheresesäule (4) strömt das abgereicherte Plasma durch die Plasmaleitung (8B) in die venöse Leitung (6).

Durch die "Umleitung" der Fließrichtung des abgetrennten Plasmas gemäß Schritt (A) fließt das abgetrennte Plasma fortan nicht mehr durch die Apheresesäule (4), sondern umgeht diese, indem es in die Bypass-Leitung (12) umgeleitet wird.

Unter dem Begriff "Einleitung" wie hierin verwendet gemäß Schritt (B) kann je nach Ausführungsform der vorliegenden Erfindung die Einspeisung zumindest einer Regenerationslösung (unter Verwendung bzw. Betätigung von Schlauchklemmen, Steuerungselementen, Ventilen und/oder Schlauchpumpen) in die Plasmaleitung (8A) bzw. in die Apheresesäule (4) verstanden werden.

Durch die "Umleitung" der Fließrichtung des aus der Apheresesäule (4) austretenden Flüssigkeitsstromes gemäß Schritt (C) fließt die austretende Flüssigkeit fortan nicht mehr in die Plasmaleitung (8B) sondern direkt in die Abfallleitung (13). Erfindungsgemäß ist es bevorzugt, dass die Abfallleitung (13) direkt bzw. unmittelbar von bzw. nach der Apheresesäule (4) abzweigt, um somit das Volumen an Regenerationslösung, das nötig ist um die Apheresesäule (4) zu regenerieren, minimiert wird. Im Sinne der Erfindung kann die Abfallleitung (13) auch aus der Plasmaleitung (8B) abzweigen und muss somit nicht direkt aus der Apheresesäule abzweigen.

Durch die "Umleitung" der Fließrichtung des abgetrennten Plasmas gemäß Schritt (D) fließt das abgetrennte Plasma fortan wieder durch die Apheresesäule (4) und nicht mehr in die Bypass-Leitung (12). In bestimmten Ausführungsformen ist eine Pumpe in der Bypass-Leitung (12) vorhanden, wodurch das in der Bypass-Leitung (12) vorhandene Plasma nach Umleitung gemäß Schritt (D) in die Plasmaleitung (8B) und über die venöse Leitung (6) in den Patienten gepumpt wird. Hierbei wird bevorzugt das in der Bypass-Leitung befindliche Plasma durch eine NaCI-Lösung aus der Regenerationsleitung (14) verdrängt. Bevorzugt handelt es sich dabei um eine 0,9%ige NaCl Lösung. Ebenso wäre es denkbar, dass ein separater Flüssigkeitsbehälter an die Bypass-Leitung (12) angeschlossen werden kann, durch den besagte NaCI-Lösung zur Verdrängung bereitgestellt wird.

Daher betrifft eine insbesondere bevorzugte Ausführungsform der vorliegenden Erfindung ein erfindungsgemäßes Verfahren zur Regeneration einer Apheresesäule (4) zur affinitätschromatographischen Entfernung von CRP in der Apheresevorrichtung (1), wobei das Verfahren die Regeneration im laufenden Betrieb ermöglicht und durch die im folgenden Verfahren offenbarten Schritte gekennzeichnet ist.

Besonders bevorzugt ist daher ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Spüllösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4),
(E) Stoppen der Einleitung der Regenerationslösung und Übergang zur Einleitung der Spüllösung über die zumindest eine Regenerationsleitung (14) in die Plasmaleitung (8A) oder direkt in die Apheresesäule (4),
(F) Stoppen der Einleitung von Spüllösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(G) Schließung der Abfallleitung (13) und Weiterleiten des aus der Apheresesäule (4) austretenden Flüssigkeitsstromes in die venöse Leitung (6)..

Alternativ ist auch besonders bevorzugt ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Spüllösung über die zumindest eine Regenerationsleitung (14) in die Plasmaleitung (8A) oder direkt in die Apheresesäule (4),
(C) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4),
(D) Beginn der Umleitung des aus der Apheresesäule (4) austretenden Flüssigkeitsstromes von der Plasmaleitung (8B) in die Abfallleitung (13),
(E) Stoppen der Einleitung der Regenerationslösung und Übergang zur Einleitung der Spüllösung über die zumindest eine Regenerationsleitung (14) in die Plasmaleitung (8A) oder direkt in die Apheresesäule (4),
(F) Schließung der Abfallleitung (13) und Weiterleiten des aus der Apheresesäule (4) austretenden Flüssigkeitsstromes in die venöse Leitung (6);
(G) Stoppen der Einleitung von Spüllösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bei den beiden vorgenannten Verfahren wird zusätzlich zur Regenerationslösung noch eine Spüllösung eingesetzt. Die Spüllösung ist vorzugsweise physiologisch verträglich und dient vorrangig zur Verdrängung des Blutplasmas aus der Plasmaleitung (8A) ab dem Punkt P2, aus der Apheresesäule (4) sowie aus der Plasmaleitung (8B) bis zum Punkt P4. Die Spüllösung dient weniger oder nicht zur Regeneration der Apheresesäule (4). Durch die Spüllösung wird daher der Plasmaverlust minimiert oder sogar vollständig vermieden. Erst wenn das Blutplasma aus dem mit Regenerationslösung zu durchspülenden Abschnitt der Apheresevorrichtung (1) weitestgehend bis vollständig verdrängt ist, wird die Regenerationslösung eingeleitet, um die Apheresesäule (4) zu regenerieren. Nach erfolgter Regeneration wird dann erst wieder Spüllösung in den mit Regenerationslösung durchspülten Abschnitt der Apheresevorrichtung (1) (also in Flussrichtung von Punkt P2 durch die Apheresesäule (4) bis zum Punkt P4) geleitet, bis die Regenerationslösung vollständig durch die Abfallleitung (13) entsorgt worden ist. Erst dann wird die Bypass-Leitung (12) geschlossen und wieder Blutplasma durch die Apheresesäule (4) geleitet. Bei den beiden vorgenannten Verfahren sind die Schritte (C) und (D) vertauschbar, d.h. können in beliebiger Reihenfolge und auch zeitgleich erfolgen und können auch in einem Schritt zusammengefasst werden. Die Ausführung des Schrittes (D) vor dem Schritt (C) ist jedoch bevorzugt.

Bei diesem Verfahren handelt es sich bei der Spüllösung vorzugsweise um eine physiologische NaCI-Lösung und bei der Regenerationslösung um eine Citrat-Lösung.

Die bevorzugte Ausführungsform des erfindungsgemäßen Verfahrens dient zur effizienteren Durchführung des Verfahrens ohne Verlust von Blutplasma. Durch die zeitgleiche Umleitung des abgetrennten Plasmas und der parallelen Einleitung der Spüllösung in die Apheresesäule (4) entsteht kein Verlust oder kein nennenswerter Verlust von Blutplasma. Ferner ist an der bevorzugten Ausführungsform von Vorteil, dass ein Durchmischen von Regenerationslösung und Bluplasma vollständig vermieden wird. Dadurch wird sichergestellt, dass keine Regenerationslösung in den Patienten gelangt und andererseits kein Verlust von Blutplasma für den Patienten auftritt.

Dies wird durch die sequenzielle Abfolge der Schritte (B) bis (E) sichergestellt. Eine Verdünnung des Blutplasmas findet wenn überhaupt nur durch Spüllösung statt. Hingegen wird eine Vermischung von Blutplasma mit Regenerationslösung vollständig vermieden.
Das Volumen an Spüllösung gemäß Schritt (B) entspricht vorzugsweise dem 3- bis 4-fachen Volumen der Matrix der Apheresesäule (4).
Minimal entspricht das Volumen an Spüllösung gemäß Schritt (B) dem Volumen der Plasmaleitung (8A) ab Punkt P2 bis zur Apheresesäule (4) zuzüglich dem Volumen der Matrix der Apheresesäule (4) und zuzüglich dem Volumen der Plasmaleitung (8B) ab der Apheresesäule (4) bis zum Punkt P4.

Das Volumen an Regenerationslösung gemäß Schritt (C) entspricht vorzugsweise dem 2-bis 100-fachen Volumen der Matrix der Apheresesäule (4).

Das Volumen an Spüllösung gemäß Schritt (E) entspricht vorzugsweise dem 2- bis 4-fachen Volumen der Matrix der Apheresesäule (4).
Zumindest entspricht das Volumen an Spüllösung gemäß Schritt (E) dem Volumen der Plasmaleitung (8A) ab Punkt P2 bis zur Apheresesäule (4) zuzüglich dem Volumen der Matrix der Apheresesäule (4) und zuzüglich dem Volumen der Plasmaleitung (8B) ab der Apheresesäule (4) bis zum Punkt P4.

Gemäß dieser noch bevorzugteren Ausführungsform wird eine Verdünnung des Plasmas weitgehend vermieden und eine Durchmischung mit Regenerationslösung vollständig verhindert. Der Benutzer oder die Benutzerin ist mit keiner zu großen Komplexität hinsichltich der Benutzung der Apheresevorrichtung (1) konfrontiert. In einer alternativen Ausführungsform können somit die Verfahrensschritte auch manuell betätigt werden, ohne zu komplex für den Benutzer oder die Benutzerin zu erscheinen oder zu sein.

Das "Volumen der Matrix der Apheresesäule", wie hierin verwendet, bedeutet das Volumen der festen Phase innerhalb der Säule, welche wiederum ein Matrixsubstratmaterial und daran gebundene Verbindungen umfasst, die die Eigenschaft haben, CRP spezifisch zu binden. Abzugrenzen hiervon ist das "Totvolumen der Apheresesäule", d.h. der Raum innerhalb der Säule, der der mobilen Phase (z.B. des Plasmas) zur Verfügung steht. Das "Totvolumen der Apheresesäule" ergibt sich aus der Differenz aus dem durch das Gehäuse der Apheresesäule umschlossenen Volumen und dem Volumen, das durch die gequollene Matrix beansprucht wird (d.h. dem "Volumen der Matrix der Apheresesäule").

Ein weiterer Aspekt der vorliegenden Erfindung ist gerichtet auf ein Verfahren zur Regeneration einer Apheresesäule (4') zur affinitätschromatographischen Entfernung von CRP während des laufenden Betriebes einer zweiten Apheresesäule (4") in einer Apheresevorrichtung (II) umfassend die folgenden Schritte:
(A) Ausgehend vom Fluss des Blutplasmas durch die Apheresesäule (4"), Beginn der Einleitung des abgetrennten Blutplasmas über die Plasmaleitung (8A) in die Apheresesäule (4') und Leiten des CRP-abgereicherten Blutplasmas in die venöse Leitung (6) und damit Stoppen der Einleitung des abgetrennten Plasmas über den Bypass-Leitungsabschnitt (12') der Bypass-Leitung (12) in die Apheresesäule (4"),
(B) Beginn der Einleitung von Regenerationslösung über die zumindest eine Regenerationsleitung (14) in den Bypass-Leitungsabschnitt (12') der Bypass-Leitung (12) oder direkt in die Apheresesäule (4"),
(C) Beginn der Umleitung des aus der Apheresesäule (4") austretenden Flüssigkeitsstromes von dem Bypass-Leitungsabschnitt (12") der Bypass-Leitung (12) in die Abfallleitung (13"),
(D) Beginn der Einleitung des abgetrennten Blutplasmas über den Bypass-Leitungsabschnitt (12') der Bypass-Leitung (12) in die Apheresesäule (4") und Leiten des CRP-abgereicherten Plasmas in die venöse Leitung (6) und damit Stoppen der Einleitung des abgetrennten Blutplasmas über die Plasmaleitung (8A) in Apheresesäule (4')
(E) Schließung der Abfallleitung (13") und Beginn der Umleitung des aus der Apheresesäule (4') austretenden Flüssigkeitsstromes von der Plasmaleitung (8B) in die Abfallleitung (13').

Hinsichtlich der beiden vorgenannten Verfahren handelt es sich bei der Regenerationslösung vorzugsweise um eine Kochsalzlösung bzw. physiologische NaCl-Lösung.

**"Im laufenden Betrieb",** wie in diesem Zusammenhang verwendet, bedeutet, dass zur Durchführung des erfindungsgemäßen Verfahrens zur Regeneration einer Apheresesäule (4') oder Regeneration einer Apheresesäule (4") die Blutabnahme und -zufuhr sowie der Betrieb des Zellseparators nicht gestoppt werden müssen. Somit wird der Kreislauf des Patienten nicht belastet, weil das kontinuierlich entnommene Blut ohne Verzögerung dem Patienten wieder zurückgeführt wird.

Anders ausgedrückt betrifft die vorliegende Erfindung in einer Ausführungsform ein Verfahren zur Regeneration einer Apheresesäule (4') zur affinitätschromatographischen Entfernung von CRP während des laufenden Betriebes einer zweiten Apheresesäule (4") in einer Apheresevorrichtung (II), umfassend die folgenden Schritte:
(A) Ausgehend vom Fluss des Blutplasmas durch die Apheresesäule (4"), Einleitung des abgetrennten Plasmas über die Plasmaleitung (8A) in die Apheresesäule (4') und Leiten des CRP-abgereicherten Plasmas in die venöse Leitung (6) und damit Stoppen der Einleitung des abgetrennten Plasmas über den Bypass-Leitungsabschnitt (12') der Bypass-Leitung (12) in die Apheresesäule (4"),
(B) Einleitung zumindest einer Regenerationslösung aus einem Flüssigkeitsbehälter an der Anschlussleitung (11) über die zumindest eine Regenerationsleitung (14) in den Bypass-Leitungsabschnitt (12') der Bypass-Leitung (12) oder direkt in die Apheresesäule (4"),
(C) Umleitung des aus der Apheresesäule (4") austretenden Flüssigkeitsstromes von dem Bypass-Leitungsabschnitt (12") der Bypass-Leitung (12) in die Abfallleitung (13"),
(D) Einleitung des abgetrennten Plasmas über den Bypass-Leitungsabschnitt (12') der Bypass-Leitung (12) in die Apheresesäule (4") und Leiten des CRP-abgereicherten Plasmas in die venöse Leitung (6) und damit Stoppen der Einleitung des abgetrennten Plasmas über die Plasmaleitung (8A) in Apheresesäule (4')
(E) Schließung der Abfallleitung (13") und Umleitung des aus der Apheresesäule (4') austretenden Flüssigkeitsstromes von der Plasmaleitung (8B) in die Abfallleitung (13').

Ferner betrifft die vorliegende Erfindung ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in die venöse Leitung (6) und damit Stoppen der Einleitung des abgetrennten Plasmas über den Bypass-Leitungsabschnitt (12') der Bypass-Leitung (12) in die Apheresesäule (4"),
(B) Beginn der Einleitung einer Spüllösung über die zumindest eine Regenerationsleitung (14) in den Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12") der Bypass-Leitung (12) in die Abfallleitung (13"),
(D) Stoppen der Einleitung der Spüllösung und Übergang zur Einleitung der Regenerationslösung über die zumindest eine Regenerationsleitung (14) in den Bypass-Leitungsabschnitt (12') der Bypass-Leitung (12) oder direkt in die Apheresesäule (4"),
(E) Stoppen der Einleitung der Regenerationslösung und Übergang zur Einleitung der Spüllösung über die zumindest eine Regenerationsleitung (14) in den Bypass-Leitungsabschnitt (12') der Bypass-Leitung (12) oder direkt in Apheresesäule (4"),
(F) Beginn der Einleitung der Spüllösung in die Plasmaleitung (8A) über die Apheresesäule (4') und damit Einleitung des abgetrennten Plasmas über den Bypass-Leitungsabschnitt (12') der Bypass-Leitung (12) in Apheresesäule (4"),
(G) Schließung der Abfallleitung (13") und Weiterleiten des aus der Apheresesäule (4") austretenden Flüssigkeitsstromes in die venöse Leitung (6),
(H) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4').
(J) Stoppen der Einleitung der Regenerationslösung und Übergang zur Einleitung der Spüllösung über die zumindest eine Regenerationsleitung (14) in die Plasmaleitung (8A) oder direkt in die Apheresesäule (4').

Bei den beiden vorgenannten Verfahren wird zusätzlich zur Regenerationslösung noch eine Spüllösung eingesetzt. Die Spüllösung ist vorzugsweise physiologisch verträglich und dient vorrangig zur Verdrängung des Blutplasmas aus der Plasmaleitung (8A) oder dem Bypass-Leitungsabschnitt (12') der Bypass-Leitung (12) ab dem Punkt P2, aus der Apheresesäule (4') oder Apheresesäule (4") sowie aus der Bypass-Leitungsabschnitt (12") der Bypass-Leitung (12) bis zum Punkt P3 und der Plasmaleitung (8B) bis zum Punkt P4. Die Spüllösung dient weniger oder nicht zur Regeneration der Apheresesäule (4') oder der Apheresesäule (4"). Durch die Spüllösung wird daher der Plasmaverlust minimiert oder sogar vollständig vermieden. Erst wenn das Blutplasma aus dem mit Regenerationslösung zu durchspülenden Abschnitt der Apheresevorrichtung (II) weitestgehend bis vollständig verdrängt ist, wird die Regenerationslösung eingeleitet, um die Apheresesäule (4') oder die Apheresesäule (4") zu regenerieren. Nach erfolgter Regeneration wird dann erst wieder Spüllösung in den mit Regenerationslösung durchspülten Abschnitt der Apheresevorrichtung (II) (also in Flussrichtung von Punkt P2 durch die Apheresesäule (4") bis zum Punkt P8) oder durch die Apheresesäule (4') bis zum Punkt P4) geleitet, bis die Regenerationslösung vollständig durch die Abfallleitungen (13', 13") entsorgt worden ist.

Bei diesem Verfahren handelt es sich bei der Spüllösung vorzugsweise um eine physiologische NaCI-Lösung und bei der Regenerationslösung um eine Citrat-Lösung.

Die bevorzugte Ausführungsform des erfindungsgemäßen Verfahrens dient zur effizienteren Durchführung des Verfahrens ohne Verlust von Blutplasma. Durch die zeitgleiche Umleitung des abgetrennten Plasmas und der parallelen Einleitung der Spüllösung in die Apheresesäule (4") entsteht kein Verlust oder kein nennenswerter Verlust von Blutplasma. Ferner ist an der bevorzugten Ausführungsform von Vorteil, dass ein Durchmischen von Regenerationslösung und Blutplasma vollständig vermieden wird. Dadurch wird sichergestellt, dass keine Regenerationslösung in den Patienten gelangt und andererseits kein Verlust von Blutplasma für den Patienten auftritt.
Dadurch wird sichergestellt, dass keine Regenerationslösung in den Patienten gelangt und andererseits kein Verlust von Blutplasma für den Patienten auftritt.

Dies wird durch die sequenzielle Abfolge der Schritte (B) bis (E) sichergestellt. Eine Verdünnung des Blutplasmas findet wenn überhaupt nur durch Spüllösung statt. Hingegen wird eine Vermischung von Blutplasma mit Regenerationslösung vollständig vermieden.

Das Volumen an Spüllösung gemäß Schritt (B) entspricht vorzugsweise dem 3- bis 4-fachen Volumen der Matrix der Apheresesäule (4"). Minimal entspricht das Volumen an Spüllösung gemäß Schritt (B) dem Volumen dem Bypass-Leitungsabschnitt (12') der Bypass-Leitung (12) ab Punkt P2 bis zur Apheresesäule (4") zuzüglich dem Volumen der Matrix der Apheresesäule (4") und zuzüglich dem Volumen dem Bypass-Leitungsabschnitt (12') der Bypass-Leitung (12) ab der Apheresesäule (4") bis zum Punkt (P3).

Das Volumen an Spüllösung gemäß Schritt (F) entspricht vorzugsweise dem 3- bis 4-fachen Volumen der Matrix der Apheresesäule (4'). Minimal entspricht das Volumen an Spüllösung gemäß Schritt (B) dem Volumen der Plasmaleitung (8A') ab dem Punkt (P2) bis zur Apheresesäule (4') zuzüglich dem Volumen der Matrix der Apheresesäule (4') und zuzüglich dem Volumen der Plasmaleitung (8B) ab der Apheresesäule bis zum Punkt (P4).

Das Volumen an Regenerationslösung gemäß Schritt (D) entspricht vorzugsweise dem 2- bis 100-fachen Volumen der Matrix der Apheresesäule (4").

Das Volumen an Regenerationslösung gemäß Schritt (I) entspricht vorzugsweise dem 2- bis 100-fachen Volumen der Matrix der Apheresesäule (4').

Das Volumen an Spüllösung gemäß Schritt (E) entspricht vorzugsweise dem 2- bis 4-fachen Volumen der Matrix der Apheresesäule (4").
Zumindest entspricht das Volumen an Spüllösung gemäß Schritt (E) dem Volumen des Bypass-Leitungsabschnitt (12') der Bypass-Leitung (12) ab Punkt P2 bis zur Apheresesäule (4") zuzüglich dem Volumen der Matrix der Apheresesäule (4") und zuzüglich dem Volumen des Bypass-Leitungsabschnitt (12') der Bypass-Leitung (12) ab der Apheresesäule (4') bis zum Punkt P3.

Das Volumen an Spüllösung gemäß Schritt (J) entspricht vorzugsweise dem 2- bis 4-fachen Volumen der Matrix der Apheresesäule (4').
Zumindest entspricht das Volumen an Spüllösung gemäß Schritt (E) dem Volumen der Plasmaleitung (8A) ab Punkt P2 bis zur Apheresesäule (4') zuzüglich dem Volumen der Matrix der Apheresesäule (4') und zuzüglich dem Volumen der Plasmaleitung (8B) ab der Apheresesäule (4') bis zum Punkt P4.

### Regenerationslösung

Insbesondere handelt es sich bei der Regenerationslösung um eine Citrat-Lösung, eine TRIS-Glycin-Lösung, eine NaCI-Lösung, eine Vollelektrolyt-Lösung oder eine EDTA-Lösung, bevorzugt um eine Citrat-Lösung, eine TRIS-Glycin-Lösung oder um eine NaCl-Lösung, weiter bevorzugt um eine Citrat-Lösung oder um eine NaCI-Lösung und am meisten bevorzugt um eine Citrat-Lösung.

Die vorliegende Erfindung betrifft daher auch ein erfindungsgemäßes Verfahren zur Regeneration einer Apheresesäule, wobei die Regenerationslösung ausgewählt wird aus der Gruppe umfassend oder bestehend aus: NaCI-Lösung, NaCI-Lösung mit Zusatz von Citrat, Citrat-Lösung alleine, TRIS-Glycin-Lösung und EDTA-Lösung.

Gemäß dieser Ausführungsform kann als Regenerationslösung die bereits im System vorhandene Antikoagulationslösung verwendet werden. Folglich kann die Regeneration ohne zusätzliche Flüssigkeiten betrieben werden.

Der Begriff **"Citrat-Lösung",** wie hierin verwendet, umfasst wässrige Lösungen die zumindest eine Citrat-Verbindung enthalten.

Der Begriff **"Citrat",** wie hierin verwendet, bezieht sich auf das Citrat-Anion, also das Salz der Zitronensäure, oder anders ausgedrückt ein organisches Tricarboxylat der folgenden chemischen Formel:

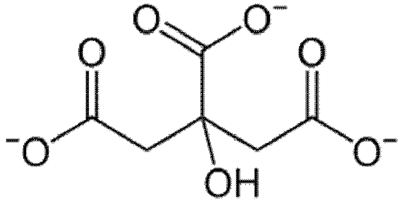

Das Citrat kann in verschiedenen Formen (bzw. Verbindungen) auftreten, also z.B. als Zitronensäure (in ein bis dreifach protonierter Form), als Salz der Zitronensäure in Kombination mit anderen (anders als H⁺) anorganischen Kationen (z.B. als Metallsalz zusammen mit Metallkationen oder als Ammoniumsalz zusammen mit Ammoniumionen), aber auch als partieller Citratester. In diesem Zusammenhang wird hierin auch der Begriff **"Citrat-Verbindung"** verwendet.

Wird das Salz der Zitronensäure, also das Citrat-Anion, mit einem anorganischen Kation komplexiert, spricht man hierin auch von **"Citrat-Salz"** als spezielle Form der Citrat-Verbindung. Somit umfasst der Begriff "Citrat-Verbindung", wie hierin verwendet, sowohl Zitronensäure als auch deren Salze.

Erfindungsgemäß ist bevorzugt, wenn die Citrat-Lösung mindestens eine der Citrat-Verbindungen aus der Gruppe umfassend oder bestehend aus Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat, Trikaliumcitrat, Lithiumdihydrogencitrat, Dilithiumhydrogencitrat, Trilithiumcitrat, Ammoniumdihydrogencitrat, Diammoniumhydrogencitrat, Triammoniumcitrat, Tricalciumdicitrat (Calciumcitrat), Trimagnesiumdicitrat (Magnesiumcitrat) und/oder partielle Citratester enthält.

Wird in dieser Anmeldung der recht allgemeine Begriff "Natriumcitrat" verwendet, umfasst dieser Begriff die verschieden protonierten Formen des Natriumcitrates, also sowohl die unprotonierte (Trinatriumcitrat), als auch die einfach protonierte (Dinatriumhydrogencitrat) oder die zweifach protonierte Form (Natriumdihydrogencitrat). Wird in dieser Anmeldung der recht allgemeine Begriff "Kaliumcitrat" verwendet, umfasst dieser Begriff die verschieden protonierten Formen des Kaliumcitrates, also sowohl die unprotonierte (Trikaliumcitrat), als auch die einfach protonierte (Dikaliumhydrogencitrat) oder die zweifach protonierte Form (Kaliumdihydrogencitrat). Wird in dieser Anmeldung der recht allgemeine Begriff "Lithiumcitrat" verwendet, umfasst dieser Begriff die verschieden protonierten Formen des Lithiumcitrates, also sowohl die unprotonierte (Trilithiumcitrat), als auch die einfach protonierte (Dilithiumhydrogencitrat) oder die zweifach protonierte Form (Lithiumdihydrogencitrat). Wird in dieser Anmeldung der recht allgemeine Begriff "Ammoniumcitrat" verwendet, umfasst dieser Begriff die verschieden protonierten Formen des Ammoniumcitrates, also sowohl die unprotonierte (Triammoniumcitrat), als auch die einfach protonierte (Diammoniumhydrogencitrat) oder die zweifach protonierte Form (Ammoniumdihydrogencitrat).

Eine Citrat-Lösung bestehend aus Zitronensäure, Trinatriumcitrat, D-Glucose und Wasser wird auch als **"Acid-Citrat-Dextrose-Lösung (ACD-Lösung)"** bezeichnet. Bevorzugte Varianten der erfindungsgemäß verwendeten Citrat-Lösung betreffen ACD-Lösungen, welche zwischen 22,9 mM und 38,0 mM Zitronensäure, zwischen 44,9 mM und 74,8 mM Trinatriumcitrat, zwischen 74,2 mM und 123,6 mM D-Glucose und Wasser enthalten. Eine besonders bevorzugte Variante der erfindungsgemäß verwendeten Citrat-Lösung betrifft eine ACD-Lösung, welche 38 mM Zitronensäure, 74,8 mM Trinatriumcitrat, 123,6 mM D-Glucose und Wasser enthält. Diese wird auch als "ACD-A Lösung" bezeichnet.

Eine Citrat-Lösung bestehend aus Zitronensäure, Trinatriumcitrat, Natriumhydrogenphosphat, D-Glucose und Wasser wird auch als **"Citrat-Phosphat-Dextrose-Lösung (CPD)"** bezeichnet. Eine bevorzugte Variante der erfindungsgemäß verwendeten Citrat-Lösung betrifft eine CPD-Lösung, welche 15,6 mM Zitronensäure, 89,4 mM Trinatriumcitrat, 128,7 mM D-Glucose, 16,1 mM Natriumhydrogenphosphat und Wasser enthält. Eine Citrat-Lösung bestehend aus Zitronensäure, Trinatriumcitrat, Natriumhydrogenphosphat, D-Glucose, Adenin und Wasser wird auch als **"Citrat-Phosphat-Dextrose-Lösung mit Adenin (CPDA)"** bezeichnet. Eine bevorzugte Variante der erfindungsgemäß verwendeten Citrat-Lösung betrifft eine CPDA-Lösung, welche 15,6 mM Zitronensäure, 89,4 mM Trinatriumcitrat, zwischen 128,7 mM und 160,9 mM D-Glucose, 16,1 mM Natriumhydrogenphosphat, 2 mM Adenin und Wasser enthält. Eine bevorzugte Variante der erfindungsgemäß verwendeten Citrat-Lösung betrifft eine CPDA-Lösung, welche 15,6 mM Zitronensäure, 89,4 mM Trinatriumcitrat, zwischen 128,7 mM D-Glucose, 16,1 mM Natriumhydrogenphosphat, 2 mM Adenin und Wasser enthält. Eine besonders bevorzugte Variante der erfindungsgemäß verwendeten Citrat-Lösung betrifft eine CPDA-Lösung, welche 15,6 mM Zitronensäure, 89,4 mM Trinatriumcitrat, 160,9 mM D-Glucose, 16,1 mM Natriumhydrogenphosphat, 2 mM Adenin und Wasser enthält.

Der Begriff **"NaCl-Lösung",** wie hierin verwendet, umfasst wässrige Lösungen, die Natriumchlorid (d.h. NaCl, auch als Kochsalz bezeichnet) als Hauptbestandteil enthalten. "Hauptbestandteil" bedeutet hierbei, dass die molare Konzentration an Natriumchlorid in der NaCI-Lösung höher ist als die jeweilige molare Konzentration aller sonstigen Verbindungen innerhalb der NaCI-Lösung, ausgenommen jedoch Wasser. Bevorzugt umfasst die NaCI-Lösung 0,1 bis 5 Gew.% Natriumchlorid, besonders bevorzugt 0,9 Gew.%. Bei der Spüllösung handelt es sich vorzugsweise um eine solche NaCl-Lösung.

Der Begriff "TRIS-Glycin-Lösung", wie hierin verwendet, umfasst wässrige Lösungen die Tris(hydroxmethyl)aminomethan (2-Amino-2-(hydroxymethyl)propan-1,3-diol; TRIS) und Glycin enthalten. Vorzugsweise handelt es sich bei einer "TRIS-Glycin-Lösung" um ein TRIS-Glycin-Puffer. Besonders bevorzugt handelt es sich bei der Tri-Glycin-Lösung um ein TRIS-Glycin-Puffer mit einem pH von 8.3 aus TRIS (25 mM) und Glycin (192 mM). Weiterhin bevorzugt handelt es sich bei der Tri-Glycin-Lösung um ein TRIS-Glycin-Puffer mit einem pH von 8.3 aus TRIS (25 mM), Glycin (192 mM) und SDS (Natriumlaurylsulfat) (0.1% m/V). Vorzugsweise entspricht der pH-Wert dem pH-Wert bei 25°C.

Bei einer bevorzugten konkreten Ausführungsform handelt es sich bei der Spüllösung um eine Kochsalzlösung bzw. eine physiologische Kochsalzlösung oder um eine PBS-Lösung (phosphate buffered saline) oder um eine Kombination von Kochsalzlösung und PBS-Lösung nacheinander oder zeitgleich und bei der Regenerationslösung um eine Citrat-Lösung.

Somit betrifft eine bevorzugte konkrete Ausführungsform ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Kochsalzlösung und Übergang zur Einleitung einer Citrat-Lösung über die zumindest eine Regenerationsleitung (14) in die Plasmaleitung (8A) oder direkt in die Apheresesäule (4),
(E) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4),
(F) Stoppen der Einleitung von Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(G) Schließung der Abfallleitung (13) und Weiterleiten des aus der Apheresesäule (4) austretenden Flüssigkeitsstromes in die venöse Leitung (6)..

Alternativ betrifft die bevorzugte konkrete Ausführungsform ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Stoppen der Einleitung der Kochsalzlösung und Übergang zur Einleitung einer Citrat-Lösung über die zumindest eine Regenerationsleitung (14) in die Plasmaleitung (8A) oder direkt in die Apheresesäule (4),
(D) Beginn der Umleitung des aus der Apheresesäule (4) austretenden Flüssigkeitsstromes von der Plasmaleitung (8B) in die Abfallleitung (13),
(E) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4),
(F) Schließung der Abfallleitung (13) und Weiterleiten des aus der Apheresesäule (4) austretenden Flüssigkeitsstromes in die venöse Leitung (6);
(G) Stoppen der Einleitung von Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bei den beiden vorgenannten Verfahren sind die Schritte (C) und (D) vertauschbar, d.h. können in beliebiger Reihenfolge und auch zeitgleich erfolgen und können auch in einem Schritt zusammengefasst werden.

Bevorzugt werden die erfindungsgemäßen Regenerationsverfahren so durchgeführt, dass zuerst das Plasma mit einer Spüllösung, wie z.B. einer Kochsalzlösung bzw. physiologischen Kochsalzlösung, aus der Apheresesäule (4) verdrängt und zurück in den Patienten geführt wird bis hin zu dem Punkt, dass fast nur noch Kochsalzlösung zurückgeführt wird. Erst dann wird die Kochsalzlösung in die Abfallleitung (13) geleitet und Regenerationslösung, wie z.B. eine Citrat-Lösung, wird in Flussrichtung nach der Bypass-Leitung (12) in die Plasmaleitung (8A) eingeleitet, welche die Kochsalzlösung verdrängt, die Apheresesäule (4) regeneriert, vollständig in die Abfallleitung (13) eingeleitet und verworfen wird. Nach erfolgter Regeneration der Apheresesäule (4) mit mehreren Apheresesäulevolumina an Regenerationslösung wird wieder eine Spüllösung, wie z.B. eine Kochsalzlösung bzw. physiologische Kochsalzlösung, so lange eingeleitet, bis die Regenerationslösung vollständig aus der Apheresevorrichtung (1) verdrängt und verworfen ist. Erst danach erfolgt die Schließung der Abfallleitung (13), die Rückführung der Spüllösung in den Patienten, die Schließung der Bypass-Leitung (12) und die erneute Einleitung von Plasma durch die Plasmaleitung (8A) in die Apheresesäule (4) zeitgleich oder unmittelbar nacheinander, wobei die Reihenfolge der Schritte vertauschbar ist.

Eine weitere bevorzugte konkrete Ausführungsform betrifft ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Kochsalzlösung und Übergang zur Einleitung einer Citrat-Lösung über die zumindest eine Regenerationsleitung (14) in die Plasmaleitung (8A) oder direkt in die Apheresesäule (4),
(E1) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4),
(E2) Stoppen der Einleitung der Kochsalzlösung und Übergang zur Einleitung einer PBS-Lösung über die zumindest eine Regenerationsleitung (14) in die Plasmaleitung (8A) oder direkt in die Apheresesäule (4),
(E3) Stoppen der Einleitung der PBS-Lösung und Übergang zur Einleitung einer Kochsalzlösung über die zumindest eine Regenerationsleitung (14) in die Plasmaleitung (8A) oder direkt in die Apheresesäule (4),
(F) Stoppen der Einleitung von Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(G) Schließung der Abfallleitung (13) und Weiterleiten des aus der Apheresesäule (4) austretenden Flüssigkeitsstromes in die venöse Leitung (6)..

Eine alternative bevorzugte konkrete Ausführungsform betrifft ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Stoppen der Einleitung der Kochsalzlösung und Übergang zur Einleitung einer Citrat-Lösung über die zumindest eine Regenerationsleitung (14) in die Plasmaleitung (8A) oder direkt in die Apheresesäule (4),
(D) Beginn der Umleitung des aus der Apheresesäule (4) austretenden Flüssigkeitsstromes von der Plasmaleitung (8B) in die Abfallleitung (13),
(E1) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4),
(E2) Stoppen der Einleitung der Kochsalzlösung und Übergang zur Einleitung einer PBS-Lösung über die zumindest eine Regenerationsleitung (14) in die Plasmaleitung (8A) oder direkt in die Apheresesäule (4),
(E3) Stoppen der Einleitung der PBS-Lösung und Übergang zur Einleitung einer Kochsalzlösung über die zumindest eine Regenerationsleitung (14) in die Plasmaleitung (8A) oder direkt in die Apheresesäule (4),
(F) Schließung der Abfallleitung (13) und Weiterleiten des aus der Apheresesäule (4) austretenden Flüssigkeitsstromes in die venöse Leitung (6);
(G) Stoppen der Einleitung von Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bei den beiden vorgenannten Verfahren sind die Schritte (C) und (D) vertauschbar, d.h. können in beliebiger Reihenfolge und auch zeitgleich erfolgen und können auch in einem Schritt zusammengefasst werden.

Somit betrifft die vorliegende Erfindung ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in venöse Leitung (6) und damit Stoppen der Einleitung des abgetrennten Plasmas über den Bypass-Leitungsabschnitt (12') der Bypass-Leitung (12) in die Apheresesäule (4"),
(B) Beginn der Einleitung einer **Kochsalzlösung** über die zumindest eine Regenerationslösung (14) in den Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12") der Bypass-Leitung (12) in die Abfallleitung (13"),
(D) Stoppen der Einleitung der **Kochsalzlösung** und Übergang zur Einleitung der **Citrat-Lösung** über die zumindest eine Regenerationsleitung (14) in den Bypass-Leitungsabschnitt (12') der Bypass-Leitung (12) oder direkt in die Apheresesäule (4"),
(E) Stoppen der Einleitung der **Citrat-Lösung** und Übergang zur Einleitung der **Kochsalzlösung** über die zumindest eine Regenerationsleitung (14) in den Bypass-Leitungsabschnitt (12') der Bypass-Leitung (12) oder direkt in Apheresesäule (4"),
(F) Beginn der Einleitung der **Kochsalzlösung** in die Plasmaleitung (8A) über die Apheresesäule (4') und damit Einleitung des abgetrennten Plasmas über den Bypass-Leitungsabschnitt (12') der Bypass-Leitung (12) in die Apheresesäule (4"),
(G) Schließung der Abfallleitung (13") und Weiterleiten des aus der Apheresesäule (4") austretenden Flüssigkeitsstromes in die venöse Leitung (6),
(H) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer **Citrat-Lösung** über die zumindest eine Regenerationsleitung (13) in die Plasmaleitung (8A) oder direkt in die Apheresesäule (4'),
(J) Stoppen der Einleitung der **Citrat-Lösung** und Übergang zur Einleitung der **Kochsalzlösung** über die zumindest eine Regenerationsleitung (14) in die Plasmaleitung (8A) oder direkt in die Apheresesäule (4').

Bevorzugt werden die erfindungsgemäßen Regenerationsverfahren so durchgeführt, dass zuerst das Plasma mit einer Spüllösung, wie z.B. einer Kochsalzlösung bzw. physiologischen Kochsalzlösung, aus der Apheresesäule (4") verdrängt und zurück in den Patienten geführt wird bis hin zu dem Punkt, dass fast nur noch Kochsalzlösung zurückgeführt wird. Erst dann wird die Kochsalzlösung in die Abfallleitung (13") geleitet und Regenerationslösung, wie z.B. eine Citrat-Lösung, wird in Flussrichtung an dem Punkt P2 in den Bypass-Leitungsabschnitt (12') der Bypass-Leitung (12) eingeleitet, welche die Kochsalzlösung verdrängt, die Apheresesäule (4") regeneriert, vollständig in die Abfallleitung (13") eingeleitet und verworfen wird. Nach erfolgter Regeneration der Apheresesäule (4") mit mehreren Apheresesäulevolumina an Regenerationslösung wird wieder eine Spüllösung, wie z.B. eine Kochsalzlösung bzw. physiologische Kochsalzlösung, so lange eingeleitet, bis die Regenerationslösung vollständig aus der Apheresevorrichtung (II) verdrängt und verworfen ist. Erst danach erfolgt die Schließung der Plasmaleitung (8A), die Rückführung der Spüllösung in den Patienten, und die erneute Einleitung von Plasma durch den Bypass-Leitungsabschnitt (12') der Bypass-Leitung (12) in die Apheresesäule (4") zeitgleich oder direkt nacheinander.

Eine weitere bevorzugte konkrete Ausführungsform betrifft ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in venöse Leitung (6) und damit Stoppen der Einleitung des abgetrennten Plasmas über den Bypass-Leitungsabschnitt (12') der Bypass-Leitung (12) in die Apheresesäule (4"),
(B) Beginn der Einleitung einer **Kochsalzlösung** über die zumindest eine Regenerationsleitung (14) in die Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12') der Bypass-Leitung (12) in die Abfallleitung (13"),
(D) Stoppen der Einleitung der **Kochsalzlösung** und Übergang zur Einleitung der **Citrat-Lösung** über die zumindest eine Regenerationsleitung (14) in den Bypass-Leitungsabschnitt (12') der Bypass-Leitung (12) oder direkt in die Apheresesäule (4"),
(E1) Stoppen der Einleitung der **Citrat-Lösung** und Übergang zur Einleitung der **Kochsalzlösung** über die zumindest eine Regenerationsleitung (14) in den Bypass-Leitungsabschnitt (12') der Bypass-Leitung (12) oder direkt in Apheresesäule (4"),
(E2) Stoppen der Einleitung der **Kochsalzlösung** und Übergang zur Einleitung einer **PBS-Lösung** über die zumindest eine Regenerationsleitung (14) in den Bypass-Leitungsabschnitt (12') der Bypass-Leitung (12) oder direkt in die Apheresesäule (4"),
(E3) Stoppen der Einleitung der PBS-Lösung und Übergang zur Einleitung einer Kochsalzlösung über die zumindest eine Regenerationsleitung (14) in den Bypass-Leitungsabschnitt (12') der Bypass-Leitung (12) oder direkt in die Apheresesäule (4"),
(F) Beginn der Einleitung der **Kochsalzlösung** in die Plasmaleitung (8A) über die Apheresesäule (4') und damit Einleitung des abgetrennten Plasmas über den Bypass-Leitungsabschnitt (12') der Bypass-Leitung (12) in die Apheresesäule (4"),
(G) Schließung der Abfallleitung (13") und Weiterleiten des aus der Apheresesäule (4") austretenden Flüssigkeitsstromes in die venöse Leitung (6),
(H) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer **Citrat-Lösung** über die zumindest eine Regenerationsleitung (14) in die Plasmaleitung (8A) oder direkt in die Apheresesäule (4'),
(J) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4').

### Beispiele

### Anwendungsbeispiel:

Der Begriff "Matrixvolumen" (auch als MV abgekürzt), wie hierin verwendet, bezieht sich auf das Volumen der Matrix, welche im Adsorber enthalten ist.

Der Begriff "Adsorbervolumen" (auch als AV abgekürzt), wie hierin verwendet, bezieht sich auf das Volumen des Adsorbergehäuses.

### Beispiel 1: Apherese mit einer Bypass-Leitung und einer Apheresesäule

### Vorbereitung:

In die Apheresevorrichtung (1) zur extrakorporalen Entfernung von CRP aus Blut eines Patienten gemäß Fig. 3, mit einer Plasmazentrifuge als Zellseparator (7), wird ein passendes Schlauchsystem eingelegt. An die Anschlussleitung wird ein 5 L Beutel mit 0,9% NaCI-Lösung sowie ein 500 ml Beutel mit ACD-A Lösung angeschlossen. An die Abfallleitung (13) werden zwei 3 L Abfallbeutel angeschlossen (z. B. über einen 3 Wege-Hahn).

Die arterielle (5) und die venöse (6) Leitung werden mit einem Adapter miteinander verbunden. Ebenso werden die Plasmaleitungen (8A und 8B) vor und nach dem Adsorber mit einem Adapter (ohne Adsorber dazwischen) verbunden, so dass ein geschlossenes System entsteht.

Durch Vorspülen mit 1 L 0,9 % NaCI Lösung (200 ml/Min) wird das gesamte System mit NaCl Lösung gefüllt; die vorhandene Luft wird in den ersten Abfallbeutel verdrängt. Anschließend wird anstelle des Adapters ein aufgeschüttelter CRP-Adsorber (MV 20 ml, AV 30 ml) in die Plasmaleitung (8A und 8B) eingesetzt. Der Adsorber wird mit 1 L NaCl Lösung (100 ml/min) vorgespült. Das NaCl wird ebenfalls in den ersten Abfallbeutel geleitet.

Als letzter Schritt der Vorbereitung wird die Plasmazentrifuge mit 0,9% NaCl Lösung und 1:15 verdünnter ACD-A Lösung vorgefüllt. Das benötigte Volumen setzt sich zusammen aus dem Volumen des Schlauchsystems in der Plasmazentrifuge (7), der Anschlussleitung (11) bis zur Plasmazentrifuge und der Plasmaleitung zwischen der Plasmazentrifuge und P2. Das verdrängte NaCl wird über P4/P6 in den ersten Abfallbeutel geleitet.

### Apherese:

1. Nach Ende der Vorbereitung wird auf den zweiten Abfallbeutel umgeschaltet. Der Patient wird an die arterielle (5) und die venöse (6) Leitung angeschlossen. Bei Beginn der Apherese wird das Blut in die Zentrifuge geleitet (60 - 80 ml/min). Während der gesamten Behandlung wird ACD-A im Verhältnis 1:15 (1 ml ACD-A auf 15 ml Blut) über die Anschlussleitung (11) dem Blut zugemischt.
   Das dadurch verdrängte NaCl wird über P2, die Bypassleitung (12) und P4/P6 in den zweiten Abfallbeutel geleitet. Beginnt die Plasmaseparation, so wird nach einem Volumen, das der Schlauchleitung von der Plasmazentrifuge bis zum Punkt P4/6 entspricht, so umgeschaltet, dass das Plasma in die venöse Leitung (6), und damit zum Patienten zurück, fließt. Nachdem für 3 Minuten ein konstanter Plasmafluss von ca. 30 ml/min erzielt wurde, kann der erste Zyklus beginnen.
2. Die Bypassleitung (12) wird geschlossen und das Plasma wird über den Adsorber geleitet (Beladung). Dabei wird das in der Plasmaleitung (8A und 8B) und dem Adsorber befindliche NaCl bis zu einem Volumen, bestehend aus dem Volumen der Plasmaleitung (8A und 8B) plus dem AV, über P4/P6 in den zweiten Abfallbeutel geleitet. Der Adsorber wird anschließend mit 50 - 100 MV (1000 bis 2000 ml) Plasma beladen. Anschließend beginnt die Regeneration.
3. Dazu wird das Plasma wieder über die Bypassleitung (12) zum Patienten zurückgeführt. Der Adsorber wird nun über die Regenerationsleitung (14) und die Plasmaleitung (8A und 8B) mit 0,9% NaCl gespült (30 ml/min). Das dafür benötigte Volumen berechnet sich aus dem AV und dem Volumen der Plasmaleitung (8A und 8B). Das in der Plasmaleitung (8A und 8B) und dem Adsorber befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.
   Im nächsten Schritt wird mit 3 MV (60 ml) 0,9% NaCl und danach mit 1:15 ACD-A Lösung regeneriert (100 ml/min). Danach wird mit 0,9% NaCl nachgespült (100 ml/min). Das dafür benötigte Volumen berechnet sich aus dem AV, dem Volumen der Regenerationsleitung (14) und der Plasmaleitung (8A und 8B).
   Anschließend kann wieder Schritt 2 (Beladung), danach Schritt 3 erfolgen. Bei Bedarf muß der Beutel mit ACD-A Lösung ersetzt werden.
4. Nachdem die letzte Beladung erfolgte, wird eine abschließende Regeneration durchgeführt. Gleichzeitig wird die arterielle Leitung (5) geschlossen. Über die Anschlussleitung (11) wird mit 0,9% NaCl (30 ml/min) das Blut aus der Plasmazentrifuge (7) über die Zellleitung (9) sowie das restliche Plasma aus der Plasmaleitung bis P2 und der Bypassleitung (12) verdrängt und dem Patienten zurückgeführt. Das dazu benötigte Volumen setzt sich zusammen aus dem Volumen der Plasmazentrifuge (7), dem Volumen der Plasmaleitung bis P2, Bypassleitung (12), der Zellleitung (9) und der arteriellen Leitung (6). Der Patient kann anschließend von der Apheresevorrichtung getrennt werden.
5. Falls gewünscht, kann nun der NaCI-Beutel durch einen Beutel mit Konservierungslösung (z. B. PBS mit Na-Azid) ersetzt werden. Über die Regenerationsleitung wird der Adsorber mit 10 MV Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Beispiel 2: Abwechselnde Verwendung der parallel geschalteten Apheresesäule

### Vorbereitung:

In die Apheresevorrichtung (II) zur extrakorporalen Entfernung von CRP aus Blut eines Patienten gemäß Fig. 13, mit einer Plasmazentrifuge als Zellseparator (7), wird ein passendes Schlauchsystem eingelegt. An die Anschlussleitung wird ein 5 L Beutel mit 0,9% NaCI-Lösung sowie ein 500 ml Beutel mit ACD-A Lösung angeschlossen. An die Abfallleitung (13) werden zwei 3 L Abfallbeutel angeschlossen (z. B. über einen 3 Wege-Hahn).

Die arterielle (5) und die venöse (6) Leitung werden mit einem Adapter miteinander verbunden. Ebenso werden vor die Plasmaleitungen (8A und 8B) und nach dem Adsorber mit einem Adapter (ohne Adsorber dazwischen) verbunden sowie die Bypass-Leitungsabschnitte (12' und 12") der Bypass-Leitung (12) vor und nach dem Adsorber mit einem Adapter (ohne Adsorber dazwischen) verbunden, so dass ein geschlossenes System entsteht.

Durch Vorspülen mit 1 L 0,9 % NaCl Lösung (200 ml/Min) wird das gesamte System mit NaCl Lösung gefüllt; die vorhandene Luft wird in den ersten Abfallbeutel verdrängt. Anschließend wird anstelle des Adapters ein aufgeschüttelter CRP-Adsorber (MV 20 ml, AV 30 ml) in den die Bypass-Leitungsabschnitten (12' und 12") und in die Plasmaleitung (8A und 8B) eingesetzt. Der Adsorber wird mit 1 L NaCl Lösung (100 ml/min) vorgespült. Das NaCl wird ebenfalls in den ersten Abfallbeutel geleitet.

Als letzter Schritt der Vorbereitung wird die Plasmazentrifuge mit 0,9% NaCl Lösung und 1:15 verdünnter ACD-A Lösung vorgefüllt. Das benötigte Volumen setzt sich zusammen aus dem Volumen des Schlauchsystems in der Plasmazentrifuge (7), der Anschlussleitung (11) bis zur Plasmazentrifuge und der Plasmaleitung zwischen der Plasmazentrifuge und P2. Das verdrängte Natriumchlorid wird über P8/P4/P6 in den ersten Abfallbeutel geleitet.

### Apherese:

1. Nach Ende der Vorbereitung wird auf den zweiten Abfallbeutel umgeschaltet. Der Patient wird an die arterielle (5) und die venöse (6) Leitung angeschlossen. Bei Beginn der Apherese wird das Blut in die Zentrifuge geleitet (60 - 80 ml/min). Während der gesamten Behandlung wird ACD-A im Verhältnis 1:15 (1 ml ACD-A auf 15 ml Blut) über die Anschlussleitung (11) dem Blut zugemischt.
   Das dadurch verdrängte NaCl wird über P2, den Bypass-Leitungsabschnitt 12' und P8/P4/P6 in den zweiten Abfallbeutel geleitet. Beginnt die Plasmaseparation, so wird nach einem Volumen, das der Schlauchleitung von der Plasmazentrifuge bis zum Punkt P8/P4/P6 entspricht, so umgeschaltet, dass das Plasma in die venöse Leitung (6), und damit zum Patienten zurück fließt. Nachdem für 3 Minuten ein konstanter Plasmafluss von ca. 30 ml/min erzielt wurde, kann der erste Zyklus beginnen.
2. Die Plasmaleitung (8A) in dem Bereich zwischen dem Knotenpunkt (P2) und dem Adsorber (4') wird geschlossen und das Plasma wird über den Adsorber (4") geleitet (Beladung). Dabei wird das in den Bypass-Leitungsabschnitten (12' und 12") und dem Adsorber (4") befindliche NaCl bis zu einem Volumen, bestehend aus dem Volumen der die Bypass-Leitungsabschnitte (12' und 12") plus dem AV, über P3/P4/P6 in den zweiten Abfallbeutel geleitet. Der Adsorber (4") wird anschließend mit 50 - 100 MV (1000 bis 2000 ml) Plasma beladen. Anschließend wird das Blutplasma mit der Natriumchlorid-Lösung aus dem Adsorber (4") verdrängt.
3. Es wird auf den zweiten Adsorber umgeschaltet und der Bypass-Leitungsabschnitt (12') in den Bereich zwischen dem Knotenpunkt (P2) und dem Adsorber (4") geschlossen. Das Plasma wird über den Adsorber (4') geleitet (Beladung). Dabei wird die in den Bypass-Leitungsabschnitten (12' und 12") und dem Adsorber (4') befindliche Natriumchlorid-Lösung bis zu einem Volumen, bestehend aus dem Volumen der Plasmaleitung (8A und 8B) plus dem AV, über P8/P4/P6 in den zweiten Abfallbeutel geleitet. Der Adsorber (4') wird anschließend mit 50 - 100 MV (1000 bis 2000 ml) Plasma beladen. Anschließend wird das Blutplasma mit der Natriumchlorid-Lösung aus dem Adsorber (4') verdrängt und dem Patienten zugeführt.
   Anschließend kann wieder Schritt 2 (Beladung), danach Schritt 3 erfolgen. Bei Bedarf muss der Beutel mit ACD-A Lösung ersetzt werden.
4. Nachdem die letzte Beladung erfolgte, wird eine abschließende Regeneration durchgeführt. Gleichzeitig wird die arterielle Leitung (5) geschlossen. Über die Anschlussleitung (11) wird mit 0,9% NaCl (30 ml/min) das Blut aus der Plasmazentrifuge (7) über die Zellleitung (9) verdrängt und dem Patienten zurückgeführt. Das dazu benötigte Volumen setzt sich zusammen aus dem Volumen der Plasmazentrifuge (7) und dem Volumen der Zellleitung (9) und der arteriellen Leitung (6). Der Patient kann anschließend von der Apheresevorrichtung getrennt werden.
5. Falls gewünscht, kann nun der Natriumchlorid-Lösungs-Beutel durch einen Beutel mit Konservierungslösung (z. B. PBS mit Na-Azid) ersetzt werden. Über die Regenerationsleitung wird der Adsorber mit 10 MV Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Beispiel 3: Abwechselnde Verwendung der parallel geschalteten Apheresesäulen (4', 4") und Regeneration im laufenden Betrieb

### Vorbereitung:

In die Apheresevorrichtung (II) zur extrakorporalen Entfernung von CRP aus Blut eines Patienten gemäß Fig. 5, mit einer Plasmazentrifuge als Zellseparator (7), wird ein passendes Schlauchsystem eingelegt. An die Anschlussleitung wird ein 5 L Beutel mit 0,9% NaCI-Lösung sowie ein 500 ml Beutel mit ACD-A Lösung angeschlossen. An die Abfallleitung (13) werden zwei 3 L Abfallbeutel angeschlossen (z. B. über einen 3 Wege-Hahn).

Die arterielle (5) und die venöse (6) Leitung werden mit einem Adapter miteinander verbunden. Ebenso werden die Bypass-Leitungsabschnitte (12' und 12") vor und nach dem Adsorber mit einem Adapter (ohne Adsorber dazwischen) verbunden sowie die Plasmaleitungen (8A und 8B) vor und nach dem Adsorber mit einem Adapter (ohne Adsorber dazwischen) verbunden, so dass ein geschlossenes System entsteht.

Durch Vorspülen mit 1 L 0,9 % NaCl Lösung (200 ml/Min) wird das gesamte System mit NaCl Lösung gefüllt; die vorhandene Luft wird in den ersten Abfallbeutel verdrängt. Anschließend wird anstelle des Adapters ein aufgeschüttelter CRP-Adsorber (MV 20 ml, AV 30 ml) in den Bypass-Leitungsabschnitten (12' und 12")und in die Plasmaleitung (8A und 8B) eingesetzt. Der Adsorber wird mit 1 L NaCl Lösung (100 ml/min) vorgespült. Das NaCl wird ebenfalls in den ersten Abfallbeutel geleitet.

Als letzter Schritt der Vorbereitung wird die Plasmazentrifuge mit 0,9% NaCl Lösung und 1:15 verdünnter ACD-A Lösung vorgefüllt. Das benötigte Volumen setzt sich zusammen aus dem Volumen des Schlauchsystems in der Plasmazentrifuge (7), der Anschlussleitung (11) bis zur Plasmazentrifuge und der Plasmaleitung zwischen der Plasmazentrifuge und P2. Das verdrängte Natriumchlorid wird über P8/P4/P6 in den ersten Abfallbeutel geleitet.

### Apherese:

1. Nach Ende der Vorbereitung wird auf den zweiten Abfallbeutel umgeschaltet. Der Patient wird an die arterielle (5) und die venöse (6) Leitung angeschlossen. Bei Beginn der Apherese wird das Blut in die Zentrifuge geleitet (60 - 80 ml/min). Während der gesamten Behandlung wird ACD-A im Verhältnis 1:15 (1 ml ACD-A auf 15 ml Blut) über die Anschlussleitung (11) dem Blut zugemischt.
   Das dadurch verdrängte NaCl wird über P2, der Bypass-Leitungsabschnitten (12') und P8/P4/P6 in den zweiten Abfallbeutel geleitet. Beginnt die Plasmaseparation, so wird nach einem Volumen, das der Schlauchleitung von der Plasmazentrifuge bis zum Punkt P8/P4/6 entspricht, so umgeschaltet, dass das Plasma in die venöse Leitung (6), und damit zum Patienten zurück, fließt. Nachdem für 3 Minuten ein konstanter Plasmafluß von ca. 30 ml/min erzielt wurde, kann der erste Zyklus beginnen.
2. Die Plasmaleitung (8A) wird geschlossen und das Plasma wird über den Adsorber (4") geleitet (Beladung). Dabei wird das in den Bypass-Leitungsabschnitten (12' und 12") und dem Adsorber (4") befindliche NaCl bis zu einem Volumen, bestehend aus dem Volumen der Bypass-Leitungsabschnitten (12' und 12") plus dem AV, über P8/P4/P6 in den zweiten Abfallbeutel geleitet. Der Adsorber wird anschließend mit 50 - 100 MV (1000 bis 2000 ml) Plasma beladen. Anschließend wird das Blutplasma mit der Natriumchlorid-Lösung aus dem Adsorber (4") verdrängt.
3. Es wird auf den zweiten Adsorber umgeschaltet und der Bypass-Leitungsabschnitt (12') in den Bereich zwischen dem Knotenpunkt (P2) und dem Adsorber (4") geschlossen. Das Plasma wird über den Adsorber (4') geleitet (Beladung). Dabei wird die in den Bypass-Leitungsabschnitten (12' und 12") und dem Adsorber (4") befindliche Natriumchlorid-Lösung bis zu einem Volumen, bestehend aus dem Volumen der Plasmaleitung (8A und 8B) plus dem AV, über P8/P4/P6 in den zweiten Abfallbeutel geleitet. Der Adsorber (4') wird anschließend mit 50 - 100 MV (1000 bis 2000 ml) Plasma beladen. Anschließend wird das Blutplasma mit der Natriumchlorid-Lösung aus dem Adsorber (4') verdrängt und dem Patienten zugeführt.
   Zeitgleich wird der Adsorber (4") nun über die Regenerationsleitung (14) und die Bypass-Leitungsabschnitte (12' und 12") mit 0,9% NaCl gespült (30 ml/min). Das dafür benötigte Volumen berechnet sich aus dem AV und dem Volumen der Bypass-Leitungsabschnitte (12' und 12"). Das in den Bypass-Leitungsabschnitten (12' und 12") und dem Adsorber (4") befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.
   Im nächsten Schritt wird mit 3 MV (60 ml) 0,9% NaCl und danach mit 1:15 ACD-A Lösung regeneriert (100 ml/min). Danach wird mit 0,9% NaCl nachgespült (100 ml/min). Das dafür benötigte Volumen berechnet sich aus dem AV, dem Volumen der Regenerationsleitung (14) und der Plasmaleitung (8A und 8B).
   Anschließend kann wieder Schritt 2 (Beladung), danach Schritt 3 erfolgen. Bei Bedarf muss der Beutel mit ACD-A Lösung ersetzt werden.
4. Nachdem die letzte Beladung erfolgte, wird eine abschließende Regeneration durchgeführt. Gleichzeitig wird die arterielle Leitung (5) geschlossen. Über die Anschlussleitung (11) wird mit 0,9% NaCl (30 ml/min) das Blut aus der Plasmazentrifuge (7) über die Zellleitung (9) verdrängt und dem Patienten zurückgeführt. Das dazu benötigte Volumen setzt sich zusammen aus dem Volumen der Plasmazentrifuge (7) und dem Volumen der Zellleitung (9) und der arteriellen Leitung (6). Der Patient kann anschließend von der Apheresevorrichtung getrennt werden.
5. Falls gewünscht, kann nun der Natriumchlorid-Lösungs-Beutel durch einen Beutel mit Konservierungslösung (z. B. PBS mit Na-Azid) ersetzt werden. Über die Regenerationsleitung wird der Adsorber mit 10 MV Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Beschreibung der Figuren

- **Fig. 1:**: **Schema-Zeichnung** einer Ausführungsform der erfindungsgemäßen Apheresevorrichtung (1) zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P4) von der Plasmaleitung (8B) ab. Zusätzlich mündet die Regenerationsleitung (14) zum Anschluss eines Flüssigkeitsbehälters (F2) in einem Bereich zwischen dem Knotenpunkt (P2) und der Apheresesäule (4) in die Plasmaleitung (8A). Alternativ kann die Regenerationsleitung (14) auch direkt in die Apheresesäule (4) führen (nicht dargestellt). Zur verbesserten Übersichtlichkeit ist die ebenfalls zur erfindungsgemäßen Apheresevorrichtung gehörende zentrale Recheneinheit (10) nicht dargestellt.
- **Fig. 2**:: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11), die in die arterielle Leitung (5) mündet oder alternativ direkt in den Zellseparator (7) führt (gestrichelte Linie). Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P6) von der Plasmaleitung (8B) ab. Zusätzlich mündet die Regenerationsleitung (14) an dem Knotenpunkt (P2) in die Plasmaleitung (8A). Zur verbesserten Übersichtlichkeit ist die ebenfalls zur erfindungsgemäßen Apheresevorrichtung gehörende zentrale Recheneinheit (10) nicht dargestellt.
- **Fig. 3**:: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F), die in die arterielle Leitung (5) mündet oder alternativ direkt in den Zellseparator (7) führt (gestrichelte Linie). Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P6) von der Plasmaleitung (8B) ab. Zusätzlich mündet die Regenerationsleitung (14), welche an dem Punkt (P5) von der Anschlussleitung (11) abzweigt, an dem Knotenpunkt (P2) in die Plasmaleitung (8A). Zur verbesserten Übersichtlichkeit ist die ebenfalls zur erfindungsgemäßen Apheresevorrichtung gehörende zentrale Recheneinheit (10) nicht dargestellt.
- **Fig. 4:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11), die in die arterielle Leitung (5) mündet oder alternativ direkt in den Zellseparator (7) führt (gestrichelte Linie). Die Bypass-Leitung (12) zweigt an dem Knotenpunkt (P2) von der Plasmaleitung (8A) ab und mündet an dem Knotenpunkt (P3) in die Zellleitung (9). Die Abfallleitung (13) zweigt an dem Knotenpunkt (P1) von der Plasmaleitung (8B) ab. Zusätzlich mündet die Regenerationsleitung (14), welche an dem Punkt (P5) von der Anschlussleitung (11) abzweigt, an dem Knotenpunkt (P2) in die Plasmaleitung (8A). Zur verbesserten Übersichtlichkeit ist die ebenfalls zur erfindungsgemäßen Apheresevorrichtung gehörende zentrale Recheneinheit (10) nicht dargestellt.
- **Fig. 5**:: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11'), die in die arterielle Leitung (5) mündet aber auch direkt in den Zellseparator (7) hätte münden können, sowie eine Anschlussleitung (11"), die in den Zellseparator (7) mündet aber auch in die arterielle Leitung (5) hätte münden können. Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P6) von der Plasmaleitung (8B) ab. Zusätzlich mündet die Regenerationsleitung (14), welche an dem Punkt (P5') in Verbindung mit der Anschlussleitung (11') steht und an dem Punkt (P5") in Verbindung mit der Anschlussleitung (11") steht, an dem Knotenpunkt (P2) in die Plasmaleitung (8A). Zur verbesserten Übersichtlichkeit ist die ebenfalls zur erfindungsgemäßen Apheresevorrichtung gehörende zentrale Recheneinheit (10) nicht dargestellt.
- **Fig. 6:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11'), die in die arterielle Leitung (5) mündet aber auch direkt in den Zellseparator (7) hätte münden können, sowie eine Anschlussleitung (11"), die in den Zellseparator (7) mündet aber auch in die arterielle Leitung (5) hätte münden können. Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P6) von der Plasmaleitung (8B) ab. Zusätzlich münden eine erste Regenerationsleitung (14'), welche an dem Punkt (P5') von der Anschlussleitung (11') abzweigt, und eine zweite Regenerationsleitung (14"), welche an dem Punkt (P5") von der Anschlussleitung (11") abzweigt, beide an dem Knotenpunkt (P2) in die Plasmaleitung (8A). Zur verbesserten Übersichtlichkeit ist die ebenfalls zur erfindungsgemäßen Apheresevorrichtung gehörende zentrale Recheneinheit (10) nicht dargestellt.
- **Fig. 7**:: **Schema-Zeichnung** einer Ausführungsform der erfindungsgemäßen Apheresevorrichtung zur extrakorporalen Entfernung von CRP aus Blut wie in Fig. 3 beschrieben, mit dem Unterschied, dass von der Regenerationsleitung (14) ein zusätzlicher Anschluss für einen Flüssigkeitsbehälter vorhanden ist. Die Anschlussleitung (11) mündet in die arterielle Leitung (5) und könnte aber auch direkt in den Zellseparator (7) führen, was durch die gestrichelte Linie angedeutet ist. Die Regenerationsleitung (14) weist diesmal einen zusätzlichen Anschluss für einen Flüssigkeitsbehälter auf, wobei dieser Anschluss in Flussrichtung hinter dem Zellseparator (7) liegt, so dass Flüssigkeit aus diesem zusätzlichen Flüssigkeitsbehälter nicht in den Zellseparator (7) geleitet und nicht vor dem Zellseparator (7) in die arterielle Leitung (5) geführt werden kann, sondern nur in die Plasmaleitung (8A) in Flussrichtung hinter dem Zellseparator (7) oder direkt in die Apheresesäule (4).

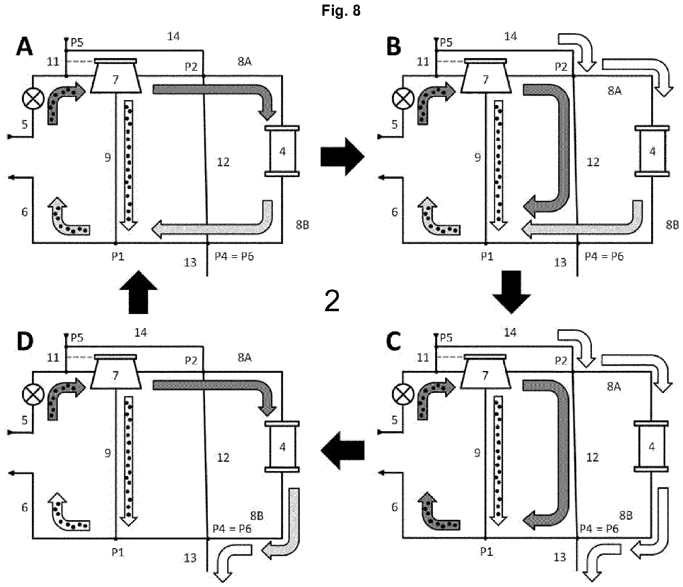

- **Fig. 8:**: **Schematische Darstellung** eines erfindungsgemäßen Regenerationsverfahrens.
(**A**) Im Normalbetrieb wird das unbehandelte Blut (dunkelgrau & punktierter Pfeil) vom Patienten im Zellseparator (7) in das unbehandelte Plasma (dunkelgrauer Pfeil) und die zellulären Bestandteile (weißer & gepunkteter Pfeil) aufgetrennt. Das unbehandelte Plasma wird über die Plasmaleitung (8A) in die Apheresesäule (4) geleitet und CRP wird dort abgereichert. Das derart behandelte Plasma (hellgrauer Pfeil) wird über die Plasmaleitung (8B) zu Punkt (P1) geführt, wo es mit den zellulären Bestandteilen zusammengeführt wird. Das behandelte Blut (hellgrauer & gepunkteter Pfeil) wird über die venöse Leitung zurück zum Patienten geführt.
(**B**) Durch Ventilumstellung am Punkt (P2) wird das unbehandelte Plasma in die Bypass-Leitung (12) umgeleitet und Regenerationslösung (weißer Pfeil) in die Apheresesäule eingeleitet. Die hierdurch verdrängte Menge an Plasma wird zu einem Großteil dem Patienten zugeführt.
(**C**) Anschließend wird durch Ventilumstellung am Punkt (P6) die Regenerationslösung nach Durchfließen der Apheresesäule (4) in die Abfallleitung (13) umgeleitet und somit verworfen.
(**D**) Anschließend wird durch Ventilumstellung am Punkt (P2) die Einleitung von Regenerationslösung gestoppt und das unbehandelte Plasma wieder in die Apheresesäule (4) eingeleitet. Die hierdurch verdrängte Menge an Regenerationslösung wird zu einem Großteil über die Abfallleitung (13) verworfen. Durch anschließende Ventilumstellung an Punkt (P6) wird wieder in den Normalbetrieb (A) umgeschaltet.

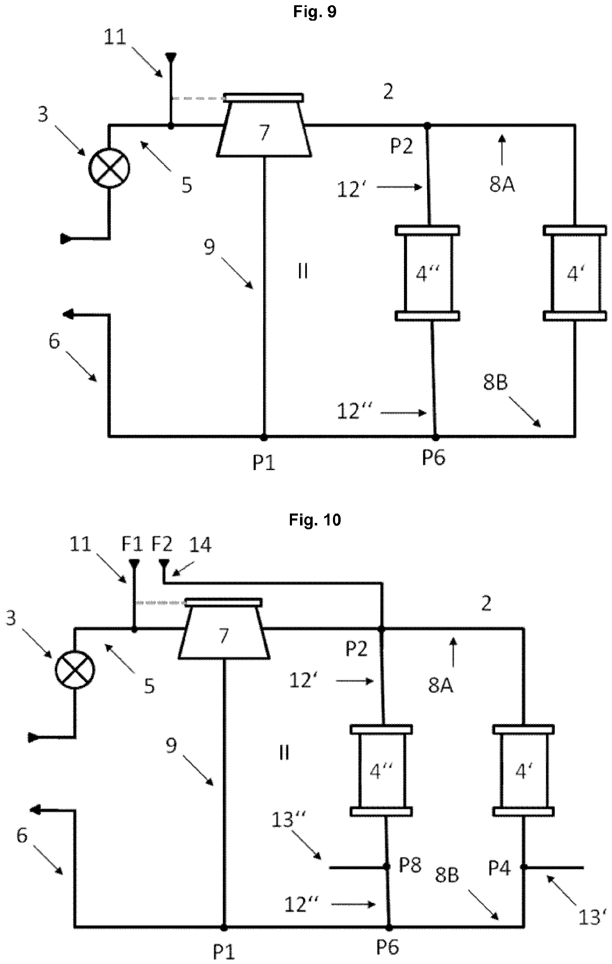

- **Fig. 9:**: **Schema-Zeichnung** einer Ausführungsform der erfindungsgemäßen Apheresevorrichtung (II) zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Die Plasmaleitung (8A) und der Bypass-Leitungsabschnitt (12') der Bypass-Leitung (12) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Zur verbesserten Übersichtlichkeit ist die ebenfalls zur erfindungsgemäßen Apheresevorrichtung gehörende zentrale Recheneinheit (10) nicht dargestellt.
- **Fig. 10:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Die Abfallleitung (13") zweigt an dem Knotenpunkt (P8) von dem Bypass-Leitungsabschnitt (12') der Bypass-Leitung (12) ab und die Abfallleitung (13') zweigt an dem Knotenpunkt (P4) von der Plasmaleitung (8B). Zusätzlich mündet die Regenerationsleitung (14) zum Anschluss eines Flüssigkeitsbehälters (F2) an dem Knotenpunkt (P2) in das extrakorporale Zirkulationssystem (2). Zur verbesserten Übersichtlichkeit ist die ebenfalls zur erfindungsgemäßen Apheresevorrichtung gehörende zentrale Recheneinheit (10) nicht dargestellt.
- **Fig. 11:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Die Abfallleitung (13") zweigt an dem Knotenpunkt (P8) von dem Bypass-Leitungsabschnitt (12") der Bypass-Leitung (12) ab und die Abfallleitung (13') zweigt an dem Knotenpunkt (P4) von der Plasmaleitung (8B). Zusätzlich führt die Regenerationsleitung (14) zum Anschluss eines Flüssigkeitsbehälters (F2) bis zu dem Knotenpunkt (P7). Am Knotenpunkt (P7) zweigen zwei Leitungen (15', 15") ab. Die Leitung (15') mündet am Knotenpunkt (P2) in das extrakorporale Zirkulationssystem (2) und die Leitung (15") mündet in dem Bereich zwischen dem Knotenpunkt (P2) und der Apheresesäule (4"). Zur verbesserten Übersichtlichkeit ist die ebenfalls zur erfindungsgemäßen Apheresevorrichtung gehörende zentrale Recheneinheit (10) nicht dargestellt.
- **Fig. 12:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A") laufen am Knotenpunkt (P2) auseinander und am Knotenpunkt (P6) laufen der Bypass-Leitungsabschnitt (12") der Bypass-Leitung (12) und die Plasmaleitung (8B") zusammen. Die Abfallleitung (13) zweigt an dem Knotenpunkt (P6) vom extrakorporalen Zirkulationssystem (2) ab. Zusätzlich mündet die Regenerationsleitung (14) zum Anschluss eines Flüssigkeitsbehälters (F2) an dem Knotenpunkt (P2) in das extrakorporale Zirkulationssystem (2). Zur verbesserten Übersichtlichkeit ist die ebenfalls zur erfindungsgemäßen Apheresevorrichtung gehörende zentrale Recheneinheit (10) nicht dargestellt.
- **Fig. 13:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12') der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Die Abfallleitung (13) zweigt an dem Knotenpunkt (P6) vom extrakorporalen Zirkulationssystem (2) ab. Zusätzlich mündet die Regenerationsleitung (14), welche an dem Punkt (P5) von der Anschlussleitung (11) abzweigt, an dem Knotenpunkt (P2) in das extrakorporale Zirkulationssystem (2). Zur verbesserten Übersichtlichkeit ist die ebenfalls zur erfindungsgemäßen Apheresevorrichtung gehörende zentrale Recheneinheit (10) nicht dargestellt.
- **Fig. 14:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11'), die in die arterielle Leitung (5) mündet aber auch direkt in den Zellseparator (7) hätte münden können, sowie eine Anschlussleitung (11"), die in den Zellseparator (7) mündet aber auch in die arterielle Leitung (5) hätte münden können. Der Bypass-Leitungsabschnitt (12') der Bypass-Leitung (12) und die Plasmaleitung (8A) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Die Abfallleitung (13) zweigt an dem Knotenpunkt (P6) vom extrakorporalen Zirkulationssystem (2) ab. Zusätzlich mündet die Regenerationsleitung (14), welche an dem Punkt (P5') in Verbindung mit der Anschlussleitung (11') steht und an dem Punkt (P5") in Verbindung mit der Anschlussleitung (11") steht, an dem Knotenpunkt (P2) in das extrakorporale Zirkulationssystem (2). Zur verbesserten Übersichtlichkeit ist die ebenfalls zur erfindungsgemäßen Apheresevorrichtung gehörende zentrale Recheneinheit (10) nicht dargestellt.
- **Fig. 15:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11'), die in die arterielle Leitung (5) mündet aber auch direkt in den Zellseparator (7) hätte münden können, sowie eine Anschlussleitung (11"), die in den Zellseparator (7) mündet aber auch in die arterielle Leitung (5) hätte münden können. Der Bypass-Leitungsabschnitt (12') der Bypass-Leitung (12) und die Plasmaleitung (8A) laufen am Knotenpunkt (P2) auseinander und am Knotenpunkt (P6) laufen die Plasmaleitung (8B') und die Plasmaleitung (8B") zusammen. Die Abfallleitung (12) zweigt an dem Knotenpunkt (P6) vom extrakorporalen Zirkulationssystem (2) ab. Zusätzlich münden eine erste Regenerationsleitung (14'), welche an dem Punkt (P5') von der Anschlussleitung (11') abzweigt, und eine zweite Regenerationsleitung (14"), welche an dem Punkt (P5") von der Anschlussleitung (11") abzweigt, beide an dem Knotenpunkt (P2) in das extrakorporale Zirkulationssystem (2). Zur verbesserten Übersichtlichkeit ist die ebenfalls zur erfindungsgemäßen Apheresevorrichtung gehörende zentrale Recheneinheit (10) nicht dargestellt.
- **Fig. 16:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Die Abfallleitung (13) zweigt an dem Knotenpunkt (P6) vom extrakorporalen Zirkulationssystem (2) ab. Zusätzlich mündet die Regenerationsleitung (14), welche an dem Punkt (P5) von der Anschlussleitung (11) abzweigt, an dem Knotenpunkt (P2) in das extrakorporale Zirkulationssystem (2). Die Zulaufleitung weist diesmal einen zusätzlichen Anschluss für einen Flüssigkeitsbehälter auf, wobei dieser Anschluss in Flussrichtung hinter dem Zellseparator (7) liegt, sodass Flüssigkeit aus diesem zusätzlichen Flüssigkeitsbehälter nicht in den Zellseparator (7) und nicht vor dem Zellseparator (7) in die arterielle Leitung (5) geführt werden kann, sondern nur in den Bypass-Leitungsabschnitt (12') der Bypass-Leitung (12) oder in die Plasmaleitung (8A) in Flussrichtung hinter dem Zellseparator (7) oder direkt in die Apheresesäule (4') oder direkt in die Apheresesäule (4"). Zur verbesserten Übersichtlichkeit ist die ebenfalls zur erfindungsgemäßen Apheresevorrichtung gehörende zentrale Recheneinheit (10) nicht dargestellt.

### Verzeichnis der Bezugszeichen

- 1 -: Apheresevorrichtung
- 2 -: extrakorporales Zirkulationssystem
- 3 -: Mittel zur Generierung und Regulation eines Flusses von Blut (oder Blutplasma) in dem extrakorporalen Zirkulationssystem (Pumpe)
- 4 -: Apheresesäule zur affinitätschromatographischen Entfernung von CRP
- 4' -: Apheresesäule zur affinitätschromatographischen Entfernung von CRP
- 4" -: Apheresesäule zur affinitätschromatographischen Entfernung von CRP
- 5 -: arterielle Leitung
- 6 -: venöse Leitung
- 7 -: Zellseparator
- 8A -: Plasmaleitung (vor der Apheresesäule)
- 8B -: Plasmaleitung (nach der Apheresesäule)
- 9: Zellleitung
- 10 -: zentrale Recheneinheit (CPU)
- 11 -: Anschlussleitung
- 12 -: Bypass-Leitung
- 12' -: Bypass-Leitungsabschnitt der Bypass-Leitung
- 12" -: Bypass-Leitungsabschnitt der Bypass-Leitung
- 13 -: Abfallleitung
- 13' -: Abfallleitung
- 13" -: Abfallleitung
- 14 -: Regenerationsleitung
- 14' -: Regenerationsleitung
- 14" -: Regenerationsleitung
- F -: Flüssigkeitsbehälter
- F1 -: Flüssigkeitsbehälter 1
- F2 -: Flüssigkeitsbehälter 2
- P1 -: Knotenpunkt, an dem die Plasmaleitung (8B) in die venöse Leitung (6) übergeht oder Knotenpunkt, an dem der Bypass-Leitungsabschnitt (12") der Bypass-Leitung (12) oder (8B) und die Zellleitung (9) zusammenlaufen und in die venöse Leitung (6) übergehen
- P2 -: Knotenpunkt, an dem die Bypass-Leitung (12) von der Plasmaleitung (8A) abzweigt oder Knotenpunkt, an dem der Bypass-Leitungsabschnitt (12') der Bypass-Leitung und die Plasmaleitung (8A) auseinanderlaufen
- P3 -: Knotenpunkt, an dem die Bypass-Leitung (12) in die Zellleitung (9) mündet
- P4 -: Knotenpunkt, an dem die Abfallleitung (13) von der Plasmaleitung (8B) abzweigt oder Knotenpunkt, an dem die Abfallleitung (13') von der Plasmaleitung (8B) abzweigt.
- P5 -: Knotenpunkt, an dem die Regenerationsleitung (14) von der AnschlussLeitung (11) abzweigt
- P5, P5' -: Knotenpunkt, an dem die Regenerationsleitung (14) von der Anschlussleitung (11) bzw. (11') abzweigt.
- P6 -: Knotenpunkt, an dem die Bypass-Leitung (12) in die Plasmaleitung (8B) mündet oder Knotenpunkt, an dem der Bypass-Leitungsabschnitt (12") der Bypass-Leitung (12) und die Plasmaleitung (8B) zusammenlaufen und zusammen als Bypass-Leitungsabschnitt (12") der Bypass-Leitung (12) oder (8B) bis zum Punkt P1 laufen.
- P7 -: Knotenpunkt in der Regenerationsleitung (14), ab dem sich die Regenerationsleitung (14) die Leitungen (15') und (15") teilt.
- P8 -: Knotenpunkt, an dem die Abfallleitung (13") von dem Bypass-Leitungsabschnitt (12") der Bypass-Leitung abzweigt.

## Patentansprüche

1. Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile,
mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7), eine Plasmaleitung (8A) von dem Zellseparator (7) zur Apheresesäule (4), eine Plasmaleitung (8B) für CRP-abgereichertes Blutplasma von der Apheresesäule (4) bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (1),
zumindest eine Anschlussleitung (11) zum Anschluss von zumindest einem Flüssigkeitsbehälter (F) an die arterielle Leitung (5) oder den Zellseparator (7),
**dadurch gekennzeichnet, dass**
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4) mündet.

2. Vorrichtung gemäß Anspruch 1, wobei die zumindest eine Regenerationsleitung (14), die in die Plasmaleitung (8A) oder direkt in die Apheresesäule (4) führt, von einem Punkt (P5) in der zumindest einen Anschlussleitung (11) ausgeht.

3. Vorrichtung gemäß Anspruch 1 oder 2, wobei die Apheresevorrichtung (1) zumindest zwei Anschlussleitungen (11) zum Anschluss von jeweils zumindest einem Flüssigkeitsbehälter (F) an die arterielle Leitung (5) oder den Zellseparator (7) aufweist, und wobei je Flüssigkeitsbehälter (F) eine Regenerationsleitung (14) existiert, die von dem jeweiligen Flüssigkeitsbehälter (F) oder dessen Anschlussleitung (11) abgehen und die jeweils in die Plasmaleitung (8A) oder direkt in die Apheresesäule (4) führen.

4. Vorrichtung gemäß einem der Ansprüche 1 - 3, wobei die Apheresevorrichtung (1) zwei Anschlussleitungen (11', 11") zum Anschluss von jeweils zumindest einem Flüssigkeitsbehälter (F) an die arterielle Leitung (5) oder den Zellseparator (7) aufweist, und wobei die zumindest eine Regenerationsleitung (14), die in die Plasmaleitung (8A) oder direkt in die Apheresesäule (4) führt, sich an einem Punkt (P5') mit der Anschlussleitung (11') und an einem Punkt (P5") mit der Anschlussleitung (11") verbindet.

5. Vorrichtung gemäß einem der Ansprüche 1 - 4, wobei die Apheresevorrichtung (1) zwei Anschlussleitungen (11', 11") zum Anschluss von jeweils einem Flüssigkeitsbehälter (F1, F2) an die arterielle Leitung (5) oder den Zellseparator (7) aufweist, und wobei zwei Regenerationsleitungen (14', 14") von den zwei Flüssigkeitsbehältern (F1, F2) oder den zwei Anschlussleitungen (11', 11") abgehen und in die Plasmaleitung (8A) oder direkt in die Apheresesäule (4) führen.

6. Vorrichtung gemäß einem der Ansprüche 1 - 5, wobei die Apheresevorrichtung (1) eine Anschlussleitung (11') zum Anschluss von einem Flüssigkeitsbehälter (F1) an die arterielle Leitung (5) oder den Zellseparator (7) und eine Anschlussleitung (11") zum Anschluss von einem Flüssigkeitsbehälter (F2) an die arterielle Leitung (5) oder den Zellseparator (7) aufweist, und wobei eine Regenerationsleitung (14') von dem Flüssigkeitsbehälter (F1) oder der Anschlussleitung (11') abgeht und in Flussrichtung nach der Abzweigung der Bypass-Leitung (12) in die Plasmaleitung (8A) oder direkt in die Apheresesäule (4) führt und eine Regenerationsleitung (14") von dem Flüssigkeitsbehälter (F2) oder der Anschlussleitung (11") abgeht und in Flussrichtung nach der Abzweigung der Bypass-Leitung (12) in die Plasmaleitung (8A) oder in die Regenerationsleitung (14') oder direkt in die Apheresesäule (4) führt.

7. Vorrichtung gemäß einem der Ansprüche 1 - 6, wobei die Bypass-Leitung (12) von einem Punkt (P2) in der Plasmaleitung (8A) zu einem Punkt (P6) in der Plasmaleitung (8B) führt, und die Abfallleitung (13) von einem Punkt (P4) von der Plasmaleitung (8B) abgeht, und die zumindest eine Regenerationsleitung (14) am Punkt (P2) in die Plasmaleitung (8A) mündet.

8. Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile,
zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
eine zentrale Recheneinheit (10) zur Steuerung der Apheresevorrichtung (1), zumindest eine Anschlussleitung (11) zum Anschluss von zumindest einem Flüssigkeitsbehälter (F) an die arterielle Leitung (5) oder den Zellseparator (7),
**dadurch gekennzeichnet, dass**
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F) oder der zumindest einen Anschlussleitung (11) abgeht und in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind.

9. Verfahren zur Regeneration einer Apheresesäule (4) zur affinitätschromatographischen Entfernung von CRP in einer Apheresevorrichtung (1) gemäß einem der Ansprüche 1 - 7, wobei das Verfahren die Regeneration im laufenden Betrieb ermöglicht und durch die folgende Schritte gekennzeichnet ist:
(A) Beginn der Umleitung des abgetrennten Plasmas aus der Plasmaleitung (8A) in die Bypass-Leitung (12) und damit Stoppen der Einleitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung und Stoppen der Umleitung des abgetrennten Plasmas aus der Plasmaleitung (8A) in die Bypass-Leitung (12) und damit Einleitung des abgetrennten Plasmas aus der Plasmaleitung (8A) in die Apheresesäule (4),
(E) Schließung der Abfallleitung (13) und Weiterleiten des aus der Apheresesäule (4) austretenden Flüssigkeitsstromes in die venöse Leitung (6).

10. Verfahren gemäß Anspruch 9 zur Regeneration einer Apheresesäule (4) zur affinitätschromatographischen Entfernung von CRP in einer Apheresevorrichtung (1) gemäß einem der Ansprüche 1 - 7, wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Spüllösung über die zumindest eine Regenerationsleitung (14) in die Plasmaleitung (8A) oder direkt in die Apheresesäule (4),
(C) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4),
(D) Beginn der Umleitung des aus der Apheresesäule (4) austretenden Flüssigkeitsstromes von der Plasmaleitung (8B) in die Abfallleitung (13),
(E) Stoppen der Einleitung der Regenerationslösung und Übergang zur Einleitung der Spüllösung über die zumindest eine Regenerationsleitung (14) in die Plasmaleitung (8A) oder direkt in die Apheresesäule (4),
(F) Schließung der Abfallleitung (13) und Weiterleiten des aus der Apheresesäule (4) austretenden Flüssigkeitsstromes in die venöse Leitung (6);
(G) Stoppen der Einleitung von Spüllösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

11. Verfahren gemäß Anspruch 9 oder 10, wobei die Regenerationslösung/-en ausgewählt werden aus der Gruppe umfassend oder bestehend aus: Citrat-Lösung, TRIS-Glycin-Lösung, NaCI-Lösung, Vollelektrolyt-Lösung und EDTA-Lösung und insbesondere Citrat-Lösung.

12. Verfahren gemäß Anspruch 9, 10 oder 11, wobei es sich bei der Spüllösung um eine physiologische NaCI-Lösung und/oder bei der Regenerationslösung um eine Citrat-Lösung handelt.

13. Verfahren gemäß Anspruch 12, wobei Schritt (C) initiiert wird, nachdem ein Gesamtvolumen X an Regenerationslösung/-en in die Plasmaleitung (8A) bzw. direkt in die Apheresesäule (4) eingeleitet wurde, wobei X mindestens 75 % des Volumens der Vorrichtung zwischen dem Punkt, an dem die Regenerationsleitung (14) in Flussrichtung nach der Abzweigung der Bypass-Leitung (12) in das extrakorporale Zirkulationssystem (2) mündet, und dem Punkt, an dem die Abfallleitung (13) dem extrakorporalen Zirkulationssystem (2) entspringt, entspricht.

14. Verfahren zur Regeneration einer Apheresesäule (4') zur affinitätschromatographischen Entfernung von CRP während des laufenden Betriebes einer zweiten Apheresesäule (4") in einer Apheresevorrichtung (II) gemäß Anspruch 8 umfassend die folgenden Schritte:
(A) Ausgehend vom Fluss des Blutplasmas durch die Apheresesäule (4"), Beginn der Einleitung des abgetrennten Blutplasmas über die Plasmaleitung (8A) in die Apheresesäule (4') und Leiten des CRP-abgereicherten Blutplasmas in die venöse Leitung (6) und damit Stoppen der Einleitung des abgetrennten Plasmas über den Bypass-Leitungsabschnitt (12') der Bypass-Leitung (12) in die Apheresesäule (4"),
(B) Beginn der Einleitung von Regenerationslösung über die zumindest eine Regenerationsleitung (14) in den Bypass-Leitungsabschnitt (12') der Bypass-Leitung (12) oder direkt in die Apheresesäule (4"),
(C) Beginn der Umleitung des aus der Apheresesäule (4") austretenden Flüssigkeitsstromes von dem Bypass-Leitungsabschnitt (12") der Bypass-Leitung (12) in die Abfallleitung (13"),
(D) Beginn der Einleitung des abgetrennten Blutplasmas über den Bypass-Leitungsabschnitt (12') der Bypass-Leitung (12) in die Apheresesäule (4") und Leiten des CRP-abgereicherten Plasmas in die venöse Leitung (6) und damit Stoppen der Einleitung des abgetrennten Blutplasmas über die Plasmaleitung (8A) in Apheresesäule (4')
(E) Schließung der Abfallleitung (13") und Beginn der Umleitung des aus der Apheresesäule (4') austretenden Flüssigkeitsstromes von der Plasmaleitung (8B) in die Abfallleitung (13').
